# EUROPEAN PATENT SPECIFICATION

(11) **EP 1 135 411 B1**
(45) Date of publication and mention of the grant of the patent: **17.05.2006**
(21) Application number: 99967188.6
(22) Date of filing: 03.12.1999
(51) Int. Cl.: C07K 14/47, A61K 38/17

(54) **METHODS AND COMPOSITIONS FOR TREATING DISORDERS INVOLVING EXCITOTOXICITY**
VERFAHREN UNFD ZUSAMMENSETZUNGEN ZUR BEHANDLUNG VON KRANKHEITEN BEI DENEN EXITOTOXIZITÄT BETEILIGT IST
PROCEDES ET COMPOSITIONS SERVANT A TRAITER DES TROUBLES ENTRAINANT UNE EXCITOTOXICITE

(30) Priority: 03.12.1998 WO PCT/US98/25676; 27.01.1999 US 238243; 03.06.1999 US 325602
(43) Date of publication of application: 26.09.2001
(73) Proprietor: Curis, Inc., Cambridge, MA 02138 (US)
(72) Inventor: GALDES, Alphonse, Lexington, MA 02173 (US); MAHANTHAPPA, Nagesh, Cambridge, MA 02139 (US)
(74) Representative: Horner, Martin Grenville
(86) International application number: PCT/US1999/028721
(87) International publication number: WO 2000/035948

(56) References cited:
- EP-A- 0 953 575
- EP-A- 0 953 576
- WO-A-95/23223
- WO-A-96/16668
- WO-A-98/30576
- WO-A-99/28343
- WO-A-99/28876
- PORTER ET AL: "Cholesterol modification of hedgehog signaling proteins in animal development" SCIENCE,US,AMERICAN ASSOCIATION FOR THE ADVANCEMENT OF SCIENCE,, vol. 274, 11 October 1996 (1996-10-11), pages 255-259, XP002082038 ISSN: 0036-8075

### SEQUENCE LISTING

(2) INFORMATION FOR SEQ ID NO:1:
   (i) SEQUENCE CHARACTERISTICS:
      (A) LENGTH: 1277 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1275
   (xi) SEQUENCE DESCRIPTION: SEQ ID NO:1:
   ATG GTC GAA ATG CTG CTG TTG ACA AGA ATT CTC TTG GTG GGC TTC ATC        48
   Met Val Glu Met Leu Leu Leu Thr Arg Ile Leu Leu Val Gly Phe Ile
    1               5                  10                  15
   TGC GCT CTT TTA GTC TCC TCT GGG CTG ACT TGT GGA CCA GGC AGG GGC        96
   Cys Ala Leu Leu Val Ser Ser Gly Leu Thr Cys Gly Pro Gly Arg Gly
               20                  25                  30
   ATT GGA AAA AGG AGG CAC CCC AAA AAG CTG ACC CCG TTA GCC TAT AAG       144
   Ile Gly Lys Arg Arg His Pro Lys Lys Leu Thr Pro Leu Ala Tyr Lys
           35                  40                  45
   CAG TTT ATT CCC AAT GTG GCA GAG AAG ACC CTA GGG GCC AGT GGA AGA       192
   Gln Phe Ile Pro Asn Val Ala Glu Lys Thr Leu Gly Ala Ser Gly Arg
       50                  55                  60
   TAT GAA GGG AAG ATC ACA AGA AAC TCC GAG AGA TTT AAA GAA CTA ACC       240
   Tyr Glu Gly Lys Ile Thr Arg Asn Ser Glu Arg Phe Lys Glu Leu Thr
   65                  70                  75                  80
   CCA AAT TAC AAC CCT GAC ATT ATT TTT AAG GAT GAA GAG AAC ACG GGA       288
   Pro Asn Tyr Asn Pro Asp Ile Ile Phe Lys Asp Glu Glu Asn Thr Gly
                   85                  90                  95
   GCT GAC AGA CTG ATG ACT CAG CGC TGC AAG GAC AAG CTG AAT GCC CTG       336
   Ala Asp Arg Leu Met Thr Gln Arg Cys Lys Asp Lys Leu Asn Ala Leu
               100                 105                 110
   GCG ATC TCG GTG ATG AAC CAG TGG CCC GGG GTG AAG CTG CGG GTG ACC       384
   Ala Ile Ser Val Met Asn Gln Trp Pro Gly Val Lys Leu Arg Val Thr
           115                 120                 125
   GAG GGC TGG GAC GAG GAT GGC CAT CAC TCC GAG GAA TCG CTG CAC TAC       432
   Glu Gly Trp Asp Glu Asp Gly His His Ser Glu Glu Ser Leu His Tyr
       130                 135                 140
   GAG GGT CGC GCC GTG GAC ATC ACC ACG TCG GAT CGG GAC CGC AGC AAG       480
   Glu Gly Arg Ala Val Asp Ile Thr Thr Ser Asp Arg Asp Arg Ser Lys
   145                 150                 155                 160
   TAC GGA ATG CTG GCC CGC CTC GCC GTC GAG GCC GGC TTC GAC TGG GTC      528
   Tyr Gly Met Leu Ala Arg Leu Ala Val Glu Ala Gly Phe Asp Trp Val
                   165                 170                 175
   TAC TAC GAG TCC AAG GCG CAC ATC CAC TGC TCC GTC AAA GCA GAA AAC      576
   Tyr Tyr Glu Ser Lys Ala His Ile His Cys Ser Val Lys Ala Glu Asn
               180                 185                 190
   TCA GTG GCA GCG AAA TCA GGA GGC TGC TTC CCT GGC TCA GCC ACA GTG      624
   Ser Val Ala Ala Lys Ser Gly Gly Cys Phe Pro Gly Ser Ala Thr Val
           195                 200                 205
   CAC CTG GAG CAT GGA GGC ACC AAG CTG GTG AAG GAC CTG AGC CCT GGG      672
   His Leu Glu His Gly Gly Thr Lys Leu Val Lys Asp Leu Ser Pro Gly
       210                 215                 220
   GAC CGC GTG CTG GCT GCT GAC GCG GAC GGC CGG CTG CTC TAC AGT GAC      720
   Asp Arg Val Leu Ala Ala Asp Ala Asp Gly Arg Leu Leu Tyr Ser Asp
   225                 230                 235                 240
   TTC CTC ACC TTC CTC GAC CGG ATG GAC AGC TCC CGA AAG CTC TTC TAC      768
   Phe Leu Thr Phe Leu Asp Arg Met Asp Ser Ser Arg Lys Leu Phe Tyr
                   245                 250                 255
   GTC ATC GAG ACG CGG CAG CCC CGG GCC CGG CTG CTA CTG ACG GCG GCC      816
   Val Ile Glu Thr Arg Gln Pro Arg Ala Arg Leu Leu Leu Thr Ala Ala
               260                 265                 270
   CAC CTG CTC TTT GTG GCC CCC CAG CAC AAC CAG TCG GAG GCC ACA GGG      864
   His Leu Leu Phe Val Ala Pro Gln His Asn Gln Ser Glu Ala Thr Gly
           275                 280                 285
   TCC ACC AGT GGC CAG GCG CTC TTC GCC AGC AAC GTG AAG CCT GGC CAA      912
   Ser Thr Ser Gly Gln Ala Leu Phe Ala Ser Asn Val Lys Pro Gly Gln
       290                 295                 300
   CGT GTC TAT GTG CTG GGC GAG GGC GGG CAG CAG CTG CTG CCG GCG TCT      960
   Arg Val Tyr Val Leu Gly Glu Gly Gly Gln Gln Leu Leu Pro Ala Ser
   305                 310                 315                 320
   GTC CAC AGC GTC TCA TTG CGG GAG GAG GCG TCC GGA GCC TAC GCC CCA     1008
   Val His Ser Val Ser Leu Arg Glu Glu Ala Ser Gly Ala Tyr Ala Pro
                   325                 330                 335
   CTC ACC GCC CAG GGC ACC ATC CTC ATC AAC CGG GTG TTG GCC TCC TGC     1056
   Leu Thr Ala Gln Gly Thr Ile Leu Ile Asn Arg Val Leu Ala Ser Cys
               340                 345                 350
   TAC GCC GTC ATC GAG GAG CAC AGT TGG GCC CAT TGG GCC TTC GCA CCA     1104
   Tyr Ala Val Ile Glu Glu His Ser Trp Ala His Trp Ala Phe Ala Pro
           355                 360                 365
   TTC CGC TTG GCT CAG GGG CTG CTG GCC GCC CTC TGC CCA GAT GGG GCC     1152
   Phe Arg Leu Ala Gln Gly Leu Leu Ala Ala Leu Cys Pro Asp Gly Ala
       370                 375                 380
   ATC CCT ACT GCC GCC ACC ACC ACC ACT GGC ATC CAT TGG TAC TCA CGG     1200
   Ile Pro Thr Ala Ala Thr Thr Thr Thr Gly Ile His Trp Tyr Ser Arg
   385                 390                 395                 400
   CTC CTC TAC CGC ATC GGC AGC TGG GTG CTG GAT GGT GAC GCG CTG CAT      1248
   Leu Leu Tyr Arg Ile Gly Ser Trp Val Leu Asp Gly Asp Ala Leu His
                   405                 410                 415
   CCG CTG GGC ATG GTG GCA CCG GCC AGC TG                               1277
   Pro Leu Gly Met Val Ala Pro Ala Ser
               420                 425
(2) INFORMATION FOR SEQ ID NO:2:
   (i) SEQUENCE CHARACTERISTICS:
      (A) LENGTH: 1190 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1191
(2) INFORMATION FOR SEQ ID NO:3:
   (i) SEQUENCE CHARACTERISTICS:
      (A) LENGTH: 1281 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1233
   (xi) SEQUENCE DESCRIPTION: SEQ ID NO:3:
   ATG TCT CCC GCC TGG CTC CGG CCC CGA CTG CGG TTC TGT CTG TTC CTG       48
   Met Ser Pro Ala Trp Leu Arg Pro Arg Leu Arg Phe Cys Leu Phe Leu
    1               5                  10                  15
   CTG CTG CTG CTT CTG GTG CCG GCG GCG CGG GGC TGC GGG CCG GGC CGG       96
   Leu Leu Leu Leu Leu Val Pro Ala Ala Arg Gly Cys Gly Pro Gly Arg
               20                  25                  30
   GTG GTG GGC AGC CGC CGG AGG CCG CCT CGC AAG CTC GTG CCT CTT GCC      144
   Val Val Gly Ser Arg Arg Arg Pro Pro Arg Lys Leu Val Pro Leu Ala
           35                  40                  45
   TAC AAG CAG TTC AGC CCC AAC GTG CCG GAG AAG ACC CTG GGC GCC AGC      192
   Tyr Lys Gln Phe Ser Pro Asn Val Pro Glu Lys Thr Leu Gly Ala Ser
       50                  55                  60
   GGG CGC TAC GAA GGC AAG ATC GCG CGC AGC TCT GAG CGC TTC AAA GAG      240
   Gly Arg Tyr Glu Gly Lys Ile Ala Arg Ser Ser Glu Arg Phe Lys Glu
   65                  70                  75                  80
   CTC ACC CCC AAC TAC AAT CCC GAC ATC ATC TTC AAG GAC GAG GAG AAC      288
   Leu Thr Pro Asn Tyr Asn Pro Asp Ile Ile Phe Lys Asp Glu Glu Asn
                   85                  90                  95
   ACG GGT GCC GAC CGC CTC ATG ACC CAG CGC TGC AAG GAC CGT CTG AAC      336
   Thr Gly Ala Asp Arg Leu Met Thr Gln Arg Cys Lys Asp Arg Leu Asn
               100                 105                 110
   TCA CTG GCC ATC TCT GTC ATG AAC CAG TGG CCT GGT GTG AAA CTG CGG      384
   Ser Leu Ala Ile Ser Val Met Asn Gln Trp Pro Gly Val Lys Leu Arg
           115                 120                 125
   GTG ACC GAA GGC CGG GAT GAA GAT GGC CAT CAC TCA GAG GAG TCT TTA      432
   Val Thr Glu Gly Arg Asp Glu Asp Gly His His Ser Glu Glu Ser Leu
       130                 135                 140
   CAC TAT GAG GGC CGC GCG GTG GAT ATC ACC ACC TCA GAC CGT GAC CGA      480
   His Tyr Glu Gly Arg Ala Val Asp Ile Thr Thr Ser Asp Arg Asp Arg
   145                 150                 155                 160
   AAT AAG TAT GGA CTG CTG GCG CGC TTA GCA GTG GAG GCC GGC TTC GAC      528
   Asn Lys Tyr Gly Leu Leu Ala Arg Leu Ala Val Glu Ala Gly Phe Asp
                   165                 170                 175
   TGG GTG TAT TAC GAG TCC AAG GCC CAC GTG CAT TGC TCT GTC AAG TCT      576
   Trp Val Tyr Tyr Glu Ser Lys Ala His Val His Cys Ser Val Lys Ser
               180                 185                 190
   GAG CAT TCG GCC GCT GCC AAG ACA GGT GGC TGC TTT CCT GCC GGA GCC      624
   Glu His Ser Ala Ala Ala Lys Thr Gly Gly Cys Phe Pro Ala Gly Ala
           195                 200                 205
   CAG GTG CGC CTA GAG AAC GGG GAG CGT GTG GCC CTG TCA GCT GTA AAG      672
   Gln Val Arg Leu Glu Asn Gly Glu Arg Val Ala Leu Ser Ala Val Lys
       210                 215                 220
   CCA GGA GAC CGG GTG CTG GCC ATG GGG GAG GAT GGG ACC CCC ACC TTC      720
   Pro Gly Asp Arg Val Leu Ala Met Gly Glu Asp Gly Thr Pro Thr Phe
   225                 230                 235                 240
   AGT GAT GTG CTT ATT TTC CTG GAC CGC GAG CCA AAC CGG CTG AGA GCT      768
   Ser Asp Val Leu Ile Phe Leu Asp Arg Glu Pro Asn Arg Leu Arg Ala
                   245                 250                 255
   TTC CAG GTC ATC GAG ACT CAG GAT CCT CCG CGT CGG CTG GCG CTC ACG      816
   Phe Gln Val Ile Glu Thr Gln Asp Pro Pro Arg Arg Leu Ala Leu Thr
               260                 265                 270
   CCT GCC CAC CTG CTC TTC ATT GCG GAC AAT CAT ACA GAA CCA GCA GCC      864
   Pro Ala His Leu Leu Phe Ile Ala Asp Asn His Thr Glu Pro Ala Ala
           275                 280                 285
   CAC TTC CGG GCC ACA TTT GCC AGC CAT GTG CAA CCA GGC CAA TAT GTG      912
   His Phe Arg Ala Thr Phe Ala Ser His Val Gln Pro Gly Gln Tyr Val
       290                 295                 300
   CTG GTA TCA GGG GTA CCA GGC CTC CAG CCT GCT CGG GTG GCA GCT GTC      960
   Leu Val Ser Gly Val Pro Gly Leu Gln Pro Ala Arg Val Ala Ala Val
   305                 310                 315                 320
   TCC ACC CAC GTG GCC CTT GGG TCC TAT GCT CCT CTC ACA AGG CAT GGG     1008
   Ser Thr His Val Ala Leu Gly Ser Tyr Ala Pro Leu Thr Arg His Gly
                   325                 330                 335
   ACA CTT GTG GTG GAG GAT GTG GTG GCC TCC TGC TTT GCA GCT GTG GCT     1056
   Thr Leu Val Val Glu Asp Val Val Ala Ser Cys Phe Ala Ala Val Ala
               340                 345                 350
   GAC CAC CAT CTG GCT CAG TTG GCC TTC TGG CCC CTG CGA CTG TTT CCC      1104
   Asp His His Leu Ala Gln Leu Ala Phe Trp Pro Leu Arg Leu Phe Pro
           355                 360                 365
   AGT TTG GCA TGG GGC AGC TGG ACC CCA AGT GAG GGT GTT CAC TCC TAC      1152
   Ser Leu Ala Trp Gly Ser Trp Thr Pro Ser Glu Gly Val His Ser Tyr
       370                 375                 380
   CCT CAG ATG CTC TAC CGC CTG GGG CGT CTC TTG CTA GAA GAG AGC ACC      1200
   Pro Gln Met Leu Tyr Arg Leu Gly Arg Leu Leu Leu Glu Glu Ser Thr
   385                 390                 395                 400
   TTC CAT CCA CTG GGC ATG TCT GGG GCA GGA AGC TGAAGGGACT CTAACCACTG    1253
   Phe His Pro Leu Gly Met Ser Gly Ala Gly Ser
                   405                 410
   CCCTCCTGGA ACTGCTGTGC GTGGATCC                                       1281
(2) INFORMATION FOR SEQ ID NO:4:
   (i) SEQUENCE CHARACTERISTICS:
      (A) LENGTH: 1313 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1314
   (xi) SEQUENCE DESCRIPTION: SEQ ID NO:4:
   ATG CTG CTG CTG CTG GCC AGA TGT TTT CTG GTG ATC CTT GCT TCC TCG       48
   Met Leu Leu Leu Leu Ala Arg Cys Phe Leu Val Ile Leu Ala Ser Ser
    1               5                  10                  15
   CTG CTG GTG TGC CCC GGG CTG GCC TGT GGG CCC GGC AGG GGG TTT GGA       96
   Leu Leu Val Cys Pro Gly Leu Ala Cys Gly Pro Gly Arg Gly Phe Gly
               20                  25                  30
   AAG AGG CGG CAC CCC AAA AAG CTG ACC CCT TTA GCC TAC AAG CAG TTT      144
   Lys Arg Arg His Pro Lys Lys Leu Thr Pro Leu Ala Tyr Lys Gln Phe
           35                  40                  45
   ATT CCC AAC GTA GCC GAG AAG ACC CTA GGG GCC AGC GGC AGA TAT GAA      192
   Ile Pro Asn Val Ala Glu Lys Thr Leu Gly Ala Ser Gly Arg Tyr Glu
       50                  55                  60
   GGG AAG ATC ACA AGA AAC TCC GAA CGA TTT AAG GAA CTC ACC CCC AAT      240
   Gly Lys Ile Thr Arg Asn Ser Glu Arg Phe Lys Glu Leu Thr Pro Asn
   65                  70                  75                  80
   TAC AAC CCC GAC ATC ATA TTT AAG GAT GAG GAA AAC ACG GGA GCA GAC      288
   Tyr Asn Pro Asp Ile Ile Phe Lys Asp Glu Glu Asn Thr Gly Ala Asp
                   85                  90                  95
   CGG CTG ATG ACT CAG AGG TGC AAA GAC AAG TTA AAT GCC TTG GCC ATC      336
   Arg Leu Met Thr Gln Arg Cys Lys Asp Lys Leu Asn Ala Leu Ala Ile
               100                 105                 110
   TCT GTG ATG AAC CAG TGG CCT GGA GTG AGG CTG CGA GTG ACC GAG GGC      384
   Ser Val Met Asn Gln Trp Pro Gly Val Arg Leu Arg Val Thr Glu Gly
           115                 120                 125
   TGG GAT GAG GAC GGC CAT CAT TCA GAG GAG TCT CTA CAC TAT GAG GGT      432
   Trp Asp Glu Asp Gly His His Ser Glu Glu Ser Leu His Tyr Glu Gly
       130                 135                 140
   CGA GCA GTG GAC ATC ACC ACG TCC GAC CGG GAC CGC AGC AAG TAC GGC      480
   Arg Ala Val Asp Ile Thr Thr Ser Asp Arg Asp Arg Ser Lys Tyr Gly
   145                 150                 155                 160
   ATG CTG GCT CGC CTG GCT GTG GAA GCA GGT TTC GAC TGG GTC TAC TAT      528
   Met Leu Ala Arg Leu Ala Val Glu Ala Gly Phe Asp Trp Val Tyr Tyr
                   165                 170                 175
   GAA TCC AAA GCT CAC ATC CAC TGT TCT GTG AAA GCA GAG AAC TCC GTG      576
   Glu Ser Lys Ala His Ile His Cys Ser Val Lys Ala Glu Asn Ser Val
               180                 185                 190
   GCG GCC AAA TCC GGC GGC TGT TTC CCG GGA TCC GCC ACC GTG CAC CTG      624
   Ala Ala Lys Ser Gly Gly Cys Phe Pro Gly Ser Ala Thr Val His Leu
           195                 200                 205
   GAG CAG GGC GGC ACC AAG CTG GTG AAG GAC TTA CGT CCC GGA GAC CGC      672
   Glu Gln Gly Gly Thr Lys Leu Val Lys Asp Leu Arg Pro Gly Asp Arg
       210                 215                 220
   GTG CTG GCG GCT GAC GAC CAG GGC CGG CTG CTG TAC AGC GAC TTC CTC      720
   Val Leu Ala Ala Asp Asp Gln Gly Arg Leu Leu Tyr Ser Asp Phe Leu
   225                 230                 235                 240
   ACC TTC CTG GAC CGC GAC GAA GGC GCC AAG AAG GTC TTC TAC GTG ATC      768
   Thr Phe Leu Asp Arg Asp Glu Gly Ala Lys Lys Val Phe Tyr Val Ile
                   245                 250                 255
   GAG ACG CTG GAG CCG CGC GAG CGC CTG CTG CTC ACC GCC GCG CAC CTG      816
   Glu Thr Leu Glu Pro Arg Glu Arg Leu Leu Leu Thr Ala Ala His Leu
               260                 265                 270
   CTC TTC GTG GCG CCG CAC AAC GAC TCG GGG CCC ACG CCC GGG CCA AGC      864
   Leu Phe Val Ala Pro His Asn Asp Ser Gly Pro Thr Pro Gly Pro Ser
           275                 280                 285
   GCG CTC TTT GCC AGC CGC GTG CGC CCC GGG CAG CGC GTG TAC GTG GTG      912
   Ala Leu Phe Ala Ser Arg Val Arg Pro Gly Gln Arg Val Tyr Val Val
       290                 295                 300
   GCT GAA CGC GGC GGG GAC CGC CGG CTG CTG CCC GCC GCG GTG CAC AGC      960
   Ala Glu Arg Gly Gly Asp Arg Arg Leu Leu Pro Ala Ala Val His Ser
   305                 310                 315                 320
   GTG ACG CTG CGA GAG GAG GAG GCG GGC GCG TAC GCG CCG CTC ACG GCG        1008
   Val Thr Leu Arg Glu Glu Glu Ala Gly Ala Tyr Ala Pro Leu Thr Ala
                   325                 330                 335
   CAC GGC ACC ATT CTC ATC AAC CGG GTG CTC GCC TCG TGC TAC GCT GTC        1056
   His Gly Thr Ile Leu Ile Asn Arg Val Leu Ala Ser Cys Tyr Ala Val
               340                 345                 350
   ATC GAG GAG CAC AGC TGG GCA CAC CGG GCC TTC GCG CCT TTC CGC CTG        1104
   Ile Glu Glu His Ser Trp Ala His Arg Ala Phe Ala Pro Phe Arg Leu
           355                 360                 365
   GCG CAC GCG CTG CTG GCC GCG CTG GCA CCC GCC CGC ACG GAC GGC GGG        1152
   Ala His Ala Leu Leu Ala Ala Leu Ala Pro Ala Arg Thr Asp Gly Gly
       370                 375                 380
   GGC GGG GGC AGC ATC CCT GCA GCG CAA TCT GCA ACG GAA GCG AGG GGC        1200
   Gly Gly Gly Ser Ile Pro Ala Ala Gln Ser Ala Thr Glu Ala Arg Gly
   385                 390                 395                 400
   GCG GAG CCG ACT GCG GGC ATC CAC TGG TAC TCG CAG CTG CTC TAC CAC        1248
   Ala Glu Pro Thr Ala Gly Ile His Trp Tyr Ser Gln Leu Leu Tyr His
                   405                 410                 415
   ATT GGC ACC TGG CTG TTG GAC AGC GAG ACC ATG CAT CCC TTG GGA ATG        1296
   Ile Gly Thr Trp Leu Leu Asp Ser Glu Thr Met His Pro Leu Gly Met
               420                 425                 430
   GCG GTC AAG TCC AGC TG                                                 1313
   Ala Val Lys Ser Ser
           435
(2) INFORMATION FOR SEQ ID NO:5:
   (i) SEQUENCE CHARACTERISTICS:
      (A) LENGTH: 1256 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1257
   (xi) SEQUENCE DESCRIPTION: SEQ ID NO:5:
   ATG CGG CTT TTG ACG AGA GTG CTG CTG GTG TCT CTT CTC ACT CTG TCC          48
   Met Arg Leu Leu Thr Arg Val Leu Leu Val Ser Leu Leu Thr Leu Ser
     1               5                  10                  15
   TTG GTG GTG TCC GGA CTG GCC TGC GGT CCT GGC AGA GGC TAC GGC AGA          96
   Leu Val Val Ser Gly Leu Ala Cys Gly Pro Gly Arg Gly Tyr Gly Arg
                20                  25                  30
   AGA AGA CAT CCG AAG AAG CTG ACA CCT CTC GCC TAC AAG CAG TTC ATA      144
   Arg Arg His Pro Lys Lys Leu Thr Pro Leu Ala Tyr Lys Gln Phe Ile
           35                  40                  45
   CCT AAT GTC GCG GAG AAG ACC TTA GGG GCC AGC GGC AGA TAC GAG GGC      192
   Pro Asn Val Ala Glu Lys Thr Leu Gly Ala Ser Gly Arg Tyr Glu Gly
       50                  55                  60
   AAG ATA ACG CGC AAT TCG GAG AGA TTT AAA GAA CTT ACT CCA AAT TAC      240
   Lys Ile Thr Arg Asn Ser Glu Arg Phe Lys Glu Leu Thr Pro Asn Tyr
   65                  70                  75                  80
   AAT CCC GAC ATT ATC TTT AAG GAT GAG GAG AAC ACG GGA GCG GAC AGG      288
   Asn Pro Asp Ile Ile Phe Lys Asp Glu Glu Asn Thr Gly Ala Asp Arg
                   85                  90                  95
   CTC ATG ACA CAG AGA TGC AAA GAC AAG CTG AAC TCG CTG GCC ATC TCT      336
   Leu Met Thr Gln Arg Cys Lys Asp Lys Leu Asn Ser Leu Ala Ile Ser
               100                 105                 110
   GTA ATG AAC CAC TGG CCA GGG GTT AAG CTG CGT GTG ACA GAG GGC TGG      384
   Val Met Asn His Trp Pro Gly Val Lys Leu Arg Val Thr Glu Gly Trp
           115                 120                 125
   GAT GAG GAC GGT CAC CAT TTT GAA GAA TCA CTC CAC TAC GAG GGA AGA      432
   Asp Glu Asp Gly His His Phe Glu Glu Ser Leu His Tyr Glu Gly Arg
       130                 135                 140
   GCT GTT GAT ATT ACC ACC TCT GAC CGA GAC AAG AGC AAA TAC GGG ACA      480
   Ala Val Asp Ile Thr Thr Ser Asp Arg Asp Lys Ser Lys Tyr Gly Thr
   145                 150                 155                 160
   CTG TCT CGC CTA GCT GTG GAG GCT GGA TTT GAC TGG GTC TAT TAC GAG      528
   Leu Ser Arg Leu Ala Val Glu Ala Gly Phe Asp Trp Val Tyr Tyr Glu
                   165                 170                 175
   TCC AAA GCC CAC ATT CAT TGC TCT GTC AAA GCA GAA AAT TCG GTT GCT      576
   Ser Lys Ala His Ile His Cys Ser Val Lys Ala Glu Asn Ser Val Ala
               180                 185                 190
   GCG AAA TCT GGG GGC TGT TTC CCA GGT TCG GCT CTG GTC TCG CTC CAG      624
   Ala Lys Ser Gly Gly Cys Phe Pro Gly Ser Ala Leu Val Ser Leu Gln
           195                 200                 205
   GAC GGA GGA CAG AAG GCC GTG AAG GAC CTG AAC CCC GGA GAC AAG GTG      672
   Asp Gly Gly Gln Lys Ala Val Lys Asp Leu Asn Pro Gly Asp Lys Val
       210                 215                 220
   CTG GCG GCA GAC AGC GCG GGA AAC CTG GTG TTC AGC GAC TTC ATC ATG      720
   Leu Ala Ala Asp Ser Ala Gly Asn Leu Val Phe Ser Asp Phe Ile Met
   225                 230                 235                 240
   TTC ACA GAC CGA GAC TCC ACG ACG CGA CGT GTG TTT TAC GTC ATA GAA      768
   Phe Thr Asp Arg Asp Ser Thr Thr Arg Arg Val Phe Tyr Val Ile Glu
                   245                 250                 255
   ACG CAA GAA CCC GTT GAA AAG ATC ACC CTC ACC GCC GCT CAC CTC CTT      816
   Thr Gln Glu Pro Val Glu Lys Ile Thr Leu Thr Ala Ala His Leu Leu
               260                 265                 270
   TTT GTC CTC GAC AAC TCA ACG GAA GAT CTC CAC ACC ATG ACC GCC GCG      864
   Phe Val Leu Asp Asn Ser Thr Glu Asp Leu His Thr Met Thr Ala Ala
           275                 280                 285
   TAT GCC AGC AGT GTC AGA GCC GGA CAA AAG GTG ATG GTT GTT GAT GAT      912
   Tyr Ala Ser Ser Val Arg Ala Gly Gln Lys Val Met Val Val Asp Asp
       290                 295                 300
   AGC GGT CAG CTT AAA TCT GTC ATC GTG CAG CGG ATA TAC ACG GAG GAG      960
   Ser Gly Gln Leu Lys Ser Val Ile Val Gln Arg Ile Tyr Thr Glu Glu
   305                 310                 315                 320
   CAG CGG GGC TCG TTC GCA CCA GTG ACT GCA CAT GGG ACC ATT GTG GTC     1008
   Gln Arg Gly Ser Phe Ala Pro Val Thr Ala His Gly Thr Ile Val Val
                   325                 330                 335
   GAC AGA ATA CTG GCG TCC TGT TAC GCC GTA ATA GAG GAC CAG GGG CTT     1056
   Asp Arg Ile Leu Ala Ser Cys Tyr Ala Val Ile Glu Asp Gln Gly Leu
               340                 345                 350
   GCG CAT TTG GCC TTC GCG CCC GCC AGG CTC TAT TAT TAC GTG TCA TCA     1104
   Ala His Leu Ala Phe Ala Pro Ala Arg Leu Tyr Tyr Tyr Val Ser Ser
           355                 360                 365
   TTC CTG TCC CCC AAA ACT CCA GCA GTC GGT CCA ATG CGA CTT TAC AAC     1152
   Phe Leu Ser Pro Lys Thr Pro Ala Val Gly Pro Met Arg Leu Tyr Asn
       370                 375                 380
   AGG AGG GGG TCC ACT GGT ACT CCA GGC TCC TGT CAT CAA ATG GGA ACG     1200
   Arg Arg Gly Ser Thr Gly Thr Pro Gly Ser Cys His Gln Met Gly Thr
   385                 390                 395                 400
   TGG CTT TTG GAC AGC AAC ATG CTT CAT CCT TTG GGG ATG TCA GTA AAC     1248
   Trp Leu Leu Asp Ser Asn Met Leu His Pro Leu Gly Met Ser Val Asn
                   405                 410                 415
   TCA AGC TG                                                          1256
   Ser Ser
(2) INFORMATION FOR SEQ ID NO:6:
   (i) SEQUENCE CHARACTERISTICS:
      (A) LENGTH: 1425 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1425
   (xi) SEQUENCE DESCRIPTION: SEQ ID NO:6:
   ATG CTG CTG CTG GCG AGA TGT CTG CTG CTA GTC CTC GTC TCC TCG CTG 48
   Met Leu Leu Leu Ala Arg Cys Leu Leu Leu Val Leu Val Ser Ser Leu
   1 5 10 15
   CTG GTA TGC TCG GGA CTG GCG TGC GGA CCG GGC AGG GGG TTC GGG AAG 96
   Leu Val Cys Ser Gly Leu Ala Cys Gly Pro Gly Arg Gly Phe Gly Lys
   20 25 30
   AGG AGG CAC CCC AAA AAG CTG ACC CCT TTA GCC TAC AAG CAG TTT ATC 144
   Arg Arg His Pro Lys Lys Leu Thr Pro Leu Ala Tyr Lys Gln Phe Ile
   35 40 45
   CCC AAT GTG GCC GAG AAG ACC CTA GGC GCC AGC GGA AGG TAT GAA GGG 192
   Pro Asn Val Ala Glu Lys Thr Leu Gly Ala Ser Gly Arg Tyr Glu Gly
   50 55 60
   AAG ATC TCC AGA AAC TCC GAG CGA TTT AAG GAA CTC ACC CCC AAT TAC 240
   Lys Ile Ser Arg Asn Ser Glu Arg Phe Lys Glu Leu Thr Pro Asn Tyr
   65 70 75 80
   AAC CCC GAC ATC ATA TTT AAG GAT GAA GAA AAC ACC GGA GCG GAC AGG 288
   Asn Pro Asp Ile Ile Phe Lys Asp Glu Glu Asn Thr Gly Ala Asp Arg
   85 90 95
   CTG ATG ACT CAG AGG TGT AAG GAC AAG TTG AAC GCT TTG GCC ATC TCG 336
   Leu Met Thr Gln Arg Cys Lys Asp Lys Leu Asn Ala Leu Ala Ile Ser
   100 105 110
   GTG ATG AAC CAG TGG CCA GGA GTG AAA CTG CGG GTG ACC GAG GGC TGG 384
   Val Met Asn Gln Trp Pro Gly Val Lys Leu Arg Val Thr Glu Gly Trp
   115 120 125
   GAC GAA GAT GGC CAC CAC TCA GAG GAG TCT CTG CAC TAC GAG GGC CGC 432
   Asp Glu Asp Gly His His Ser Glu Glu Ser Leu His Tyr Glu Gly Arg
   130 135 140
   GCA GTG GAC ATC ACC ACG TCT GAC CGC GAC CGC AGC AAG TAC GGC ATG 480
   Ala Val Asp Ile Thr Thr Ser Asp Arg Asp Arg Ser Lys Tyr Gly Met
   145 150 155 160
   CTG GCC CGC CTG GCG GTG GAG GCC GGC TTC GAC TGG GTG TAC TAC GAG 528
   Leu Ala Arg Leu Ala Val Glu Ala Gly Phe Asp Trp Val Tyr Tyr Glu
   165 170 175
   TCC AAG GCA CAT ATC CAC TGC TCG GTG AAA GCA GAG AAC TCG GTG GCG 576
   Ser Lys Ala His Ile His Cys Ser Val Lys Ala Glu Asn Ser Val Ala
   180 185 190
   GCC AAA TCG GGA GGC TGC TTC CCG GGC TCG GCC ACG GTG CAC CTG GAG 624
   Ala Lys Ser Gly Gly Cys Phe Pro Gly Ser Ala Thr Val His Leu Glu
   195 200 205
   CAG GGC GGC ACC AAG CTG GTG AAG GAC CTG AGC CCC GGG GAC CGC GTG 672
   Gln Gly Gly Thr Lys Leu Val Lys Asp Leu Ser Pro Gly Asp Arg Val
   210 215 220
   CTG GCG GCG GAC GAC CAG GGC CGG CTG CTC TAC AGC GAC TTC CTC ACT 720
   Leu Ala Ala Asp Asp Gln Gly Arg Leu Leu Tyr Ser Asp Phe Leu Thr
   225                 230                 235                 240
   TTC CTG GAC CGC GAC GAC GGC GCC AAG AAG GTC TTC TAC GTG ATC GAG      768
   Phe Leu Asp Arg Asp Asp Gly Ala Lys Lys Val Phe Tyr Val Ile Glu
                   245                 250                 255
   ACG CGG GAG CCG CGC GAG CGC CTG CTG CTC ACC GCC GCG CAC CTG CTC      816
   Thr Arg Glu Pro Arg Glu Arg Leu Leu Leu Thr Ala Ala His Leu Leu
               260                 265                 270
   TTT GTG GCG CCG CAC AAC GAC TCG GCC ACC GGG GAG CCC GAG GCG TCC      864
   Phe Val Ala Pro His Asn Asp Ser Ala Thr Gly Glu Pro Glu Ala Ser
           275                 280                 285
   TCG GGC TCG GGG CCG CCT TCC GGG GGC GCA CTG GGG CCT CGG GCG CTG      912
   Ser Gly Ser Gly Pro Pro Ser Gly Gly Ala Leu Gly Pro Arg Ala Leu
       290                 295                 300
   TTC GCC AGC CGC GTG CGC CCG GGC CAG CGC GTG TAC GTG GTG GCC GAG      960
   Phe Ala Ser Arg Val Arg Pro Gly Gln Arg Val Tyr Val Val Ala Glu
   305                 310                 315                 320
   CGT GAC GGG GAC CGC CGG CTC CTG CCC GCC GCT GTG CAC AGC GTG ACC     1008
   Arg Asp Gly Asp Arg Arg Leu Leu Pro Ala Ala Val His Ser Val Thr
                   325                 330                 335
   CTA AGC GAG GAG GCC GCG GGC GCC TAC GCG CCG CTC ACG GCC CAG GGC     1056
   Leu Ser Glu Glu Ala Ala Gly Ala Tyr Ala Pro Leu Thr Ala Gln Gly
               340                 345                 350
   ACC ATT CTC ATC AAC CGG GTG CTG GCC TCG TGC TAC GCG GTC ATC GAG     1104
   Thr Ile Leu Ile Asn Arg Val Leu Ala Ser Cys Tyr Ala Val Ile Glu
           355                 360                 365
   GAG CAC AGC TGG GCG CAC CGG GCC TTC GCG CCC TTC CGC CTG GCG CAC     1152
   Glu His Ser Trp Ala His Arg Ala Phe Ala Pro Phe Arg Leu Ala His
       370                 375                 380
   GCG CTC CTG GCT GCA CTG GCG CCC GCG CGC ACG GAC CGC GGC GGG GAC     1200
   Ala Leu Leu Ala Ala Leu Ala Pro Ala Arg Thr Asp Arg Gly Gly Asp
   385                 390                 395                 400
   AGC GGC GGC GGG GAC CGC GGG GGC GGC GGC GGC AGA GTA GCC CTA ACC     1248
   Ser Gly Gly Gly Asp Arg Gly Gly Gly Gly Gly Arg Val Ala Leu Thr
                   405                 410                 415
   GCT CCA GGT GCT GCC GAC GCT CCG GGT GCG GGG GCC ACC GCG GGC ATC     1296
   Ala Pro Gly Ala Ala Asp Ala Pro Gly Ala Gly Ala Thr Ala Gly Ile
               420                 425                 430
   CAC TGG TAC TCG CAG CTG CTC TAC CAA ATA GGC ACC TGG CTC CTG GAC     1344
   His Trp Tyr Ser Gln Leu Leu Tyr Gln Ile Gly Thr Trp Leu Leu Asp
           435                 440                 445
   AGC GAG GCC CTG CAC CCG CTG GGC ATG GCG GTC AAG TCC AGC NNN AGC     1392
   Ser Glu Ala Leu His Pro Leu Gly Met Ala Val Lys Ser Ser Xaa Ser
       450                 455                 460
   CGG GGG GCC GGG GGA GGG GCG CGG GAG GGG GCC                         1425
   Arg Gly Ala Gly Gly Gly Ala Arg Glu Gly Ala
   465                 470                 475
(2) INFORMATION FOR SEQ ID NO:7:
   (i) SEQUENCE CHARACTERISTICS:
      (A) LENGTH: 1622 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 51..1283
   (xi) SEQUENCE DESCRIPTION: SEQ ID NO:7:
CATCAGCCCA CCAGGAGACC TCGCCCGCCG CTCCCCCGGG CTCCCCGGCC ATG TCT        56
   CCC GCC CGG CTC CGG CCC CGA CTG CAC TTC TGC CTG GTC CTG TTG CTG      104
   CTG CTG GTG GTG CCC GCG GCA TGG GGC TGC GGG CCG GGT CGG GTG GTG      152
   GGC AGC CGC CGG CGA CCG CCA CGC AAA CTC GTG CCG CTC GCC TAC AAG      200
   CAG TTC AGC CCC AAT GTG CCC GAG AAG ACC CTG GGC GCC AGC GGA CGC      248
   TAT GAA GGC AAG ATC GCT CGC AGC TCC GAG CGC TTC AAG GAG CTC ACC      296
   CCC AAT TAC AAT CCA GAC ATC ATC TTC AAG GAC GAG GAG AAC ACA GGC      344
   GCC GAC CGC CTC ATG ACC CAG CGC TGC AAG GAC CGC CTG AAC TCG CTG      392
   GCT ATC TCG GTG ATG AAC CAG TGG CCC GGT GTG AAG CTG CGG GTG ACC      440
   GAG GGC TGG GAC GAG GAC GGC CAC CAC TCA GAG GAG TCC CTG CAT TAT      488
   GAG GGC CGC GCG GTG GAC ATC ACC ACA TCA GAC CGC GAC CGC AAT AAG      536
   TAT GGA CTG CTG GCG CGC TTG GCA GTG GAG GCC GGC TTT GAC TGG GTG      584
   TAT TAC GAG TCA AAG GCC CAC GTG CAT TGC TCC GTC AAG TCC GAG CAC      632
   TCG GCC GCA GCC AAG ACG GGC GGC TGC TTC CCT GCC GGA GCC CAG GTA      680
   CGC CTG GAG AGT GGG GCG CGT GTG GCC TTG TCA GCC GTG AGG CCG GGA      728
   GAC CGT GTG CTG GCC ATG GGG GAG GAT GGG AGC CCC ACC TTC AGC GAT      776
   GTG CTC ATT TTC CTG GAC CGC GAG CCC CAC AGG CTG AGA GCC TTC CAG      824
   GTC ATC GAG ACT CAG GAC CCC CCA CGC CGC CTG GCA CTC ACA CCC GCT      872
   CAC CTG CTC TTT ACG GCT GAC AAT CAC ACG GAG CCG GCA GCC CGC TTC      920
   CGG GCC ACA TTT GCC AGC CAC GTG CAG CCT GGC CAG TAC GTG CTG GTG      968
   GCT GGG GTG CCA GGC CTG CAG CCT GCC CGC GTG GCA GCT GTC TCT ACA     1016
   CAC GTG GCC CTC GGG GCC TAC GCC CCG CTC ACA AAG CAT GGG ACA CTG     1064
   GTG GTG GAG GAT GTG GTG GCA TCC TGC TTC GCG GCC GTG GCT GAC CAC     1112
   CAC CTG GCT CAG TTG GCC TTC TGG CCC CTG AGA CTC TTT CAC AGC TTG     1160
   GCA TGG GGC AGC TGG ACC CCG GGG GAG GGT GTG CAT TGG TAC CCC CAG        1208
   CTG CTC TAC CGC CTG GGG CGT CTC CTG CTA GAA GAG GGC AGC TTC CAC        1256
   CCA CTG GGC ATG TCC GGG GCA GGG AGC TGAAAGGACT CCACCGCTGC             1303
   CCTCCTGGAA CTGCTGTACT GGGTCCAGAA GCCTCTCAGC CAGGAGGGAG CTGGCCCTGG     1363
   AAGGGACCTG AGCTGGGGGA CACTGGCTCC TGCCATCTCC TCTGCCATGA AGATACACCA     1423
   TTGAGACTTG ACTGGGCAAC ACCAGCGTCC CCCACCCGCG TCGTGGTGTA GTCATAGAGC     1483
   TGCAAGCTGA GCTGGCGAGG GGATGGTTGT TGACCCCTCT CTCCTAGAGA CCTTGAGGCT     1543
   GGCACGGCGA CTCCCAACTC AGCCTGCTCT CACTACGAGT TTTCATACTC TGCCTCCCCC     1603
   ATTGGGAGGG CCCATTCCC                                                  1622
(2) INFORMATION FOR SEQ ID NO:8:
   (i) SEQUENCE CHARACTERISTICS:
      (A) LENGTH: 1191 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1191
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 1251 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1248
(2) INFORMATION FOR SEQ ID NO:10:
   (i) SEQUENCE CHARACTERISTICS:
      (A) LENGTH: 425 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Val Glu Met Leu Leu Leu Thr Arg Ile Leu Leu Val Gly Phe Ile
    1               5                  10                  15
   Cys Ala Leu Leu Val Ser Ser Gly Leu Thr Cys Gly Pro Gly Arg Gly
               20                  25                  30
   Ile Gly Lys Arg Arg His Pro Lys Lys Leu Thr Pro Leu Ala Tyr Lys
           35                  40                  45
   Gln Phe Ile Pro Asn Val Ala Glu Lys Thr Leu Gly Ala Ser Gly Arg
       50                  55                  60
   Tyr Glu Gly Lys Ile Thr Arg Asn Ser Glu Arg Phe Lys Glu Leu Thr
   65 70 75 80
   Pro Asn Tyr Asn Pro Asp Ile Ile Phe Lys Asp Glu Glu Asn Thr Gly
   85 90 95
   Ala Asp Arg Leu Met Thr Gln Arg Cys Lys Asp Lys Leu Asn Ala Leu
   100 105 110
   Ala Ile Ser Val Met Asn Gln Trp Pro Gly Val Lys Leu Arg Val Thr
   115 120 125
   Glu Gly Trp Asp Glu Asp Gly His His Ser Glu Glu Ser Leu His Tyr
   130 135 140
   Glu Gly Arg Ala Val Asp Ile Thr Thr Ser Asp Arg Asp Arg Ser Lys
   145 150 155 160
   Tyr Gly Met Leu Ala Arg Leu Ala Val Glu Ala Gly Phe Asp Trp Val
   165 170 175
   Tyr Tyr Glu Ser Lys Ala His Ile His Cys Ser Val Lys Ala Glu Asn
   180 185 190
   Ser Val Ala Ala Lys Ser Gly Gly Cys Phe Pro Gly Ser Ala Thr Val
   195 200 205
   His Leu Glu His Gly Gly Thr Lys Leu Val Lys Asp Leu Ser Pro Gly
   210 215 220
   Asp Arg Val Leu Ala Ala Asp Ala Asp Gly Arg Leu Leu Tyr Ser Asp
   225 230 235 240
   Phe Leu Thr Phe Leu Asp Arg Met Asp Ser Ser Arg Lys Leu Phe Tyr
   245 250 255
   Val Ile Glu Thr Arg Gln Pro Arg Ala Arg Leu Leu Leu Thr Ala Ala
   260 265 270
   His Leu Leu Phe Val Ala Pro Gln His Asn Gln Ser Glu Ala Thr Gly
   275 280 285
   Ser Thr Ser Gly Gln Ala Leu Phe Ala Ser Asn Val Lys Pro Gly Gln
   290 295 300
   Arg Val Tyr Val Leu Gly Glu Gly Gly Gln Gln Leu Leu Pro Ala Ser
   305 310 315 320
   Val His Ser Val Ser Leu Arg Glu Glu Ala Ser Gly Ala Tyr Ala Pro
   325 330 335
   Leu Thr Ala Gln Gly Thr Ile Leu Ile Asn Arg Val Leu Ala Ser Cys
   340 345 350
   Tyr Ala Val Ile Glu Glu His Ser Trp Ala His Trp Ala Phe Ala Pro
   355 360 365
   Phe Arg Leu Ala Gln Gly Leu Leu Ala Ala Leu Cys Pro Asp Gly Ala
   370 375 380
   Ile Pro Thr Ala Ala Thr Thr Thr Thr Gly Ile His Trp Tyr Ser Arg
   385 390 395 400
   Leu Leu Tyr Arg Ile Gly Ser Trp Val Leu Asp Gly Asp Ala Leu His
   405 410 415
   Pro Leu Gly Met Val Ala Pro Ala Ser
   420 425
(2) INFORMATION FOR SEQ ID NO:11:
   (i) SEQUENCE CHARACTERISTICS:
      (A) LENGTH: 396 amino acids
      (B) TYPE: amino acid
      (D) TOPOLOGY: linear
   (ii) MOLECULE TYPE: protein
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 411 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Ser Pro Ala Trp Leu Arg Pro Arg Leu Arg Phe Cys Leu Phe Leu
     1               5                  10                  15
   Leu Leu Leu Leu Leu Val Pro Ala Ala Arg Gly Cys Gly Pro Gly Arg
               20                  25                  30
   Val Val Gly Ser Arg Arg Arg Pro Pro Arg Lys Leu Val Pro Leu Ala
   35 40 45
   Tyr Lys Gln Phe Ser Pro Asn Val Pro Glu Lys Thr Leu Gly Ala Ser
   50 55 60
   Gly Arg Tyr Glu Gly Lys Ile Ala Arg Ser Ser Glu Arg Phe Lys Glu
   65 70 75 80
   Leu Thr Pro Asn Tyr Asn Pro Asp Ile Ile Phe Lys Asp Glu Glu Asn
   85 90 95
   Thr Gly Ala Asp Arg Leu Met Thr Gln Arg Cys Lys Asp Arg Leu Asn
   100 105 110
   Ser Leu Ala Ile Ser Val Met Asn Gln Trp Pro Gly Val Lys Leu Arg
   115 120 125
   Val Thr Glu Gly Arg Asp Glu Asp Gly His His Ser Glu Glu Ser Leu
   130 135 140
   His Tyr Glu Gly Arg Ala Val Asp Ile Thr Thr Ser Asp Arg Asp Arg
   145 150 155 160
   Asn Lys Tyr Gly Leu Leu Ala Arg Leu Ala Val Glu Ala Gly Phe Asp
   165 170 175
   Trp Val Tyr Tyr Glu Ser Lys Ala His Val His Cys Ser Val Lys Ser
   180 185 190
   Glu His Ser Ala Ala Ala Lys Thr Gly Gly Cys Phe Pro Ala Gly Ala
   195 200 205
   Gln Val Arg Leu Glu Asn Gly Glu Arg Val Ala Leu Ser Ala Val Lys
   210 215 220
   Pro Gly Asp Arg Val Leu Ala Met Gly Glu Asp Gly Thr Pro Thr Phe
   225 230 235 240
   Ser Asp Val Leu Ile Phe Leu Asp Arg Glu Pro Asn Arg Leu Arg Ala
   245 250 255
   Phe Gln Val Ile Glu Thr Gln Asp Pro Pro Arg Arg Leu Ala Leu Thr
   260 265 270
   Pro Ala His Leu Leu Phe Ile Ala Asp Asn His Thr Glu Pro Ala Ala
   275 280 285
   His Phe Arg Ala Thr Phe Ala Ser His Val Gln Pro Gly Gln Tyr Val
   290 295 300
   Leu Val Ser Gly Val Pro Gly Leu Gln Pro Ala Arg Val Ala Ala Val
   305 310 315 320
   Ser Thr His Val Ala Leu Gly Ser Tyr Ala Pro Leu Thr Arg His Gly
   325 330 335
   Thr Leu Val Val Glu Asp Val Val Ala Ser Cys Phe Ala Ala Val Ala
   340 345 350
   Asp His His Leu Ala Gln Leu Ala Phe Trp Pro Leu Arg Leu Phe Pro
   355 360 365
   Ser Leu Ala Trp Gly Ser Trp Thr Pro Ser Glu Gly Val His Ser Tyr
   370 375 380
   Pro Gln Met Leu Tyr Arg Leu Gly Arg Leu Leu Leu Glu Glu Ser Thr
   385 390 395 400
   Phe His Pro Leu Gly Met Ser Gly Ala Gly Ser
   405 410
(2) INFORMATION FOR SEQ ID NO:13:
   (i) SEQUENCE CHARACTERISTICS:
      (A) LENGTH: 437 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Leu Leu Leu Leu Ala Arg Cys Phe Leu Val Ile Leu Ala Ser Ser
   1 5 10 15
   Leu Leu Val Cys Pro Gly Leu Ala Cys Gly Pro Gly Arg Gly Phe Gly
   20 25 30
   Lys Arg Arg His Pro Lys Lys Leu Thr Pro Leu Ala Tyr Lys Gln Phe
   35 40 45
   Ile Pro Asn Val Ala Glu Lys Thr Leu Gly Ala Ser Gly Arg Tyr Glu
   50 55 60
   Gly Lys Ile Thr Arg Asn Ser Glu Arg Phe Lys Glu Leu Thr Pro Asn
   65 70 75 80
   Tyr Asn Pro Asp Ile Ile Phe Lys Asp Glu Glu Asn Thr Gly Ala Asp
   85 90 95
   Arg Leu Met Thr Gln Arg Cys Lys Asp Lys Leu Asn Ala Leu Ala Ile
   100 105 110
   Ser Val Met Asn Gln Trp Pro Gly Val Arg Leu Arg Val Thr Glu Gly
   115 120 125
   Trp Asp Glu Asp Gly His His Ser Glu Glu Ser Leu His Tyr Glu Gly
   130 135 140
   Arg Ala Val Asp Ile Thr Thr Ser Asp Arg Asp Arg Ser Lys Tyr Gly
   145 150 155 160
   Met Leu Ala Arg Leu Ala Val Glu Ala Gly Phe Asp Trp Val Tyr Tyr
                   165                 170                 175
   Glu Ser Lys Ala His Ile His Cys Ser Val Lys Ala Glu Asn Ser Val
               180                 185                 190
   Ala Ala Lys Ser Gly Gly Cys Phe Pro Gly Ser Ala Thr Val His Leu
           195                 200                 205
   Glu Gln Gly Gly Thr Lys Leu Val Lys Asp Leu Arg Pro Gly Asp Arg
       210                 215                 220
   Val Leu Ala Ala Asp Asp Gln Gly Arg Leu Leu Tyr Ser Asp Phe Leu
   225                 230                 235                 240
   Thr Phe Leu Asp Arg Asp Glu Gly Ala Lys Lys Val Phe Tyr Val Ile
                   245                 250                 255
   Glu Thr Leu Glu Pro Arg Glu Arg Leu Leu Leu Thr Ala Ala His Leu
               260                 265                 270
   Leu Phe Val Ala Pro His Asn Asp Ser Gly Pro Thr Pro Gly Pro Ser
           275                 280                 285
   Ala Leu Phe Ala Ser Arg Val Arg Pro Gly Gln Arg Val Tyr Val Val
       290                 295                 300
   Ala Glu Arg Gly Gly Asp Arg Arg Leu Leu Pro Ala Ala Val His Ser
   305                 310                 315                 320
   Val Thr Leu Arg Glu Glu Glu Ala Gly Ala Tyr Ala Pro Leu Thr Ala
                   325                 330                 335
   His Gly Thr Ile Leu Ile Asn Arg Val Leu Ala Ser Cys Tyr Ala Val
               340                 345                 350
   Ile Glu Glu His Ser Trp Ala His Arg Ala Phe Ala Pro Phe Arg Leu
           355                 360                 365
   Ala His Ala Leu Leu Ala Ala Leu Ala Pro Ala Arg Thr Asp Gly Gly
       370                 375                 380
   Gly Gly Gly Ser Ile Pro Ala Ala Gln Ser Ala Thr Glu Ala Arg Gly
   385                 390                 395                 400
   Ala Glu Pro Thr Ala Gly Ile His Trp Tyr Ser Gln Leu Leu Tyr His
                   405                 410                 415
   Ile Gly Thr Trp Leu Leu Asp Ser Glu Thr Met His Pro Leu Gly Met
               420                 425                 430
   Ala Val Lys Ser Ser
           435
(2) INFORMATION FOR SEQ ID NO:14:
   (i) SEQUENCE CHARACTERISTICS:
      (A) LENGTH: 418 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Met Arg Leu Leu Thr Arg Val Leu Leu Val Ser Leu Leu Thr Leu Ser
    1               5                  10                  15
   Leu Val Val Ser Gly Leu Ala Cys Gly Pro Gly Arg Gly Tyr Gly Arg
               20                  25                  30
   Arg Arg His Pro Lys Lys Leu Thr Pro Leu Ala Tyr Lys Gln Phe Ile
           35                  40                  45
   Pro Asn Val Ala Glu Lys Thr Leu Gly Ala Ser Gly Arg Tyr Glu Gly
       50                  55                  60
   Lys Ile Thr Arg Asn Ser Glu Arg Phe Lys Glu Leu Thr Pro Asn Tyr
   65                  70                  75                  80
   Asn Pro Asp Ile Ile Phe Lys Asp Glu Glu Asn Thr Gly Ala Asp Arg
                   85                  90                  95
   Leu Met Thr Gln Arg Cys Lys Asp Lys Leu Asn Ser Leu Ala Ile Ser
               100                 105                 110
   Val Met Asn His Trp Pro Gly Val Lys Leu Arg Val Thr Glu Gly Trp
           115                 120                 125
   Asp Glu Asp Gly His His Phe Glu Glu Ser Leu His Tyr Glu Gly Arg
       130                 135                 140
   Ala Val Asp Ile Thr Thr Ser Asp Arg Asp Lys Ser Lys Tyr Gly Thr
   145                 150                 155                 160
   Leu Ser Arg Leu Ala Val Glu Ala Gly Phe Asp Trp Val Tyr Tyr Glu
                   165                 170                 175
   Ser Lys Ala His Ile His Cys Ser Val Lys Ala Glu Asn Ser Val Ala
               180                 185                 190
   Ala Lys Ser Gly Gly Cys Phe Pro Gly Ser Ala Leu Val Ser Leu Gln
           195                 200                 205
   Asp Gly Gly Gln Lys Ala Val Lys Asp Leu Asn Pro Gly Asp Lys Val
       210                 215                 220
   Leu Ala Ala Asp Ser Ala Gly Asn Leu Val Phe Ser Asp Phe Ile Met
   225                 230                 235                 240
   Phe Thr Asp Arg Asp Ser Thr Thr Arg Arg Val Phe Tyr Val Ile Glu
                   245                 250                 255
   Thr Gln Glu Pro Val Glu Lys Ile Thr Leu Thr Ala Ala His Leu Leu
               260                 265                 270
   Phe Val Leu Asp Asn Ser Thr Glu Asp Leu His Thr Met Thr Ala Ala
   275 280 285
   Tyr Ala Ser Ser Val Arg Ala Gly Gln Lys Val Met Val Val Asp Asp
   290 295 300
   Ser Gly Gln Leu Lys Ser Val Ile Val Gln Arg Ile Tyr Thr Glu Glu
   305 310 315 320
   Gln Arg Gly Ser Phe Ala Pro Val Thr Ala His Gly Thr Ile Val Val
   325 330 335
   Asp Arg Ile Leu Ala Ser Cys Tyr Ala Val Ile Glu Asp Gln Gly Leu
   340 345 350
   Ala His Leu Ala Phe Ala Pro Ala Arg Leu Tyr Tyr Tyr Val Ser Ser
   355 360 365
   Phe Leu Ser Pro Lys Thr Pro Ala Val Gly Pro Met Arg Leu Tyr Asn
   370 375 380
   Arg Arg Gly Ser Thr Gly Thr Pro Gly Ser Cys His Gln Met Gly Thr
   385 390 395 400
   Trp Leu Leu Asp Ser Asn Met Leu His Pro Leu Gly Met Ser Val Asn
   405 410 415
   Ser Ser
(2) INFORMATION FOR SEQ ID NO:15:
   (i) SEQUENCE CHARACTERISTICS:
      (A) LENGTH: 475 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Leu Leu Leu Ala Arg Cys Leu Leu Leu Val Leu Val Ser Ser Leu
   1 5 10 15
   Leu Val Cys Ser Gly Leu Ala Cys Gly Pro Gly Arg Gly Phe Gly Lys
   20 25 30
   Arg Arg His Pro Lys Lys Leu Thr Pro Leu Ala Tyr Lys Gln Phe Ile
   35 40 45
   Pro Asn Val Ala Glu Lys Thr Leu Gly Ala Ser Gly Arg Tyr Glu Gly
   50 55 60
   Lys Ile Ser Arg Asn Ser Glu Arg Phe Lys Glu Leu Thr Pro Asn Tyr
   65 70 75 80
   Asn Pro Asp Ile Ile Phe Lys Asp Glu Glu Asn Thr Gly Ala Asp Arg
   85 90 95
   Leu Met Thr Gln Arg Cys Lys Asp Lys Leu Asn Ala Leu Ala Ile Ser
   100 105 110
   Val Met Asn Gln Trp Pro Gly Val Lys Leu Arg Val Thr Glu Gly Trp
   115 120 125
   Asp Glu Asp Gly His His Ser Glu Glu Ser Leu His Tyr Glu Gly Arg
   130 135 140
   Ala Val Asp Ile Thr Thr Ser Asp Arg Asp Arg Ser Lys Tyr Gly Met
   145 150 155 160
   Leu Ala Arg Leu Ala Val Glu Ala Gly Phe Asp Trp Val Tyr Tyr Glu
   165 170 175
   Ser Lys Ala His Ile His Cys Ser Val Lys Ala Glu Asn Ser Val Ala
   180 185 190
   Ala Lys Ser Gly Gly Cys Phe Pro Gly Ser Ala Thr Val His Leu Glu
   195 200 205
   Gln Gly Gly Thr Lys Leu Val Lys Asp Leu Ser Pro Gly Asp Arg Val
   210 215 220
   Leu Ala Ala Asp Asp Gln Gly Arg Leu Leu Tyr Ser Asp Phe Leu Thr
   225 230 235 240
   Phe Leu Asp Arg Asp Asp Gly Ala Lys Lys Val Phe Tyr Val Ile Glu
   245 250 255
   Thr Arg Glu Pro Arg Glu Arg Leu Leu Leu Thr Ala Ala His Leu Leu
   260 265 270
   Phe Val Ala Pro His Asn Asp Ser Ala Thr Gly Glu Pro Glu Ala Ser
   275 280 285
   Ser Gly Ser Gly Pro Pro Ser Gly Gly Ala Leu Gly Pro Arg Ala Leu
   290 295 300
   Phe Ala Ser Arg Val Arg Pro Gly Gln Arg Val Tyr Val Val Ala Glu
   305 310 315 320
   Arg Asp Gly Asp Arg Arg Leu Leu Pro Ala Ala Val His Ser Val Thr
   325 330 335
   Leu Ser Glu Glu Ala Ala Gly Ala Tyr Ala Pro Leu Thr Ala Gln Gly
   340 345 350
   Thr Ile Leu Ile Asn Arg Val Leu Ala Ser Cys Tyr Ala Val Ile Glu
   355 360 365
   Glu His Ser Trp Ala His Arg Ala Phe Ala Pro Phe Arg Leu Ala His
   370 375 380
   Ala Leu Leu Ala Ala Leu Ala Pro Ala Arg Thr Asp Arg Gly Gly Asp
   385 390 395 400
   Ser Gly Gly Gly Asp Arg Gly Gly Gly Gly Gly Arg Val Ala Leu Thr
                   405                 410                 415
   Ala Pro Gly Ala Ala Asp Ala Pro Gly Ala Gly Ala Thr Ala Gly Ile
               420                 425                 430
   His Trp Tyr Ser Gln Leu Leu Tyr Gln Ile Gly Thr Trp Leu Leu Asp
           435                 440                 445
   Ser Glu Ala Leu His Pro Leu Gly Met Ala Val Lys Ser Ser Xaa Ser
       450                 455                 460
   Arg Gly Ala Gly Gly Gly Ala Arg Glu Gly Ala
   465                 470                 475
(2) INFORMATION FOR SEQ ID NO:16:
   (i) SEQUENCE CHARACTERISTICS:
      (A) LENGTH: 411 amino acids
      (B) TYPE: amino acid
      (D) TOPOLOGY: linear
   (ii) MOLECULE TYPE: protein
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 396 amino acids
      (B) TYPE: amino acid
      (D) TOPOLOGY: linear
   (ii) MOLECULE TYPE: protein
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 416 amino acids
      (B) TYPE: amino acid
      (D) TOPOLOGY: linear
   (ii) MOLECULE TYPE: protein
   (xi) SEQUENCE DESCRIPTION: SEQ ID NO:18:
   Met Asp Val Arg Leu His Leu Lys Gln Phe Ala Leu Leu Cys Phe Ile
   1               5                   10                  15
   Ser Leu Leu Leu Thr Pro Cys Gly Leu Ala Cys Gly Pro Gly Arg Gly
               20                  25                  30
   Tyr Gly Lys Arg Arg His Pro Lys Lys Leu Thr Pro Leu Ala Tyr Lys
           35                  40                  45
   Gln Phe Ile Pro Asn Val Ala Glu Lys Thr Leu Gly Ala Ser Gly Lys
       50                  55                  60
   Tyr Glu Gly Lys Ile Thr Arg Asn Ser Glu Arg Phe Lys Glu Leu Ile
   65                  70                  75                  80
   Pro Asn Tyr Asn Pro Asp Ile Ile Phe Lys Asp Glu Glu Asn Thr Asn
                   85                  90                  95
   Ala Asp Arg Leu Met Thr Lys Arg Cys Lys Asp Lys Leu Asn Ser Leu
               100                 105                 110
   Ala Ile Ser Val Met Asn His Trp Pro Gly Val Lys Leu Arg Val Thr
           115                 120                 125
   Glu Gly Trp Asp Glu Asp Gly His His Leu Glu Glu Ser Leu His Tyr
       130                 135                 140
   Glu Gly Arg Ala Val Asp Ile Thr Thr Ser Asp Arg Asp Lys Ser Lys
   145                 150                 155                 160
   Tyr Gly Met Leu Ser Arg Leu Ala Val Glu Ala Gly Phe Asp Trp Val
                   165                 170                 175
   Tyr Tyr Glu Ser Lys Ala His Ile His Cys Ser Val Lys Ala Glu Asn
               180                 185                 190
   Ser Val Ala Ala Lys Ser Gly Gly Cys Phe Pro Gly Ser Gly Thr Val
           195                 200                 205
   Thr Leu Gly Asp Gly Thr Arg Lys Pro Ile Lys Asp Leu Lys Val Gly
       210                 215                 220
   Asp Arg Val Leu Ala Ala Asp Glu Lys Gly Asn Val Leu Ile Ser Asp
   225                 230                 235                 240
   Phe Ile Met Phe Ile Asp His Asp Pro Thr Thr Arg Arg Gln Phe Ile
                   245                 250                 255
   Val Ile Glu Thr Ser Glu Pro Phe Thr Lys Leu Thr Leu Thr Ala Ala
               260                 265                 270
   His Leu Val Phe Val Gly Asn Ser Ser Ala Ala Ser Gly Ile Thr Ala
           275                 280                 285
   Thr Phe Ala Ser Asn Val Lys Pro Gly Asp Thr Val Leu Val Trp Glu
       290                 295                 300
   Asp Thr Cys Glu Ser Leu Lys Ser Val Thr Val Lys Arg Ile Tyr Thr
   305                 310                 315                 320
   Glu Glu His Glu Gly Ser Phe Ala Pro Val Thr Ala His Gly Thr Ile
                   325                 330                 335
   Ile Val Asp Gln Val Leu Ala Ser Cys Tyr Ala Val Ile Glu Asn His
               340                 345                 350
   Lys Trp Ala His Trp Ala Phe Ala Pro Val Arg Leu Cys His Lys Leu
           355                 360                 365
   Met Thr Trp Leu Phe Pro Ala Arg Glu Ser Asn Val Asn Phe Gln Glu
       370                 375                 380
   Asp Gly Ile His Trp Tyr Ser Asn Met Leu Phe His Ile Gly Ser Trp
   385                 390                 395                 400
   Leu Leu Asp Arg Asp Ser Phe His Pro Leu Gly Ile Leu His Leu Ser
                   405                 410                 415
(2) INFORMATION FOR SEQ ID NO:19:
   (i) SEQUENCE CHARACTERISTICS:
      (A) LENGTH: 1416 base pairs
      (B) TYPE: nucleic acid
      (C) STRANDEDNESS: both
      (D) TOPOLOGY: linear
   (ii) MOLECULE TYPE: cDNA
   (ix) FEATURE:
      (A) NAME/KEY: CDS
      (B) LOCATION: 1..1413
(2) INFORMATION FOR SEQ ID NO:20:
   (i) SEQUENCE CHARACTERISTICS:
      (A) LENGTH: 471 amino acids
      (B) TYPE: amino acid
      (D) TOPOLOGY: linear
   (ii) MOLECULE TYPE: protein
   (xi) SEQUENCE DESCRIPTION: SEQ ID NO:20:
   Met Asp Asn His Ser Ser Val Pro Trp Ala Ser Ala Ala Ser Val Thr
   1               5                   10                  15
   Cys Leu Ser Leu Gly Cys Gln Met Pro Gln Phe Gln Phe Gln Phe Gln
               20                  25                  30
   Leu Gln Ile Arg Ser Glu Leu His Leu Arg Lys Pro Ala Arg Arg Thr
           35                  40                  45
   Gln Thr Met Arg His Ile Ala His Thr Gln Arg Cys Leu Ser Arg Leu
       50                  55                  60
   Thr Ser Leu Val Ala Leu Leu Leu Ile Val Leu Pro Met Val Phe Ser
   65                  70                  75                  80
   Pro Ala His Ser Cys Gly Pro Gly Arg Gly Leu Gly Arg His Arg Ala
                   85                  90                  95
   Arg Asn Leu Tyr Pro Leu Val Leu Lys Gln Thr Ile Pro Asn Leu Ser
               100                 105                 110
   Glu Tyr Thr Asn Ser Ala Ser Gly Pro Leu Glu Gly Val Ile Arg Arg
           115                 120                 125
   Asp Ser Pro Lys Phe Lys Asp Leu Val Pro Asn Tyr Asn Arg Asp Ile
       130                 135                 140
   Leu Phe Arg Asp Glu Glu Gly Thr Gly Ala Asp Gly Leu Met Ser Lys
   145                 150                 155                 160
   Arg Cys Lys Glu Lys Leu Asn Val Leu Ala Tyr Ser Val Met Asn Glu
                   165                 170                 175
   Trp Pro Gly Ile Arg Leu Leu Val Thr Glu Ser Trp Asp Glu Asp Tyr
               180                 185                 190
   His His Gly Gln Glu Ser Leu His Tyr Glu Gly Arg Ala Val Thr Ile
           195                 200                 205
   Ala Thr Ser Asp Arg Asp Gln Ser Lys Tyr Gly Met Leu Ala Arg Leu
       210                 215                 220
   Ala Val Glu Ala Gly Phe Asp Trp Val Ser Tyr Val Ser Arg Arg His
   225                 230                 235                 240
   Ile Tyr Cys Ser Val Lys Ser Asp Ser Ser Ile Ser Ser His Val His
                   245                 250                 255
   Gly Cys Phe Thr Pro Glu Ser Thr Ala Leu Leu Glu Ser Gly Val Arg
               260                 265                 270
   Lys Pro Leu Gly Glu Leu Ser Ile Gly Asp Arg Val Leu Ser Met Thr
           275                 280                 285
   Ala Asn Gly Gln Ala Val Tyr Ser Glu Val Ile Leu Phe Met Asp Arg
       290                 295                 300
   Asn Leu Glu Gln Met Gln Asn Phe Val Gln Leu His Thr Asp Gly Gly
   305                 310                 315                 320
   Ala Val Leu Thr Val Thr Pro Ala His Leu Val Ser Val Trp Gln Pro
                   325                 330                 335
   Glu Ser Gln Lys Leu Thr Phe Val Phe Ala His Arg Ile Glu Glu Lys
               340                 345                 350
   Asn Gln Val Leu Val Arg Asp Val Glu Thr Gly Glu Leu Arg Pro Gln
           355                 360                 365
   Arg Val Val Lys Leu Gly Ser Val Arg Ser Lys Gly Val Val Ala Pro
       370                 375                 380
   Leu Thr Arg Glu Gly Thr Ile Val Val Asn Ser Val Ala Ala Ser Cys
   385                 390                 395                 400
   Tyr Ala Val Ile Asn Ser Gln Ser Leu Ala His Trp Gly Leu Ala Pro
                   405                 410                 415
   Met Arg Leu Leu Ser Thr Leu Glu Ala Trp Leu Pro Ala Lys Glu Gln
               420                 425                 430
   Leu His Ser Ser Pro Lys Val Val Ser Ser Ala Gln Gln Gln Asn Gly
           435                 440                 445
   Ile His Trp Tyr Ala Asn Ala Leu Tyr Lys Val Lys Asp Tyr Val Leu
       450                 455                 460
   Pro Gln Ser Trp Arg His Asp
   465                 470
(2) INFORMATION FOR SEQ ID NO:21:
   (i) SEQUENCE CHARACTERISTICS:
      (A) LENGTH: 221 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:21:
   Cys Gly Pro Gly Arg Gly Xaa Gly Xaa Arg Arg His Pro Lys Lys Leu
   1               5                   10                  15
   Thr Pro Leu Ala Tyr Lys Gln Phe Ile Pro Asn Val Ala Glu Lys Thr
               20                  25                  30
   Leu Gly Ala Ser Gly Arg Tyr Glu Gly Lys Ile Xaa Arg Asn Ser Glu
           35                  40                  45
   Arg Phe Lys Glu Leu Thr Pro Asn Tyr Asn Pro Asp Ile Ile Phe Lys
       50                  55                  60
   Asp Glu Glu Asn Thr Gly Ala Asp Arg Leu Met Thr Gln Arg Cys Lys
   65                  70                  75                  80
   Asp Lys Leu Asn Xaa Leu Ala Ile Ser Val Met Asn Xaa Trp Pro Gly
                   85                  90                  95
   Val Xaa Leu Arg Val Thr Glu Gly Trp Asp Glu Asp Gly His His Xaa
               100                 105                 110
   Glu Glu Ser Leu His Tyr Glu Gly Arg Ala Val Asp Ile Thr Thr Ser
           115                 120                 125
   Asp Arg Asp Xaa Ser Lys Tyr Gly Xaa Leu Xaa Arg Leu Ala Val Glu
       130                 135                 140
   Ala Gly Phe Asp Trp Val Tyr Tyr Glu Ser Lys Ala His Ile His Cys
   145                 150                 155                 160
   Ser Val Lys Ala Glu Asn Ser Val Ala Ala Lys Ser Gly Gly Cys Phe
                   165                 170                 175
   Pro Gly Ser Ala Xaa Val Xaa Leu Xaa Xaa Gly Gly Xaa Lys Xaa Val
               180                 185                 190
   Lys Asp Leu Xaa Pro Gly Asp Xaa Val Leu Ala Ala Asp Xaa Xaa Gly
           195                 200                 205
   Xaa Leu Xaa Xaa Ser Asp Phe Xaa Xaa Phe Xaa Asp Arg
       210                 215                 220
(2) INFORMATION FOR SEQ ID NO:22:
   (i) SEQUENCE CHARACTERISTICS:
      (A) LENGTH: 167 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:22:
   Cys Gly Pro Gly Arg Gly Xaa Xaa Xaa Arg Arg Xaa Xaa Xaa Pro Lys
   1               5                   10                  15
   Xaa Leu Xaa Pro Leu Xaa Tyr Lys Gln Phe Xaa Pro Xaa Xaa Xaa Glu
               20                  25                  30
   Xaa Thr Leu Gly Ala Ser Gly Xaa Xaa Glu Gly Xaa Xaa Xaa Arg Xaa
           35                  40                  45
   Ser Glu Arg Phe Xaa Xaa Leu Thr Pro Asn Tyr Asn Pro Asp Ile Ile
       50                  55                  60
   Phe Lys Asp Glu Glu Asn Xaa Gly Ala Asp Arg Leu Met Thr Xaa Arg
   65                  70                  75                  80
   Cys Lys Xaa Xaa Xaa Asn Xaa Leu Ala Ile Ser Val Met Asn Xaa Trp
                   85                  90                  95
   Pro Gly Val Xaa Leu Arg Val Thr Glu Gly Xaa Asp Glu Asp Gly His
               100                 105                 110
   His Xaa Xaa Xaa Ser Leu His Tyr Glu Gly Arg Ala Xaa Asp Ile Thr
           115                 120                 125
   Thr Ser Asp Arg Asp Xaa Xaa Lys Tyr Gly Xaa Leu Xaa Arg Leu Ala
       130                 135                 140
   Val Glu Ala Gly Phe Asp Trp Val Tyr Tyr Glu Ser Xaa Xaa His Xaa
   145                 150                 155                 160
   His Xaa Ser Val Lys Xaa Xaa
                   165

## Description

### Background of the Invention

The excessive or inappropriate stimulation of excitatory amino acid receptors can lead to neuronal cell damage or loss by way of a mechanism known as excitotoxicity. For instance, excitotoxic action may be responsible for neuronal loss in stroke, cerebral palsy, epilepsy, ageing and Alzheimer's disease, Huntington's disease, and other chronic degenerative disorders. The medical consequences of such neuronal degeneration makes the abatement of these degenerative neurological processes an important therapeutic goal.

The development of neuroprotective agents for the prevention of neuronal loss in acute conditions such as stroke and epilepsy or chronic neurodegenerative disorders including Parkinson's disease, Alzheimer's disease, Huntington's chorea, and motor neuron disease has in fact focused on drugs that inhibit excitatory amino acid neurotransmission or exhibit antioxidant properties. Unfortunately, potent antagonists of the N-methyl-D-aspartate (NMDA) type glutamate receptor, which is thought to mediate excitotoxic neuronal injury, e.g., MK-801 or phencyclidine (PCP), share a high probability of inducing psychotomimetic side effects. Further, these drugs have been associated with acute neurotoxicity in vitro and in vivo, precluding their clinical use.

It is a goal of the present invention to provide compositions and methods for preventing or ameliorating neuronal degeneration, e.g., for the abatement of these degenerative neurological processes.

### Summary of the Invention

One aspect of the present application relates to the use of a lipophilic modified hedgehog polypeptide in the preparation of a medicament for treatment or prophylaxis of a disorder selected from the group consisting of domoic acid poisoning; spinal cord trauma; hypoglycemia; mechanical trauma to the nervous system; senile dementia; Korsakoff's disease; schizophrenia; AIDS dementia, multi-infarct dementia; mood disorders; depression; chemical toxicity; neuronal damage associated with uncontrolled seizures, such as epileptic seizures; neuronal injury associated with HIV and AIDS; neurodegeneration associated with Down's syndrome; neuropathic pain syndrome; olivopontocerebral atrophy; amyotrophic lateral sclerosis; mitochondrial abnormalities; Alzheimer's disease; hepatic encephalopathy; Tourette's syndrome; schizophrenia; and drug addiction, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

A further aspect of the present application relates to the use of a lipophilic modified hedgehog polypeptide in the preparation of a medicament for treating a disorder characterized by loss of dopaminergic and/or GABAergic neurons, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

In other embodiments, the present application relates to the use of a lipophilic modified hedgehog polypeptide in the preparation of a medicament for treating or preventing Parkinson's disease or Huntington's disease, wherein said hedgehog polypeptide comprises an amino acid sequence encodable by a nucleic acid sequence that hybridises under stringent conditions to a nucleic acid sequence designated in at least one of SEQ ID Nos: 1-9, and wherein said lipophilic modification comprises at least one fatty acid moiety on one or more internal sites or on an N-terminal amino acid residue of the mature hedgehog polypeptide.

In other embodiments, the present application relates to a therapeutic preparation of a lipophilic modified hedgehog polypeptide provided in a pharmaceutically acceptable carrier and in an amount sufficient to promote survival of dopaminergic cells in an adult mammal, wherein said hedgehog polypeptide comprises an amino acid sequence encodable by a nucleic acid sequence that hybridises under stringent conditions to a nucleic acid sequence designated in at least one of SEQ ID NO: 1-9, and wherein said lipophilic modification comprises at least one fatty acid moiety on one or more internal sites or on an N-terminal amino acid residue of the mature hedgehog polypeptide.

In certain embodiments, the present application relates to a therapeutic preparation of a lipophilic modified hedgehog polypeptide provided in a pharmaceutically acceptable carrier and in an amount sufficient to treat or prophylactically treat Parkinson's disease or Huntington's disease in an adult mammal, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

The subject method is carried out using a *hedgehog* therapeutic, wherein the *hedgehog* portion has an amino acid sequence at least 60, 75, 85, 95 or 100 percent identical with a *hedgehog* polypeptide of any of SEQ ID Nos. 10-18 or 20. The *hedgehog* portion can be encoded by a nucleic acid which hybridizes under stringent conditions to a nucleic acid sequence of any of SEQ ID Nos. 1-9 or 19, e.g., the *hedgehog* portion can be encoded by a vertebrate *hedgehog* gene, especially a human *hedgehog* gene.

The *hedgehog* polypeptides of the present invention are modified by a lipophilic moiety or moieties at one or more internal sites of the mature, processed extracellular domain, and may or may not be also derivatized with lipophilic moieties at the N or C-terminal residues of the mature polypeptide. In still other embodiments, the polypeptide is modified at the N-terminal residue with a cyclic (preferably polycyclic) lipophilic group. Various combinations of the above are also contemplated.

In other embodiments, the subject method can be carried out by administering a gene activation construct, wherein the gene activation construct is deigned to recombine with a genomic *hedgehog* gene of the patient to provide a heterologous transcriptional regulatory sequence operatively linked to a coding sequence of the *hedgehog* gene.

In still other embodiments, the subject method can be practiced with the administration of a gene therapy construct encoding a *hedgehog* polypeptide. For instance, the gene therapy construct can be provided in a composition selected from a group consisting of a recombinant viral particle, a liposome, and a poly-cationic nucleic acid binding agent,

In certain embodiments, the polypeptide is purified to at least 80% by dry weight, and more preferably 90 or 95% by dry weight.

In other preferred embodiments, the polypeptide has a *hedgehog* amino acid sequence encoded by a nucleic acid which hybridizes under stringent conditions to a nucleic acid sequence selected from the group consisting of SEQ ID No:1-9, which *hedgehog* amino acid sequence of the polypeptide corresponds to a natural proteolytic product of a *hedgehog* polypeptide. Such polypeptides preferably (i) binds to a *patched* protein, (ii) regulates differentiation of neuronal cells, (iii) regulates survival of differentiated neuronal cells, (iv) regulates proliferation of chondrocytes, (v) regulates proliferation of testicular germ line cells, and/or (vi) functionally replaces drosopholia *hedgehog* in transgenic drosophila fly, or a combination thereof.

In preferred embodiments, the nucleic acid encodes a *hedgehog* amino acid sequence identical to a *hedgehog* polypeptide selected from the group consisting of SEQ ID No: 10-18.

Another aspect of the present invention provides a probe/primer comprising a substantially purified oligonucleotide, said oligonucleotide containing a region of nucleotide sequence which hybridizes under stringent conditions to at least 10 consecutive nucleotides of sense or antisense sequence of SEQ ID No. 1-9, or naturally occuring mutants thereof. In preferred embodiments, the probe/primer includes a label group attached thereto and able to be detected.

### Brief Description of the Drawings

Figure 1. Shh and *Ptc* in the E14.5 rat embryo. *Shh* (A, antisense; B, sense control), and *ptc* (C, antisense; D, sense control) expression as detected by in situ hybridization with digoxigenin-labeled riboprobes and alkaline phosphataseconjugated anti-digoxigenin. The arrow in A and the double-arrow in C designate the zona limitan intrathalamica. Major anatomical structures and summary diagrams of *shh and ptc* expression are shown in E. Scale bar = 1 mm.
Figure 2. Shh promotes the survival of TH+ neurons of the ventral mesencephalon.
(A) Timecourse and dose response of the Shh effect. The number of TH+ neurons in control cultures (O ng/ml Shh) began to decline dramatically by 5 days *in vitro.* In cultures treated with Shh at 25 and 50 ng/ml there were significantly greater numbers of TH+ nurrons over control- through 24 days *in vitro* (from 5 to 24 days, p <.001 at 25 and 50 ng/ml). The 50 ng/ml dose typically gave a 50-100% increase over controls at all time points (error bars s.e.m.)P-h'otomicrographs of TH+ neurons in 50.ng/ml Shh treated (B-,,,--D) and control (C, E) cultures, 2 days (B, C) and 7 days (D, E) post-plating. Note that in addition to an increased number of TH+-cell bodies, the Shh treated cells--show extensive neuritic processes. Scale bar = 200 um.

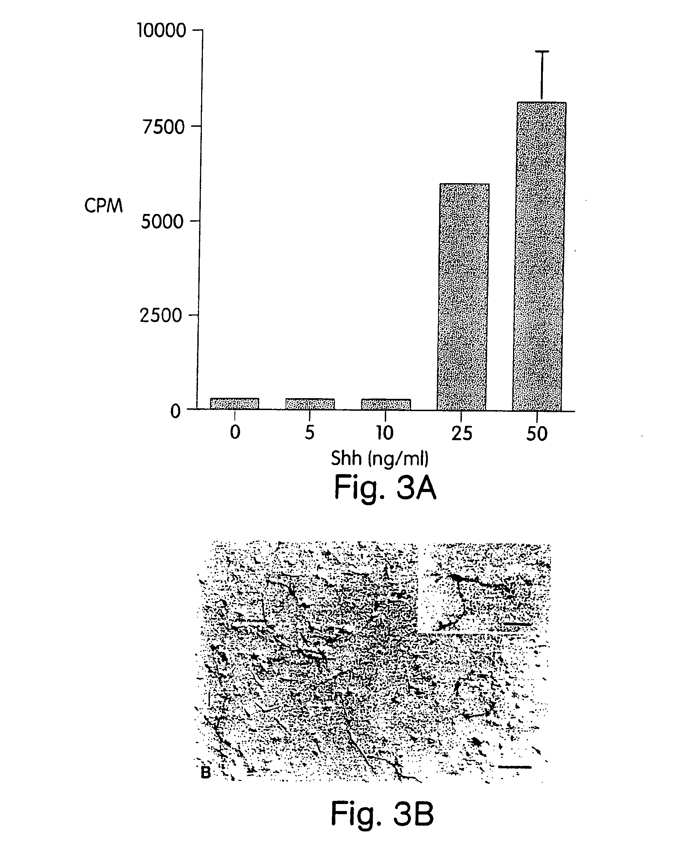

Figure 3. Transport of 3H-Dopamine. The identity and functionality of the surviving midbrain neurons was assessed by their ability to specifically transport dopamine. (A) Addition of 25 ng/ml Shh resulted in a 22-fold increase in 3H-DA cell uptake over controls and lower Shh concentrations. 50 ng/ml Shh gave a 30-fold increase in 3H-DA uptake (error bars = s.d.) (p < 0.005 at 25 and 50 ng/ml). (B) Autoradiography was performed on sister plates to visualize dopamine transport. Only cells with neuronal morphology transported 3H-DA (inset). Scale bar = 50 Jim, inset 15 m.

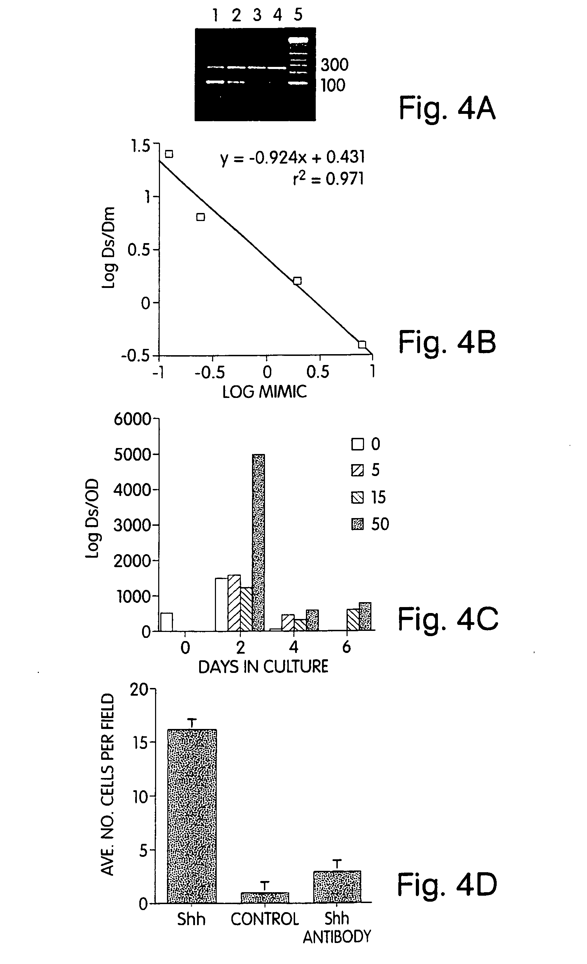

Figure 4. Specificity of Shh activity. (A) QC-PCR gel. Lanes 1-4 are CDNA from midbrain cultures that have been co-amplified with successive 4-fold dilutions of mimic oligo. Lane 5 is DNA marker lane. *Ptc* target is 254 bp and mimic is 100 bp(B) Representative plot (corresponding to A) of the log concentration of competitive mimic versus the log of the obtained band densities of target and mimic PCR substrates demonstrates the linearity of the amplification reaction. The extrapolated value of *ptc* message in the CDNA tested is determined to be equal to the value of mimic concentration where Log Ds/Dm = 0. See main text for details of the procedure. Doses in ng/ml; Ds = density of test substrate; Dm = density of competitive mimic. The r 2 value shows that determinations made within this range vary within 3%. (C) Administration of Shh induces *ptc* expression in a dose response that parallels the survival curve. The values are expressed as number of target molecules (log Ds) per total amount of CDNA used in each reaction as measured by optical density at 260 nm (OD) and were determined as demonstrated in A and B. At 4 days *in vitro* Shh at 5 ng/ml increases *ptc* expression over control, and 50 ng/ml increases expression of *ptc* over the level found in the ventral mesencephalon at the time of dissection. (D) Affinity purified anti-Shh antibody inhibited the Shh neurotrophic response (p < .001). Cultures were maintained for 5 days. Shh was added at a concentration of 50 ng/ml, and in the co-administration of 5 Shh and anti-Shh ("Shh antibody") Shh-was added at 0 g/ml and anti-Shh was added as a 5-fold molar excess (error bars s.e.m.).

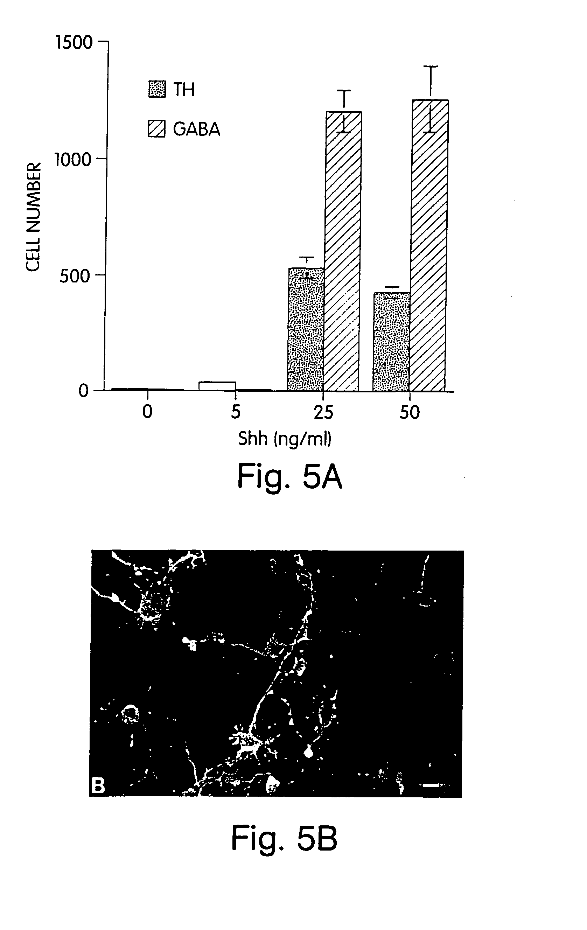

Figure 5. Shh also supports the survival of midbrain-GABA+ neurons. (A) In addition to supporting the survival of TH+ cells in the midbrain cultures, Shh promotes the survival of GABA-immunoreactive neurons with a similar dose response (error bars=s.e.m.) (For TH, p , 0.001 at 25 and 50 ng/ml; for GABA, p < .001 at 25 and 50 ng/ml). (B) Double level immunofluorescence of SSH-treated cultures shows that the majority of the GABA+ cells (Orange) do not overlap with the TH+ cells (green); scale bar = 15 m.

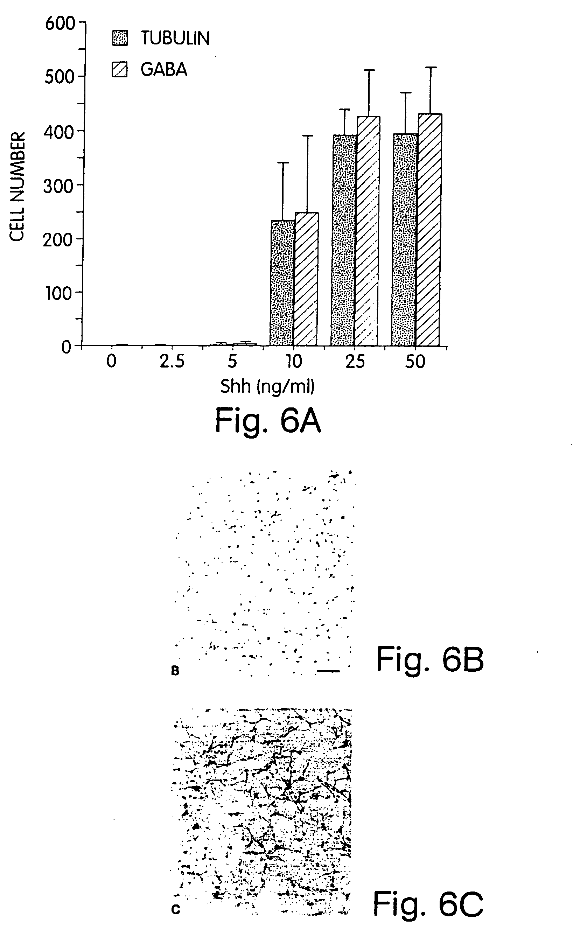

Figure 6. Shh effects on striatal cultures. (A) At concentrations of 10 ng/ml and higher, Shh promotes neuronal survival as gauged by staining for tubulin PIII, and these cells are exclusively GABA+ (error bars = S.D.) (tubulin PIII, p < 0.001 at 25 and 50 ng/ml; GABA, p < .001 at 25 and 50 ng/ml). Typical fields of neurons treated with 50 ng/ml Shh stained for tubulin plll (B) and GABA+ (C) are shown; scale bar = 100 gm.

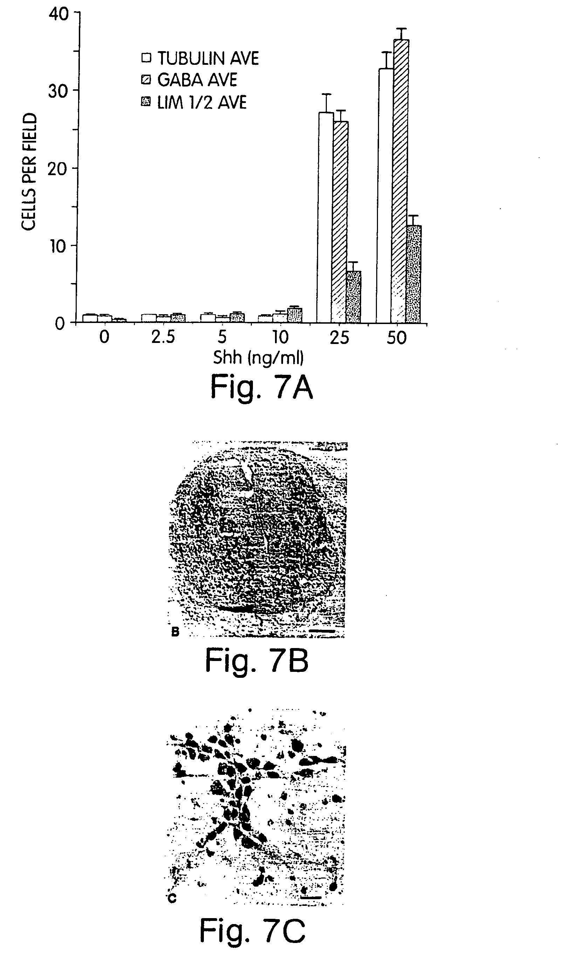

Figure 7. Shh effects on ventral spinal cultures. (A) At concentrations of 25 ng/ml and higher, Shh promotes neuronal survival as gauged by staining for tubulin PIII. The majority of the cells stain positively for GABA, while a subset stain for the nuclear marker of spinal intemeurons, Lim-1/2 (error bars = s.e.m.) (tubulin pill, p < 0.001 at 25 and 50 ng/ml; lim 1/2, p <.001 at 5,10,25, and 50 ng/ml; GABA, p < .001 at 25 and 50 ng/ml). Typical staining for Lim- - 1/2 in the E14 rat spinal cord (B, scale bar = 100 m), and spinal neurons cultured in the presence of 50 ng/ml Shh (C, scale bar = 20 m).

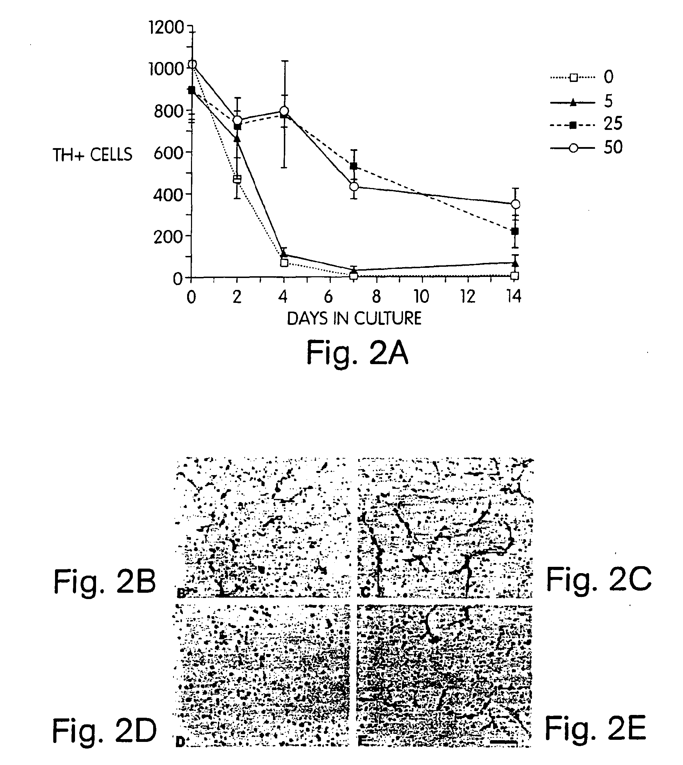

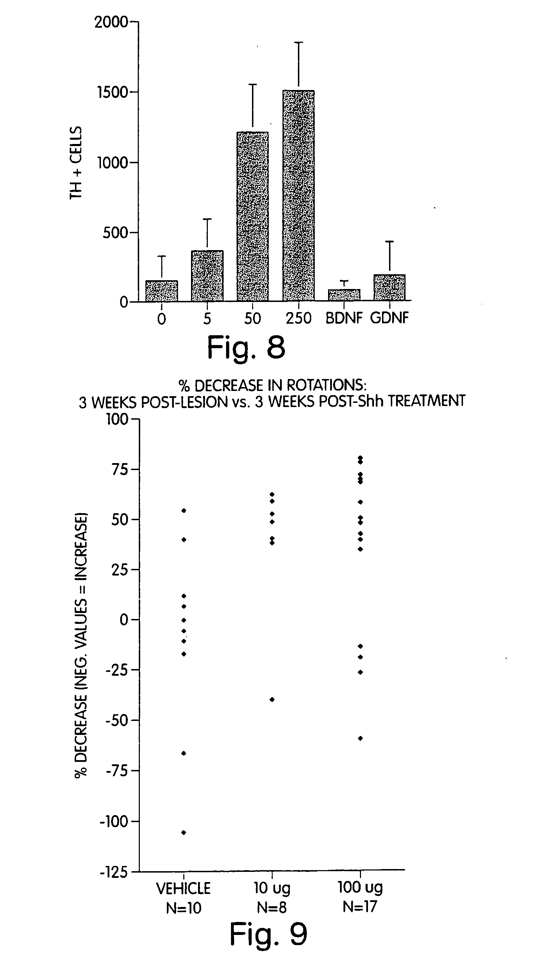

Figure 8. *Shh* protects midbrain TH+ neurons from neurotoxic insult. Cultures of ventral mesencephalon neurons were cultured in the indicated concentrations of *Shh* (ng/ml). MPP+ was added at 4 days *in vitro* for 48 hours. Cultures were then washed extensively and cultured for an additional 48 hours to allow clearance of dying neurons. Protection from MPP+ neurotoxicity could be seen at 5 ng/ml, with the effect saturating at 50 ng/ml. BDNF was used at 10 ng/ml, and GDNF at 20 ng/ml (error bars = s.e.m.) (*Shh,* p < 0.001 at 50 and 250 ng/ml; BDNF no significance; GDNF, p < .05). Note that the plating density used in this experiment was twice that used in Figure 2.

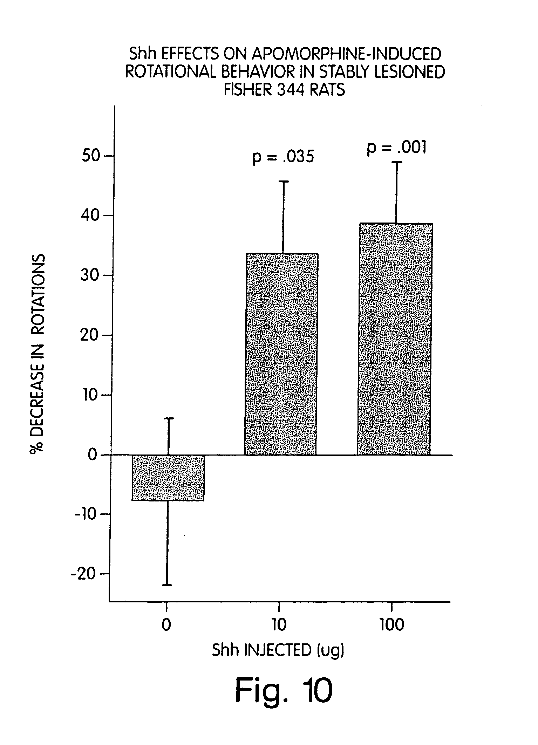

Figures 9 and 10 are graphs illustrating the effects of *Shh* on apomorphine-induced rotational behavior in stably lesioned mice.

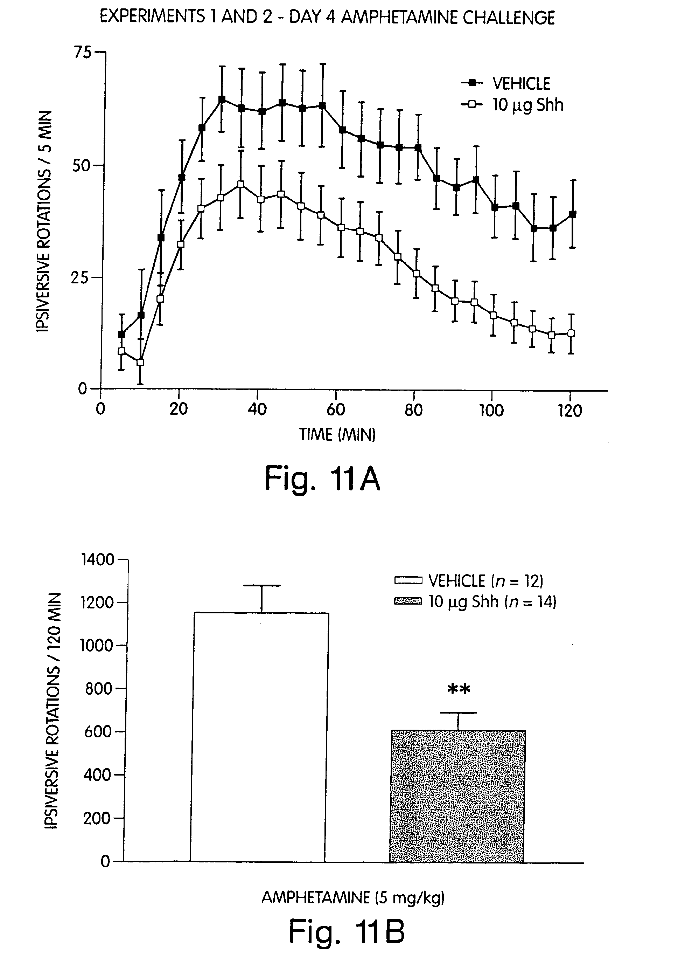

Figures 11A and 11B are graphs illustrating the neuroprotective effect of *Shh* against amphetamine challenges.

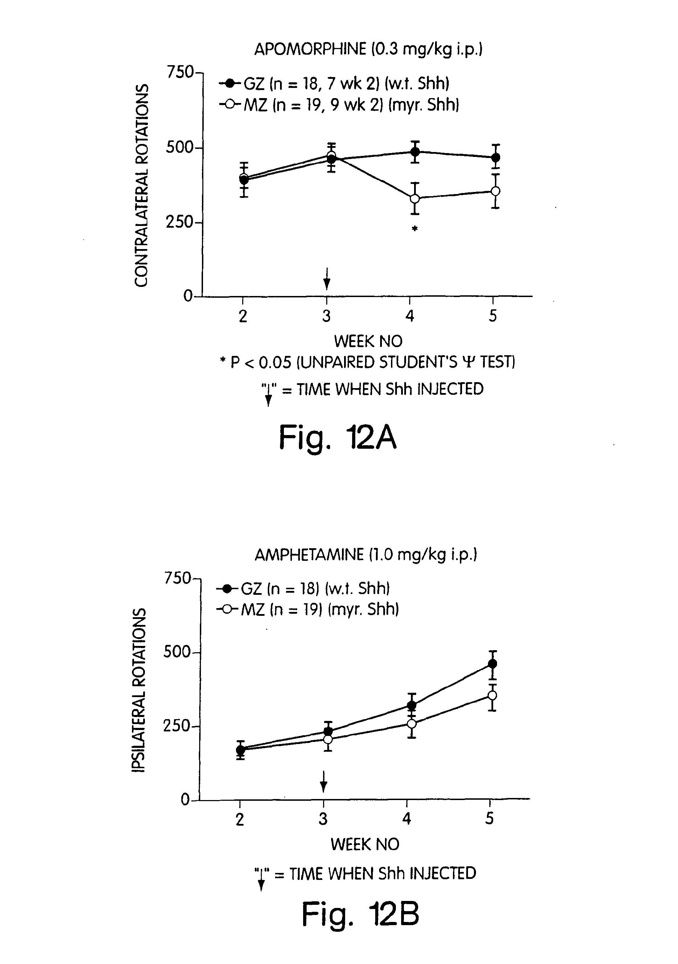

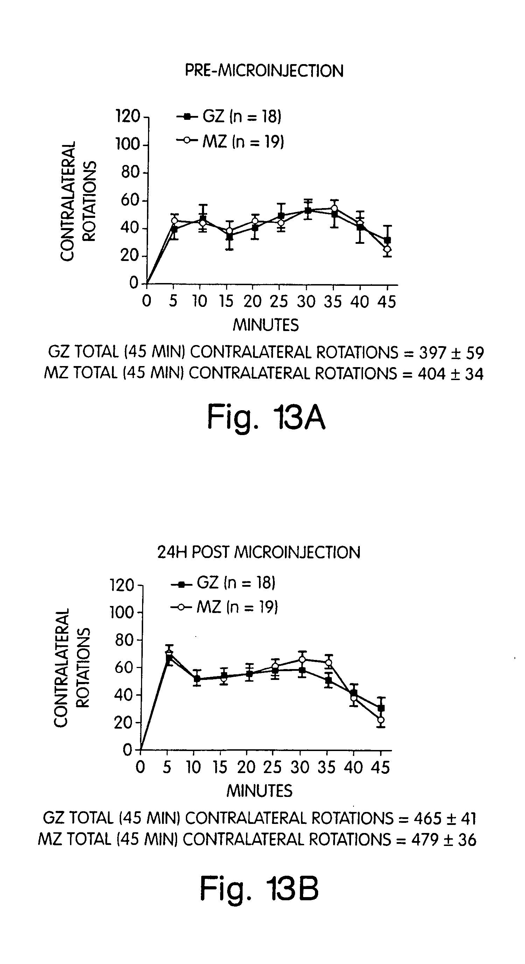

Figures 12A-B and 13A-D are graphs comparing the restorative effect of myristoylated *Shh* (Mz) and un-modified forms of Shh (Gz) on the rotational behavior of 6-OHDA lesioned mice.

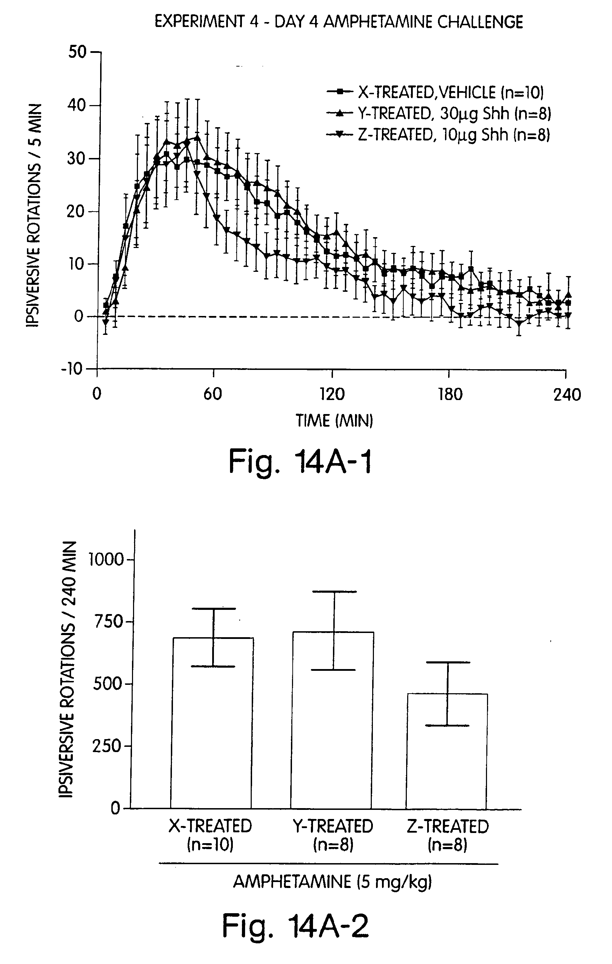

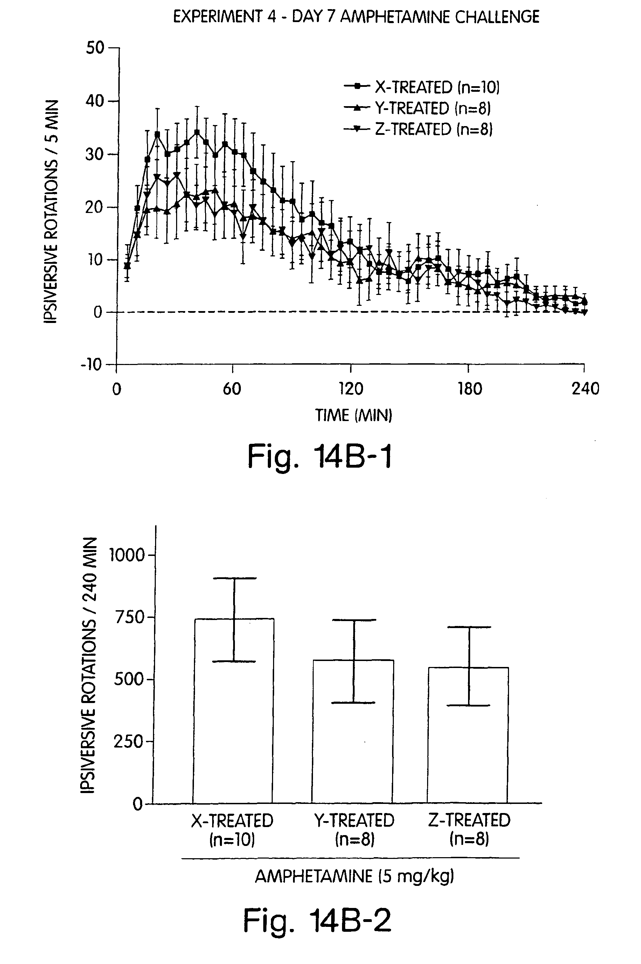

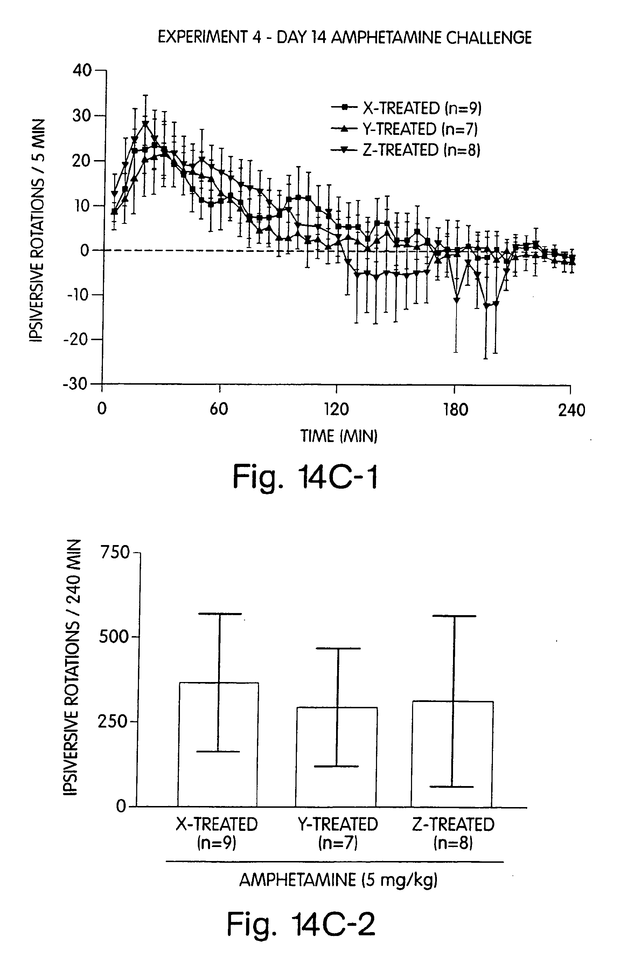

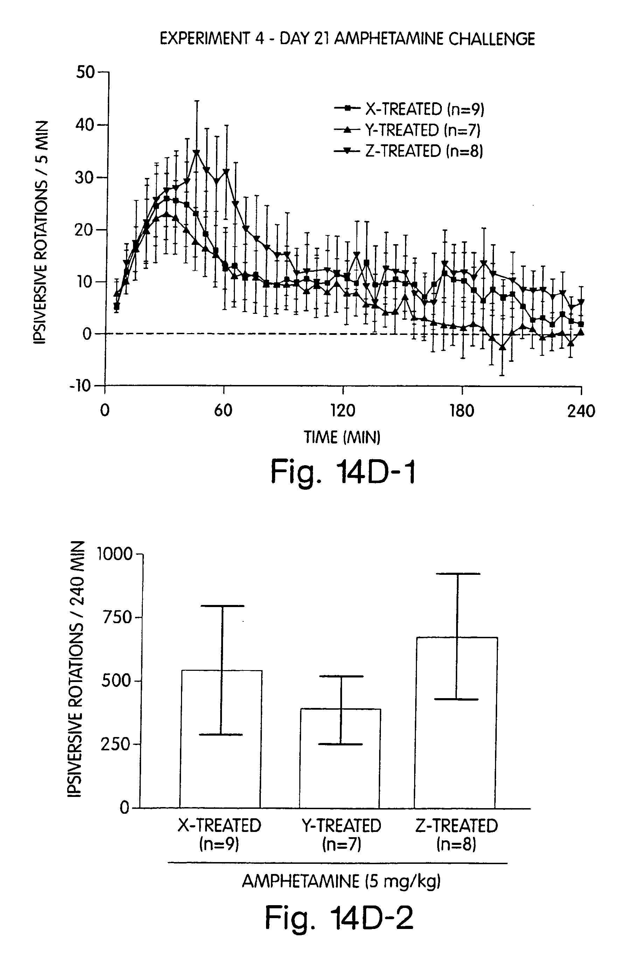

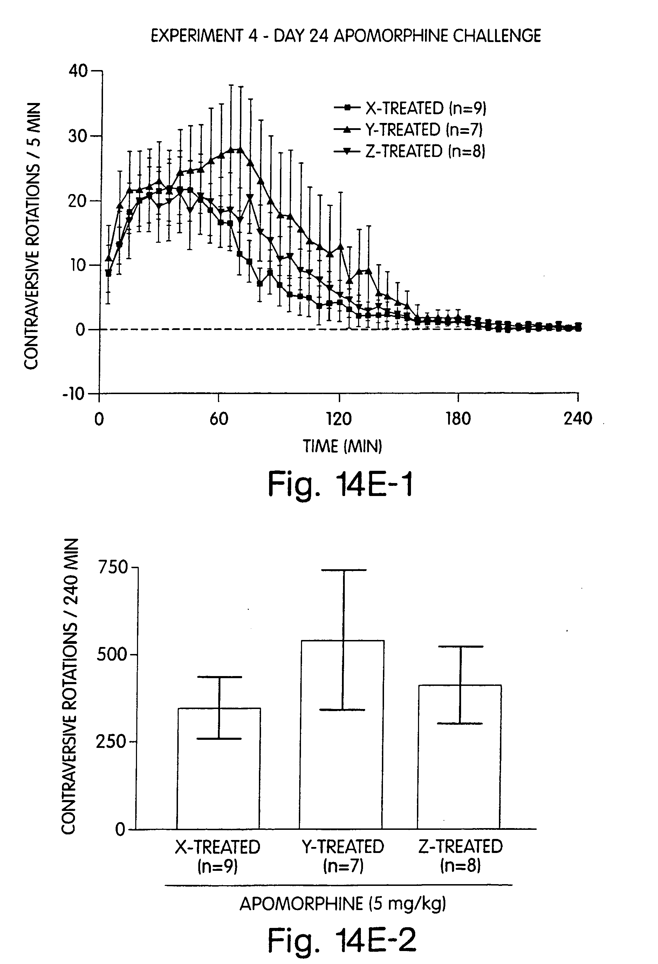

Figures 14A-E, 15A-H and 16 are graphs illustrating the neuroprotective effect of *Shh* polypeptides against amphetamine and apomorphine challenges of 6-OHDA lesioned mice.

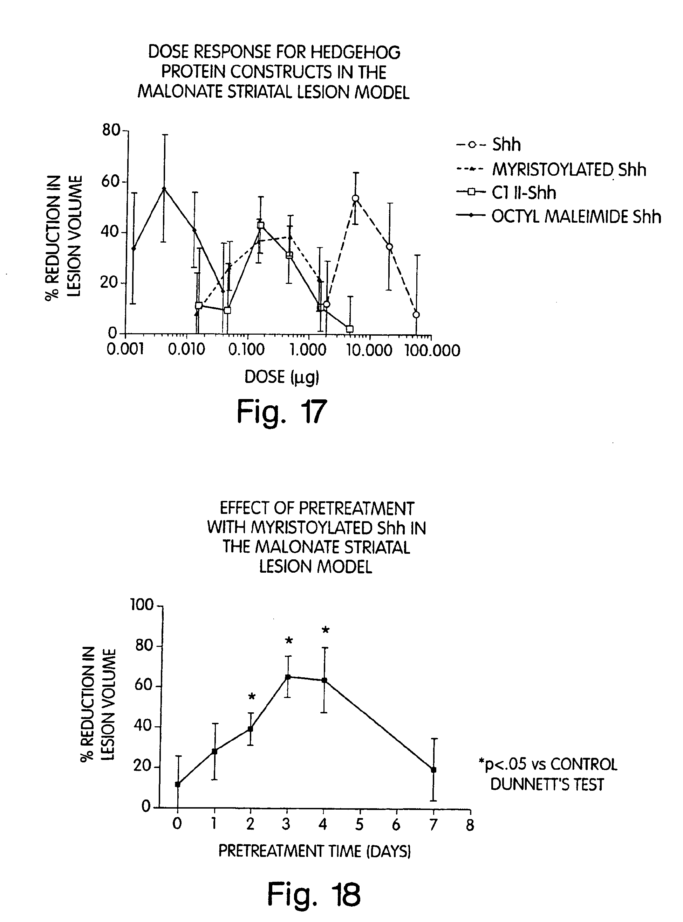

Figure 17 is a graph demonstrating the dose response for *hedgehog* polypeptide constructs in a malonate striatal lesion model.
Figure 18 is a graph showing the effect of pretreatment with myristoylated Shh in a malonate striatal lesion model.

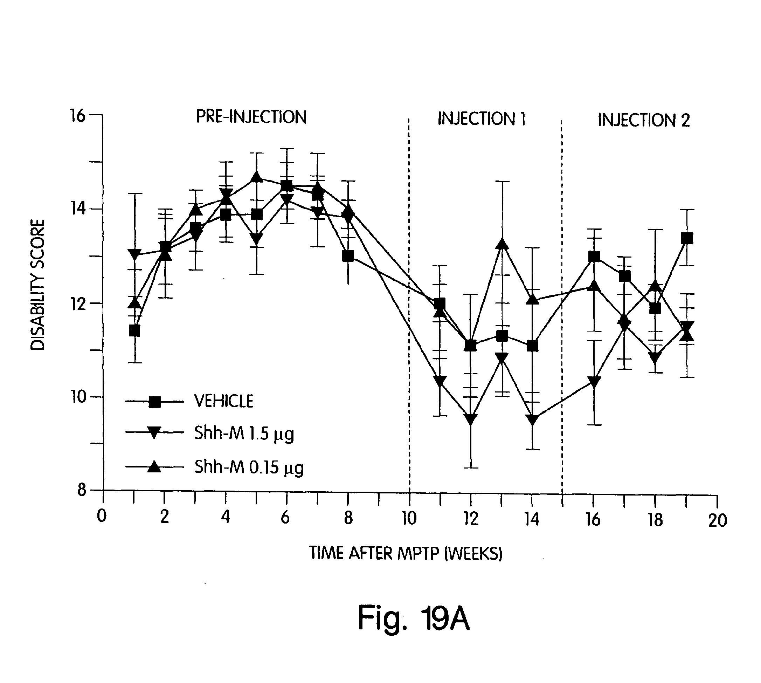

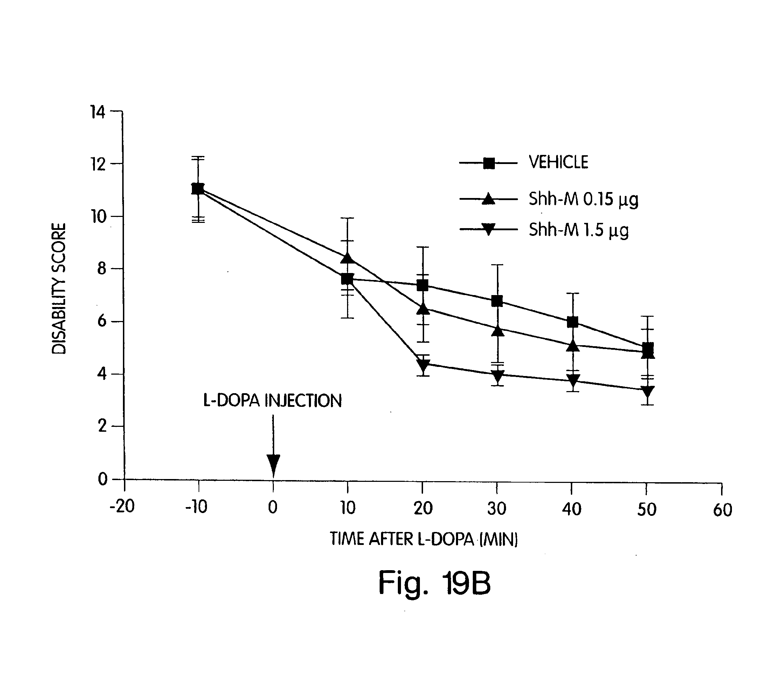

Figures 19A and 19B are graphs illustrating that intranigral injection of myristoylated Sonic Hedgehog (Shh-M) reduces parkinsonian disability and increases the therapeutic effect of L-DOPA in MPTP-treated marmosets.

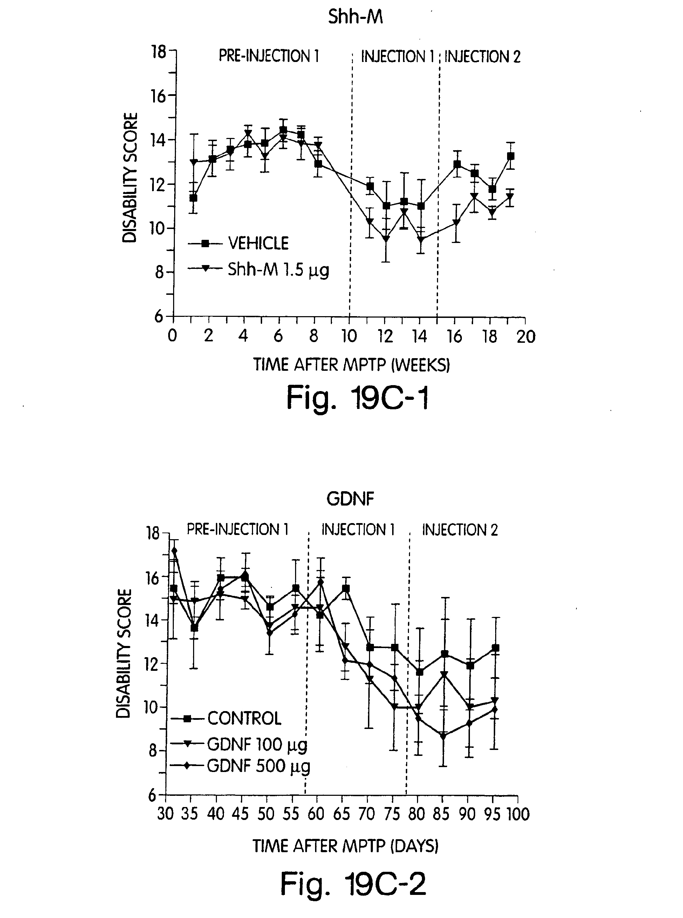

Figure 19C compares the activity of hedgehog polypeptides with GDNF.

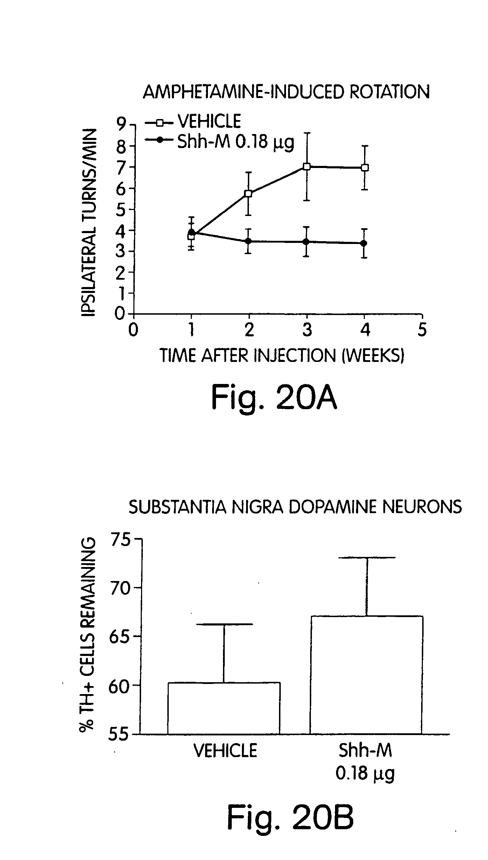

Figure 20A and 20B are graphs illustrating the protective effect of Hedgehog polypeptides against amphetamine challenges of 6-OHDA lesioned mice.

### Detailed Description of the Invention

### I. Overview

Sonic *hedgehog* (*Shh*), an axis-determining secreted protein, is expressed during early vertebrate embryogenesis in the notochord and ventral neural tube. In this site it plays a role in the phenotypic specification of ventral neurons along the length of the CNS. For example, *Shh* induces the differentiation of motor neurons in the spinal cord and dopaminergic neurons in the midbrain. *Shh* expression, however, persists beyond this induction period. We have show here that *Shh* possesses novel activities beyond phenotype specification.

Using cultures derived from the embryonic day 14.5 (E14.5) rat ventral mesencephalon, we show that *Shh* is also trophic for dopaminergic neurons. Interestingly, *Shh* not only promotes dopaminergic neuron survival, but also promotes the survival of midbrain GABA-immunoreactive (GABA-ir) neurons. In cultures derived from the E15-16 striatum, *Shh* promotes the survival of GABA-ir intemeurons to the exclusion of any other cell type. Cultures derived from E15-16 ventral spinal cord reveal that *Shh* is again trophic for intemeurons, many of which are GABA-ir and some of which express the Lim-1/2 nuclear marker, but does not appear to support motomeuron survival. *Shh* does not support survival of sympathetic or dorsal root ganglion neurons. Finally, using the midbrain cultures, we show that in the presence of MPP+, a highly specific neurotoxin, *Shh* prevents dopaminergic neuron death that normally would have occurred.

Moreover, as demonstrated in the appended examples, activation of *hedgehog* signalling pathway(s) can be used both for restoration of lesions to dopaminergic cells and other neuronal cells of the substantia nigra (SN) and ventral termental area (VTA), as well as for neuroprotection against formation of such lesions. Briefly, as described in the appended examples, we investigated the neuroprotective and restorative potential of *hedgehog* polypeptides in various animal models of lesions involving nigrostriatal and mesolimbic dopaminergic pathways. As described in detail below, our results show that the administration of *hedgehog* polypeptide can prevent and partially restore such neuronal damage.

Furthermore, as demonstrated in the appended examples, activation of the *hedgehog* signaling pathway(s) can be a more general protective measure against neurotoxic insult, and particularly for protection against cell death due to overstimulation by excitatory amino acids.

One aspect of the present application is directed to the use of a lipophilic modified 44 for the prevention and treatment of degenerations of certain neuronal cells due to exotoxicity. The invention also specifically contemplates the use of *hedgehog* agonists for treating, preventing or ameliorating neuronal loss associated with neurologic disorders which are due to overstimulation by the excitatory amino acids. These include acute neurologic disorders such as domoic acid poisoning; spinal cord trauma; hypoglycemia; mechanical trauma to the nervous system, epileptic seizures; and chronic neurologic disorders such as Huntington's disease, neuronal injury associated with HIV and AIDS, AIDS dementia, neurodegeneration associated with Down's syndrome, neuropathic pain syndrome, olivopontocerebral atrophy, amyotrophic lateral sclerosis, mitochondrial abnormalities, Alzheimer's disease, hepatic encephalopathy, Tourette's syndrome, schizophrenia, and drug addiction (see Lipton and Rosenberg, N. Engl. J. Med. 330: 613-622 (1994)).

In certain preferred embodiments, the subject invention is directed to the use of a lipophilic modified 44 for preventing or ameliorating degenration of dopaminergic cells and other neuronal cells of the substantia nigra (SN) and ventral termental area (VTA), such as resulting in Parkinson's disease. Based in part on these findings, we have determined that *Shh,* and other forms of *hedgehog* polypeptides, are useful as a protective agents in the treatment and prophylaxis for neurodegenerative disorders, particularly those resulting from the loss of dopaminergic and/or GABA-nergic neurons, or the general loss tissue from the substantia nigra. As described with greater detail below, exemplary disorders ("candidate disorders") include Parkinson's disease.

In one aspect, the present invention provides pharmaceutical preparations and methods for preventing/treating lesions involving exotoxic-dependent degeneration of neurons utilizing, as an active ingredient, a lipophilic modified *hedgehog* polypeptide.

However, without wishing to be bound by any particular theory, the protective/restorative activity of *hedgehog* polypeptides obeserved in the present studies may be due at least in part to the ability of *hedgehog* polypeptides to antagonize (directly or indirectly) *patched*-mediated regulation of gene expression and other physiological effects mediated by the *patched* gene. The *patched* gene product, a cell surface protein, is understood to signal through a pathway which regulates transcription of a variety of genes involved in neuronal cell development. In the CNS and other tissue, the introduction of *hedgehog* relieves (derepresses) this inhibition conferred by *patched,* allowing expression of particular gene programs. Accordingly, the present invention contemplates the use of other agents which are capable of mimicking the effect of the *hedgehog* polypeptide on *patched* signalling, e.g., as may be identified from the drug screening assays described below.

The subject *hedgehog* treatments are effective on both human and animal subjects afflicted with these conditions. Animal subjects to which the invention is applicable extend to both domestic animals and livestock, raised either as pets or for commercial purposes. Examples are dogs, cats, cattle, horses, sheep, hogs and goats. In terms of treatment, once a patient experiences symptoms of a candidate disorder, a goal of therapy is prevention of further loss of neuron function.

### II. Definitions

For convience, certain terms employed in the specfication, examples, and appended claims are collected here.

The term *"hedgehog* therapeutic" refers to various forms of *hedgehog* polypeptides, as well as peptidomimetics, which are neuroprotective for neuronal cells, and in particular, enhance the survival of dopaminergic and GABA-ergic neurons. These include naturally occurring forms of *hedgehog* polypeptides, as well as modified or mutant forms generated by molecular biological techniques, chemical synthesis, etc. While in preferred embodiments the *hedgehog* polypeptide is derived from a vertebrate homolog, cross-sepcies activity reported in the literature supports the use of *hedgehog* peolypeptides from invertebrate organisms as well. Naturally and non-naturally occurring *hedgehog* therapeutics referred to herein as "agonists" mimic or potentiate (collectively "agonize") the effects of a naturally-occurring *hedgehog* polypeptide as a neuroprotective agent. In addition, the term "*hedgehog* therapeutic" includes molecules which can activate expression of an endogenous *hedgehog* gene. The term also includes gene therapy constructs for causing expression of *hedgehog* polypeptides *in vivo,* as for example, expression constructs encoding recombinant *hedgehog* polypeptides as well as trans-activation constructs for altering the regulatory sequences of an endogenous *hedgehog* gene by homologous recombination.

In particular, the term "*hedgehog* polypeptide" encompasses *hedgehog* polypeptides and peptidyl fragments thereof.

As used herein the term "bioactive fragment", with reference to a portions of *hedgehog* polypeptides, refers to a fragment of a full-length *hedgehog* polypeptide, wherein the fragment specifically agonizes neuroprotective events mediated by wild-type *hedgehog* polypeptides. The *hedgehog* bioactive fragment preferably is a soluble extracellular portion of a *hedgehog* polypeptide, where solubility is with reference to physiologically compatible solutions. Exemplary bioactive fragments are described in PCT publications WO 95/18856 and WO 96/17924.

The term "*ptc* therapeutic" refers to agents which mimic the effect of naturally occurring *hedgehog* polypeptides on *patched* signalling. The *ptc* therapeutic can be, e.g., a peptide, a nucleic acid, a carbohydrate, a small organic molecule, or natural product extract (or fraction thereof).

As used herein, the term "antibody homolog" includes intact antibodies consisting of immunoglobulin light and heavy chains linked via disulfide bonds. The term "antibody homolog" is also intended to encompass a protein comprising one or more polypeptides selected from immunoglobulin heavy chains and antigen-binding fragments thereof which are capable of binding to one or more antigens (i.e., hedgehog or patched). The component polypeptides of an antibody homolog composed of more than one polypeptide may optionally be disulfide-bound or otherwise covalently crosslinked. Accordingly, therefore, "antibody homologs" include intact immunoglobulins of types IgA, IgG, IgE, IgD, IgM (as well as subtypes thereof), wherein the light chains of the immunoglobulin may be of types kappa or lambda. Preferred proteins of the invention may include portions of intact antibodies that retain antigen-binding specificity, for example, Fab fragments, Fab' fragments, F(ab')2 fragments, F(v) fragments, heavy chain monomers or dimers, light chain monomers or dimers, dimers consisting of one heavy and one light chain, and the like.

As used herein, a "humanized antibody homolog" is an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain. A "human antibody homolog" is an antibody homolog in which all the amino acids of an immunoglobulin light or heavy chain (regardless of whether or not they are required for antigen binding) are derived from a human source.

A "patient" or "subject" to be treated by the subject method are adult mammals, including humans.

An "effective amount" of, e.g., a *hedgehog* or *ptc* therapeutic, with respect to the subject method of treatment, refers to an amount of the therapeutic in a preparation which, when applied as part of a desired dosage regimen causes a increase in survival of a neuronal cell population according to clinically acceptable standards for the treatment or prevention of a particular disorder.

By "prevent degeneration" it is meant reduction in the loss of cells (such as from apoptosis), or reduction in impairment of cell function, e.g., release of dopamine in the case of dopaminergic neurons.

A "trophic factor", referring to a *hedgehog* or *ptc* therapeutic, is a molecule that directly or indirectly affects the survival or function of a *hedgehog*-responsive cell, e.g., a dopaminergic or GABAergic cell.

A "trophic amount" of a a *hedgehog* or *ptc* therapeutic is an amount sufficient to, under the circumstances, cause an increase in the rate of survival or the functional perfomance of a *hedgehog*-responsive cell, e.g., a dopaminergic or GABAergic cell.

"Homology" and "identity" each refer to sequence similarity between two polypeptide sequences, with identity being a more strict comparison. Homology and identity can each be determined by comparing a position in each sequence which may be aligned for purposes of comparison. When a position in the compared sequence is occupied by the same amino acid residue, then the polypeptides can be referred to as identical at that position; when the equivalent site is occupied by the same amino acid (e.g., identical) or a similar amino acid (e.g., similar in steric and/or electronic nature), then the molecules can be refered to as homologous at that position. A percentage of homology or identity between sequences is a function of the number of matching or homologous positions shared by the sequences. An "unrelated" or "non-homologous" sequence shares less than 40 percent identity, though preferably less than 25 percent identity, with an hedghog sequence.

In particular, the term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology/similarity or identity refers to a function of the number of identical or similar amino acids at positions shared by the compared sequences. Various alignment algorithms and/or programs may be used, including FASTA, BLAST or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences. '

The term "corresponds to", when referring to a particular polypeptide or nucleic acid sequence is meant to indicate that the sequence of interest is identical or homologous to the reference sequence to which it is said to correspond.

The terms "recombinant protein", "heterologous protein" and "exogenous protein" are used interchangeably throughout the specification and refer to a polypeptide which is produced by recombinant DNA techniques, wherein generally, DNA encoding the polypeptide is inserted into a suitable expression construct which is in turn used to transform a host cell to produce the heterologous protein. That is, the polypeptide is expressed from a heterologous nucleic acid.

A "chimeric protein" or "fusion protein" is a fusion of a first amino acid sequence encoding a *hedgehog* polypeptide with a second amino acid sequence defining a domain foreign to and not substantially homologous with any domain of *hh* protein. A chimeric protein may present a foreign domain which is found (albeit in a different protein) in an organism which also expresses the first protein, or it may be an "interspecies", "intergenic", etc. fusion of protein structures expressed by different kinds of organisms. In general, a fusion protein can be represented by the general formula (X)ₙ-(*hh*)ₘ-(Y)ₙ, wherein *hh* represents all or a portion of the *hedgehog* polypeptide, X and Y each independently represent an amino acid sequences which are not naturally found as a polypeptide chain contiguous with the *hedgehog* sequence, m is an integer greater than or equal to 1, and each occurrence of n is, independently, 0 or an integer greater than or equal to 1 (n and m are preferably no greater than 5 or 10).

As used herein, the term "vector" refers to a nucleic acid molecule capable of transporting another nucleic acid to which it has been linked. The term "expression vector" includes plasmids, cosmids or phages capable of synthesizing, for example, the subject *hedgehog* polypeptides encoded by the respective recombinant gene carried by the vector. Preferred vectors are those capable of autonomous replication and/expression of nucleic acids to which they are linked. In the present specification, "plasmid" and "vector" are used interchangeably as the plasmid is the most commonly used form of vector. Moreover, the invention is intended to include such other forms of expression vectors which serve equivalent functions and which become known in the art subsequently hereto.

"Transcriptional regulatory sequence" is a generic term used throughout the specification to refer to DNA sequences, such as initiation signals, enhancers, and promoters, as well as polyadenylation sites, which induce or control transcription of protein (or antisense) coding sequences with which they are operably linked. In preferred embodiments, transcription of a recombinant gene is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring form of the regulatory protein.

The term "operably linked" refers to the arrangement of a transcriptional regulatory element relative to other transcribable nucleic acid sequence such that the transcriptional regulatory element can regulate the rate of transcription from the transcribable sequence(s).

### III. Exemplary Applications of Method and Compositions

One aspect of the present invention relates to a method of maintaining a differentiated state, e.g., enhancing survival, of a neuronal cell responsive to a *hedgehog* polypeptide, by contacting the cells with a trophic amount of a *hedgehog* or *ptc* thereapeutic. For instance, it is contemplated by the invention that, in light of the present finding of an apparently trophic effect of *hedgehog* polypeptides in the maintenance of differentiated neurons, the subject method could be used to maintain different neuronal tissue both *in vitro* and *in vivo.* Where the trophic agent is a *hedgehog* polypeptide, it can be provided to a cell culture or animal as a purified protein or secreted by a recombinant cell, or cells or tissue explants which naturally produce one or more *hedgehog* polypeptides. For instance, neural tube explants from embryos, particularly floorplate tissue, can provide a source for *Shh* polypeptide, which source can be implanted in a patient or otherwise provided, as appropriate, for maintenance of differentiation.

### A. Cell Culture

The present method is applicable to cell culture techniques. *In vitro* neuronal culture systems have proved to be fundamental and indispensable tools for the study of neural development, as well as the identification of neurotrophic factors such as nerve growth factor (NGF), ciliary trophic factors (CNTF), and brain derived neurotrophic factor (BDNF). Once a neuronal cell has become terminally-differentiated it typically will not change to another terminally differentiated cell-type. However, neuronal cells can nevertheless readily lose their differentiated state. This is commonly observed when they are grown in culture from adult tissue, and when they form a blastema during regeneration. The present method provides a means for ensuring an adequately restrictive environment in order to maintain dopaminergic, GABAergic or other exotoxic-sensitive neuronal cells in differentiated states, and can be employed, for instance, in cell cultures designed to test the specific activities of other trophic factors.

In such embodiments of the subject method, a culture of differentiated cells including the neuronal cells can be contacted with a *hedgehog* or *ptc* therapeutic in order to maintain the integrity of a culture of terminally-differentiated neuronal cells by preventing loss of differentiation. The source of *hedgehog or ptc* therapeutic in the culture can be derived from, for example, a purified or semi-purified protein composition added directly to the cell culture media, or alternatively, supported and/or released from a polymeric device which supports the growth of various neuronal cells and which has been doped with the protein. The source of, for example, a trophic *hedgehog* polypeptide can also be a cell that is co-cultured with the neuronal cells. Alternatively, the source can be the neuronal cell itself which has been engineered to produce a recombinant *hedgehog* polypeptide. Such neuronal cultures can be used as convenient assay systems as well as sources of implantable cells for therapeutic treatments.

The subject method can be used in conjunction with agents which induce the differentiation of neuronal precursors, e.g., progenitor or stem cells, into dopaminergic neurons, GABAergic neurons or other neuronal cell-types.

Cells can be obtained from embryonic, post-natal, juvenile or adult neural tissue from any animal. By any animal is meant any multicellular animal which contains nervous tissue. More particularly, is meant any fish, reptile, bird, amphibian or mammal and the like. The most preferable donors are mammals, especially humans and non-human primates, pigs, cows, and rodents.

Intracerebral neural grafting has emerged recently as an additional potential to CNS therapy. For example, one approach to repairing damaged brain tissues involves the transplantation of cells from fetal or neonatal animals into the adult brain (Dunnett et al. (1987) *J Exp Biol* 123:265-289; and Freund et al. (1985) *J Neurosci* 5:603-616). Fetal neurons from a variety of brain regions can be successfully incorporated into the adult brain, and such grafts can alleviate behavioral defects. For example, movement disorder induced by lesions of dopaminergic projections to the basal ganglia can be prevented by grafts of embryonic dopaminergic neurons. Complex cognitive functions that are impaired after lesions of the neocortex can also be partially restored by grafts of embryonic cortical cells. Transplantation of fetal brain cells, which contain precursors of the dopaminergic neurons, has been examined with success as a treatment for Parkinson's disease. In animal models and in patients with this disease, fetal brain cell transplantations have resulted in the reduction of motor abnormalities. Furthermore, it appears that the implanted fetal dopaminergic neurons form synapses with surrounding host neurons. However, in the art, the transplantation of fetal brain cells is limited due, for example, to the limited survival time of the implanted neuronal precursors and differentiated neurons arising therefrom. The subject invention provides a means for extending the usefulness of such transplants by enhancing the survival of dopaminergic and/or GABAergic cells in the transplant.

In the specific case of Parkinson's disease, intervention by increasing the activity of *hedgehog,* by ectopic or endogenous means, can improve the *in vivo* survival of fetal and adult dopaminergic neurons, and thus can provide a more effective treatment of this disease. Cells to be transplanted for the treatment of a particular disease can be genetically modified *in vitro* so as to increase the expression of *hedgehog* in the transplant. In an exemplary embodiment of the invention, administration of an *Shh* polypeptide can be used in conjunction with surgical implantation of tissue in the treatment of Parkinson's disease.

In the case of a heterologous donor animal, the animal may be euthanized, and the brain and specific area of interest removed using a sterile procedure. Brain areas of particular interest include any area from which progenitor cells can be obtained which will provide dopaminergic or GABAergic cells upon differentiation. These regions include areas of the central nervous system (CNS) including the substantia nigra pars compacta which is found to be degenerated in Parkinson's Disease patients.

Human heterologous neural progenitor cells may be derived from fetal tissue obtained from elective abortion, or from a post-natal, juvenile or adult organ donor. Autologous neural tissue can be obtained by biopsy, or from patients undergoing neurosurgery in which neural tissue is removed, such as during epilepsy surgery.

Cells can be obtained from donor tissue by dissociation of individual cells from the connecting extracellular matrix of the tissue. Dissociation can be obtained using any known procedure, including treatment with enzymes such as trypsin, collagenase and the like, or by using physical methods of dissociation such as with a blunt instrument. Dissociation of fetal cells can be carried out in tissue culture medium, while a preferable medium for dissociation of juvenile and adult cells is artificial cerebral spinal fluid (aCSF). Regular aCSF contains 124 mM NaCl, 5 mM KCl, 1.3 mM MgCl₂, 2 mM CaCl₂, 26 mM NaHCO₃ and 10 mM D-glucose. Low Ca²⁺ aCSF contains the same ingredients except for MgCl₂ at a concentration of 3.2 mM and CaCl₂ at a concentration of 0.1 mM.

Dissociated cells can be placed into any known culture medium capable of supporting cell growth, including MEM, DMEM, RPMI, F-12, and the like, containing supplements which are required for cellular metabolism such as glutamine and other amino acids, vitamins, minerals and useful proteins such as transferrin and the like. Medium may also contain antibiotics to prevent contamination with yeast, bacteria and fungi such as penicillin, streptomycin, gentamicin and the like. In some cases, the medium may contain serum derived from bovine, equine, chicken and the like. A particularly preferable medium for cells is a mixture of DMEM and F-12.

Conditions for culturing should be close to physiological conditions. The pH of the culture media should be close to physiological pH, preferably between pH 6-8, more preferably close to pH 7, even more particularly about pH 7.4. Cells should be cultured at a temperature close to physiological temperature, preferably between 30°C-40°C, more preferably between 32°C-38°C, and most preferably between 35°C-37°C.

Cells can be grown in suspension or on a fixed substrate, but proliferation of the progenitors is preferably done in suspension to generate large numbers of cells by formation of "neurospheres" (see, for example, Reynolds et al. (1992) *Science* 255:1070-1709; and PCT Publications WO93/01275, WO94/09119, WO94/10292, and WO94/16718). In the case of propagating (or splitting) suspension cells, flasks are shaken well and the neurospheres allowed to settle on the bottom corner of the flask. The spheres are then transferred to a 50 ml centrifuge tube and centrifuged at low speed. The medium is aspirated, the cells resuspended in a small amount of medium with growth factor, and the cells mechanically dissociated and resuspended in separate aliquots of media.

Cell suspensions in culture medium are supplemented with any growth factor which allows for the proliferation of progenitor cells and seeded in any receptacle capable of sustaining cells, though as set out above, preferably in culture flasks or roller bottles. Cells typically proliferate within 3-4 days in a 37°C incubator, and proliferation can be reinitiated at any time after that by dissociation of the cells and resuspension in fresh medium containing growth factors.

In the absence of substrate, cells lift off the floor of the flask and continue to proliferate in suspension forming a hollow sphere of undifferentiated cells. After approximately 3-10 days *in vitro,* the proliferating clusters (neurospheres) are fed every 2-7 days, and more particularly every 2-4 days by gentle centrifugation and resuspension in medium containing growth factor.

After 6-7 days *in vitro,* individual cells in the neurospheres can be separated by physical dissociation of the neurospheres with a blunt instrument, more particularly by triturating the neurospheres with a pipette. Single cells from the dissociated neurospheres are suspended in culture medium containing growth factors, and differentiation of the cells can be induced by plating (or resuspending) the cells in the presence of a factor capable of sustaining differentiation, e.g., such as a *hedgehog* or *ptc* therapeutic of the present invention.

Stem cells useful in the present invention are generally known. For example, several neural crest cells have been identified, some of which are multipotent and likely represent uncommitted neural crest cells. The role of *hedgehog* polypeptides employed in the present method to culture such stem cells is to maintain differentiation a committed progenitor cell amd/or a terminally-differentiated dopaminergic or GABAergic neuronal cell. The *hedgehog* polypeptide can be used alone, or can be used in combination with other neurotrophic factors which act to more particularly enhance a particular differentiation fate of the neuronal progenitor cell.

### B. In vivo applications

In addition to the implantation of cells cultured in the presence of a functional *hedgehog* activity and other *in vitro* uses described above, yet another aspect of the present invention concerns the therapeutic application of a *hedgehog* or *ptc* therapeutic to enhance survival of neurons sensitive to exotoxic degeneration *in vivo,* e.g., such as dopaminergic and GABAergic neurons. The ability of *hedgehog* polypeptide to maintain neuronal differentiation indicates that certain of the *hedgehog* polypeptides can be reasonably expected to facilitate control of these neuronal cell-types in adult tissue with regard to maintenance, functional performance, aging and prevention of degeneration and premature death which result from loss of differentiation in certain pathological conditions.

In light of this understanding, the present invention specifically contemplates applications of the subject method to the treatment of (prevention and/or reduction of the severity of) neurological conditions deriving from exotoxic degeneration of neuronal cells, including cell death or loss of functional performance, e.g., such as loss of dopaminergic cells, loss of GABAergic cells, and/or loss of neurons of the substantia nigra. In this regard, the subject method is useful in the treatment or prevention of such neurologic disorders including Parkinson's disease, domoic acid poisoning; spinal cord trauma; hypoglycemia; mechanical trauma to the nervous system; senile dementia; Korsakoffs disease; schizophrenia; AIDS dementia, multi-infarct dementia; mood disorders; depression; chemical toxicity and neuronal damage associated with uncontrolled seizures, such as epileptic seizures; and chronic neurologic disorders such as Huntington's disease, neuronal injury associated with HIV and AIDS, AIDS dementia, neurodegeneration associated with Down's syndrome, neuropathic pain syndrome, olivopontocerebral atrophy, amyotrophic lateral sclerosis, mitochondrial abnormalities, Alzheimer's disease, hepatic encephalopathy, Tourette's syndrome, schizophrenia, and drug addiction.

The subject *hedgehog* and *ptc* therpaeutics can also used to reduce neurotoxic injury associated with conditions of hypoxia, anoxia or ischemia which typically follows stroke, cerebrovascular accident, brain or spinal chord trauma, myocardial infarct, physical trauma, drownings, suffocation, perinatal asphyxia, or hypoglycemic events.

In preferred embodiments, the subject method is used to reduce the severity or prevent Parkinson's disease or Huntington's chorea.

In general, the therapeutic method of the present invention can be characterized as including a step of administering to an animal an amount of a *ptc* or *hedgehog* therapeutic effective to enhance the survival of a dopaminergic and/or GABAergic neuronal cells. The mode of administration and dosage regimens will vary depending on the severity of the degenerative disoder being treated, e.g., the dosage may be altered as between a prophylaxis and treatment. In preferred embodiments, the *ptc* or hedeghog therapeutic is administered systemically initially, then locally for medium to long term care. In certain embodiments, a source of a *hedgehog* or *ptc* therapeutic is stereotactically provided within or proximate the area of degeneration.

The subject method may also find particular utility in treating or preventing the adverse neurological consequences of surgery. For example, certain cranial surgery can result in degeneration of neuronal populations for which the subject method can be applied.

In other embodiments, the subject method can be used to prevent or treat neurodegenerative conditions arising from the use of certain drugs, such as the compound MPTP (1-methyl-4-phenyl-1,2,3,6-tetrahydropyridine).

In still other embodiments, the subject method can be used in the prevention and/or treatment of hypoxia, e.g., as a neuroprotective agent. For instance, the subject method can be used prophylactically to lessen the neuronal cell death caused by altitude-induced hypoxia.

A method which is "neuroprotective", in the case of dopaminergic and GABAergic cells, results in diminished loss of cells of those phenotype relative to that which would occur in the absence of treatment with a *hedgehog* or *ptc* therapeutic.

### (i) Treatment of Parkinson's disease

It is now widely appreciated that the primary pathology underlying Parkinson's disease is degeneration of the dopaminergic projection from the substantia nigra to the striatum. This realisation has led to the widespread use of dopamine-replacing agents such as L-DOPA and apomorphine as symptomatic treatments for Parkinson's disease. Over the last three decades, such therapies have undoubtedly been successful in increasing the quality of life of patients suffering from Parkinson's disease, but, dopamine-replacement treatments do have limitations, especially following long-term treatment. Problems can include a wearing-off of anti-parkinsonian efficacy and the appearance of a range of dyskinesias characterised by chorea and dystonia. Ultimately, these side-effects can severely limit the usefulness of dopaminergic treatments.

As described in the appended examples, *hedgehog* exerts trophic and survival-promoting actions on substantia nigra dopaminergic neurons. *In vivo,* treatment with exogenous *hedgehog,* or other compounds of the present invention, is expected to stimulate, for example, the dopaminergic phenotype of substantia nigra neurons and restore functional deficits induced by axotomy or dopaminergic neurotoxins. Therefore it may be used in the treatment of Parkinson's disease, a neurodegenerative disease characterized by the loss of dopaminergic neurons. Thus, in one embodiment, the subject method comprises administering to an animal afflected with Parkinson's disease, or at risk of developing Parkonson's disease, an amount of a *hedgehog* or *ptc* thereapeutic effective for protecting or restoring the function of neurons affected by the Parkinson's condition, e.g., increasing the rate of survival of dopaminergic neurons in the animal. In preferred embodiments, the method includes administering to the animal an amount of a *hedgehog* or *ptc* thereapeutic which would otherwise be effective at protecting the substantia nigra from MPTP-mediated toxicity when MPTP is administered at a dose of .5mg/kg, more preferably at a dose of 2mg/kg, 5mg/kg, 10mg/kg, 20mg/kg or 50mg/kg and, more preferably, at a dose of 100mg/kg.

### (ii) Treatment of Huntington's disease

Huntington's disease involves the degeneration of intrastraital and cortical cholinergic neurons and GABAergic neurons. Treatment of patients suffering from such degenerative conditions can include the application of *hedgehog or ptc* therapeutics of the present invention, in order to control, for example, apoptotic events which give rise to loss of GABAergic neurons (e.g. to enhance survival of existing neurons.

### (iii) Treatment of amyotrophic lateral sclerosis

Recently it has been reported that in certain ALS patients and animal models a significant loss of midbrain dopaminergic neurons occurs in addition to the loss of spinal motor neurons. For instance, the literature describes degeneration of the substantia nigra in some patients with familial amyotrophic lateral sclerosis. Kostic et al. (1997) Ann Neurol 41:497-504. According the subject invention, a trophic amount of a *hedgehog* or *ptc* therapeutic can be administered to an animal suffering from, or at risk of developing, ALS.

### (iv) Treatment of epilepsy

Epilepsy is a recurrent paroxysmal disorder of cerebral function characterized by sudden brief attacks of altered consciousness, motor activity, sensory phenomena or inappropriate behavior caused by abnormal excessive discharge of cerebral neurons. Convulsive seizures, the most common form of attacks, begin with loss of consciousness and motor control, and tonic or clonic jerking of all extremities but any recurrent seizure pattern may be termed epilepsy.

The term primary or idiopathic epilepsy denotes those cases where no cause for the seizures can be identified. Secondary or symptomatic epilepsy designates the disorder when it is associated with such factors as trauma, neoplasm, infection, developmental abnormalities, cerebrovascular disease, or various metabolic conditions. Epileptic seizures are classified as partial seizures (focal, local seizures) or generalized seizures (convulsive or nonconvulsive). Classes of partial seizures include simple partial seizures, complex partial seizures and partial seizures secondarily generalized. Classes of generalized seizures include absence seizures, atypical absence seizures, myoclonic seizures, clonic seizures, tonic seizures, tonic-clonic seizures (grand mal) and atonic seizures.

Therapeutics having anticonvulsant properties are used in the treatment of seizures. Most therapeutics used to abolish or attenuate seizures act at least through effects that reduce the spread of excitation from seizure foci and prevent detonation and disruption of function of normal aggregates of neurons. Anticonvulsants which have been utilized include phenytoin, phenobarbital, primidone, carbamazepine, ethosuximide, clonazepam and valproate. For further details of seizures and their therapy (see Rall & Schleifer (1985) and The Merck Manual (1992)).

Due to the involvement of exotoxic-dependent neurodegeneration which can result from seizure, certain *hedgehog* and *ptc* therpaeutics of the present invention may be useful as part of a regimen in the treatment of epilepsy, and are preferably used in conjunction with a treatment including an anticonvulsant agent.

### (v) Treatment of reperfusion injury

The most direct approach to treating cerebral ischemia is to restore circulation. However, reperfusion following transient ischemia can induce additional mechanisms of tissue damage. This phenomenon is termed "reperfusion injury" and has been found to play a role in other organ systems as well, including the heart. Recently, it has been suggested that cerebral ischemic damage is mediated, to a large extent, via excitotoxic mechanisms. During ischemia, large elevations in extracellular glutamate occur, often reaching neurotoxic levels. Accordingly, the subject method can be used as part of a treatment or prophylaxis for ischemic or epoxic damage, particularly to alleviate certain effects of reperfusion injury.

### (vi) Treatment of glaucoma

Glaucoma is a complex set of diseases, which results in damage to axons in the optic nerve and death of the retinal ganglion cells, concluding in the permanent loss of vision. There are several mechanism that ultimately causes the axonal damage. For instance, an increase in intraocular pressure (IOP) overcomes the perfusion pressure of the optic nerve and results in an ischemic event which leads to axonal.

In addition to the primary insult and ensuing cell damage, there appears to be a secondary degenerative process in glaucoma. Clinically, patients often continue to lose visual field and optic nerve head substance even in the presence of what might be considered normal IOP. In addition, there are forms of glaucoma which manifest in the presence of normal IOP. It is thought that chemical mediators may be linked to intensification of cell degeneration and death in a secondary fashion. Much work has been done in the last five years which implicates the role of excitotoxins, such as glutamate, in the secondary damage to retinal neurons. Accordingly, the subject method can be used as part of a treatment and/or prophylaxis for glaucoma.

### (vii) Treatment of hearing loss

The mechanism by which aminoglycosides produce permanent hearing loss is mediated, in part, through an excitotoxic process. Accordingly, the subject method can be used as part of a treatment and/or prophylaxis for hearing loss.

### (viii) Conjoint Therapy

In yet other embodiments, the subject method can be carried out conjointly with the administration of growth and/or trophic factors. For instance, the combinatorial therapy can include a trophic factor such as nerve growth factor, cilliary neurotrophic growth factor, schwanoma-derived growth factor, glial growth factor, stiatal-derived neuronotrophic factor, platelet-derived growth factor, and scatter factor (HGF-SF). Antimitogenic agents can also be used, as for example, cytosine, arabinoside, 5-fluorouracil, hydroxyurea, and methotrexate.

### (ix) Formulations

Determination of a therapeutically effective amount and a prophylactically effective amount of a *hedgehog* or *ptc* therapeutic, e.g., to be adequately neuroprotective, can be readily made by the physician or veterinarian (the "attending clinician"), as one skilled in the art, by the use of known techniques and by observing results obtained under analogous circumstances. The dosages may be varied depending upon the requirements of the patient in the judgment of the attending clinician, the severity of the condition being treated, the risk of further degeneration to the CNS, and the particular agent being employed. In determining the therapeutically effective trophic amount or dose, and the prophylactically effective amount or dose, a number of factors are considered by the attending clinician, including, but not limited to: the specific cause of the degenerative state and its likelihood of recurring or worsening; pharmacodynamic characteristics of the particular agent and its mode and route of administration; the desirder time course of treatment; the species of mammal; its size, age, and general health; the response of the individual patient; the particular compound administered; the bioavailability characteristics of the preparation administered; the dose regimen selected; the kind of concurrent treatment (i.e., the interaction of the *hedgehog* or *ptc* therapeutic with other co-administered therapeutics); and other relevant circumstances.

Treatment can be initiated with smaller dosages which are less than the optimum dose of the agent. Thereafter, the dosage should be increased by small increments until the optimum effect under the circumstances is reached. For convenience, the total daily dosage may be divided and administered in portions during the day if desired. A therapeutically effective trophic amount and a prophylactically effective neuroprotective amount of a *hedgehog* polypeptide, for instance, is expected to vary from concentrations about 0.1 nanogram per kilogram of body weight per day (ng/kg/day) to about 100 mg/kg/day.

Potential *hedgehog* and *ptc* therapeutics, such as described below, can be tested by any of number of well known animal disease models. For instance, regarding Parkinson's Disease, selected agents can be evaluated in animals treated with MPTP. The compound MPTP (1-methyl-4-phenyl-1,2,3,6-tetrahydropyridine) and its metabolite MPP⁺ have been used to induce experimental parkinsonism. MPP⁺ kills dopaminergic neurons in the substantia nigra, yielding a reasonable model of late parkinsonism. Turski et al., (1991) *Nature* 349:414.

Compounds which are determined to be effective for the prevention or treatment of degeneration of dopaminergic and GABAergic neurons and the like in animals, e.g., dogs, rodents, may also be useful in treatment of disorders in humans. Those skilled in the art of treating in such disorders in humans will be guided, from the data obtained in animal studies, to the correct dosage and route of administration of the compound to humans. In general, the determination of dosage and route of administration in humans is expected to be similar to that used to determine administration in animals.

The identification of those patients who are in need of prophylactic treatment for disorders marked by degeneration of dopaminergic and/or GABAergic neurons is well within the ability and knowledge of one skilled in the art. Certain of the methods for identification of patients which are at risk and which can be treated by the subject method are appreciated in the medical arts, such as family history of the development of a particular disease state and the presence of risk factors associated with the development of that disease state in the subject patient. A clinician skilled in the art can readily identify such candidate patients, by the use of, for example, clinical tests, physical examination and medical/family history.

### IV. Exemplary hedgehog therapeutic compounds.

The *hedgehog* therapeutic compositions of the subject method can be generated by any of a variety of techniques, including purification of naturally occurring proteins, recombinantly produced proteins and synthetic chemistry. Polypeptide forms of the *hedgehog* therapeutics are preferably derived from vertebrate *hedgehog* polypeptides, e.g., have sequences corresponding to naturally occurring *hedgehog* polypeptides, or fragments thereof, from vertebrate organisms. However, it will be appreciated that the *hedgehog* polypeptide can correspond to a *hedgehog* polypeptide (or fragment thereof) which occurs in any metazoan organism.

The various naturally-occurring *hedgehog* polypeptides from which the subject therapeutics can be derived are characterized by a signal peptide, a highly conserved N-terminal region, and a more divergent C-terminal domain. In addition to signal sequence cleavage in the secretory pathway (Lee, J.J. *et al.* (1992) *Cell* 71:33-50; Tabata, T. *et al.* (1992) *Genes Dev.* 2635-2645; Chang, D.E. *et al.* (1994) *Development* 120:3339-3353), *hedgehog* precursor proteins naturally undergo an internal autoproteolytic cleavage which depends on conserved sequences in the C-terminal portion (Lee *et al.* (1994) *Science* 266:1528-1537; Porter *et al*. (1995) *Nature* 374:363-366). This autocleavage leads to a 19 kD N-terminal peptide and a C-terminal peptide of 26-28 kD (Lee *et al.* (1992) *supra;* Tabata *et al.* (1992) *supra;* Chang *et al.* (1994) *supra;* Lee *et al.* (1994) *supra;* Bumcrot, D.A., *et al.* (1995) *Mol. Cell. Biol.* 15:2294-2303; Porter *et al.* (1995) *supra;* Ekker, S.C. *et al.* (1995) *Curr. Biol.* 5:944-955; Lai, C.J. *et al.* (1995) *Development* 121:2349-2360). The N-terminal peptide stays tightly associated with the surface of cells in which it was synthesized, while the C-terminal peptide is freely diffusible both *in vitro* and *in vivo* (Lee *et al*. (1994) *supra;* Bumcrot *et al*. (1995) *supra;* Mart', E. *et al*. (1995) *Development* 121:2537-2547; Roelink, H. *et al*. (1995) *Cell* 81:445-455). Cell surface retention of the N-terminal peptide is dependent on autocleavage, as a truncated form of *hedgehog* encoded by an RNA which terminates precisely at the normal position of internal cleavage is diffusible *in vitro* (Porter *et al*. (1995) *supra*) and *in vivo* (Porter, J.A. *et al*. (1996) *Cell* 86, 21-34). Biochemical studies have shown that the autoproteolytic cleavage of the *hedgehog* precursor protein proceeds through an internal thioester intermediate which subsequently is cleaved in a nucleophilic substitution. It is suggested that the nucleophile is a small lipophilic molecule, more particularly cholesterol, which becomes covalently bound to the C-terminal end of the N-peptide (Porter *et al.* (1996) *supra*), tethering it to the cell surface (WO 98/30576).

The vertebrate family of *hedgehog* genes includes at least four members, e.g., paralogs of the single drosophila *hedgehog* gene (SEQ ID No. 19). Three of these members, herein referred to as Desert *hedgehog* (*Dhh*), Sonic *hedgehog* (*Shh*) and Indian *hedgehog* (*Ihh*), apparently exist in all vertebrates, including fish, birds, and mammals. A fourth member, herein referred to as tiggie-winkle *hedgehog* (*Thh*), appears specific to fish. According to the appended sequence listing, (see also Table 1) a chicken *Shh* polypeptide is encoded by SEQ ID No:1; a mouse *Dhh* polypeptide is encoded by SEQ ID No:2; a mouse *Ihh* polypeptide is encoded by SEQ ID No:3; a mouse *Shh* polypeptide is encoded by SEQ ID No:4 a zebrafish *Shh* polypeptide is encoded by SEQ ID No:5; a human *Shh* polypeptide is encoded by SEQ ID No:6; a human *Ihh* polypeptide is encoded by SEQ ID No:7; a human *Dhh* polypeptide is encoded by SEQ ID No. 8; and a zebrafish *Thh* is encoded by SEQ ID No. 9.

**Table I Guide to hedgehog sequences in Sequence Listing**

| | Nucleotide | Amino Acid |
|---|---|---|
| Chicken *Shh* | SEQ ID No. 1 | SEQ ID No. 10 |
| Mouse *Dhh* | SEQ ID No. 2 | SEQ ID No. 11 |
| Mouse *Ihh* | SEQ ID No. 3 | SEQ ID No. 12 |
| Mouse *Shh* | SEQ ID No. 4 | SEQ ID No. 13 |
| Zebrafish *Shh* | SEQ ID No. 5 | SEQ ID No. 14 |
| Human *Shh* | SEQ ID No. 6 | SEQ ID No. 15 |
| Human *Ihh* | SEQ ID No. 7 | SEQ ID No. 16 |
| Human *Dhh* | SEQ ID No. 8 | SEQ ID No. 17 |
| Zebrafish *Thh* | SEQ ID No. 9 | SEQ ID No. 18 |
| Drosophila *HH* | SEQ ID No. 19 | SEQ ID No. 20 |

In addition to the sequence variation between the various *hedgehog* homologs, the *hedgehog* polypeptides are apparently present naturally in a number of different forms, including a pro-form, a full-length mature form, and several processed fragments thereof. The pro-form includes an N-terminal signal peptide for directed secretion of the extracellular domain, while the full-length mature form lacks this signal sequence.

As described above, further processing of the mature form occurs in some instances to yield biologically active fragments of the protein. For instance, *sonic hedgehog* undergoes additional proteolytic processing to yield two peptides of approximately 19 kDa and 27 kDa, the 19kDa fragment corresponding to an proteolytic N-terminal portion of the mature protein.

In addition to proteolytic fragmentation, the vertebrate *hedgehog* polypeptides can also be modified post-translationally, such as by glycosylation and/or addition of lipophilic moieties, such as stents, fatty acids, etc., though bacterially produced (e.g. unmodified) forms of the proteins still maintain certain of the bioactivities of the native protein. Bioactive fragments of *hedgehog* polypeptides of the present invention have been generated and are described in great detail in, e.g., PCT publications WO 95/18856 and WO 96/17924.

There are a wide range of lipophilic moieties with which *hedgehog* polypeptides can be derivatived. The term "lipophilic group", in the context of being attached to a *hedgehog* polypeptide, refers to a group having high hydrocarbon content thereby giving the group high affinity to lipid phases. A lipophilic group can be, for example, a relatively long chain alkyl or cycloalkyl (preferably n-alkyl) group having approximately 7 to 30 carbons. The alkyl group may terminate with a hydroxy or primary amine "tail". To further illustrate, lipophilic molecules include naturally-occurring and synthetic aromatic and non-aromatic moieties such as fatty acids, sterols, esters and alcohols, other lipid molecules, cage structures such as adamantane and buckminsterfullerenes, and aromatic hydrocarbons such as benzene, perylene, phenanthrene, anthracene, naphthalene, pyrene, chrysene, and naphthacene.

In one embodiment, the *hedgehog* polypeptide is modified with one or more sterol moieties, such as cholesterol. See, for example, PCT publication WO 96/17924. In certain embodiments, the cholesterol is preferably added to the C-terminal glycine when the *hedgehog* polypeptide corresponds to the naturally-occurring N-terminal proteolytic fragment.

In another embodiment, the *hedgehog* polypeptide can be modified with a fatty acid moiety, such as a myrostoyl, palmitoyl, stearoyl, or arachidoyl moiety. See, e.g., Pepinsky et al. (1998) J Biol. Chem 273: 14037.

In addition to those effects seen by cholesterol-addition to the C-terminus or fatty acid addition to the N-terminus of extracellular fragments of the protein, at least certain of the biological activities of the *hedgehog* gene products are unexpectedly potentiated by derivativation of the protein with lipophilic moieties at other sites on the protein and/or by moieties other than cholesterol or fatty acids. Certain aspects of the invention are directed to the use of preparations of *hedgehog* polypeptides which are modified at sites other than N-terminal or C-terminal residues of the natural processed form of the protein, and/or which are modified at such terminal residues with lipophilic moieties other than a sterol at the C-terminus or fatty acid at the N-terminus.

Particularly useful as lipophilic molecules are alicyclic hydrocarbons, saturated and unsaturated fatty acids and other lipid and phospholipid moieties, waxes, cholesterol, isoprenoids, terpenes and polyalicyclic hydrocarbons including adamantane and buckminsterfullerenes, vitamins, (C1-C18)-alkyl phosphate diesters, -O-CH2-CH(OH)-O-(C12-C18)-alkyl, and in particular conjugates with pyrene derivatives. The lipophilic moiety can be a lipophilic dye suitable for use in the invention include, but are not limited to, diphenylhexatriene, Nile Red, N-phenyl-1-naphthylamine, Prodan, Laurodan, Pyrene, Perylene, rhodamine, rhodamine B, tetramethylrhodamine, Texas Red, sulforhodamine, 1,1'-didodecyl-3,3,3',3'tetramethylindocarbocyanine perchlorate, octadecyl rhodamine B and the BODIPY dyes available from Molecular Probes Inc.

Other exemplary lipophilic moietites include aliphatic carbonyl radical groups include 1- or 2-adamantylacetyl, 3-methyladamant-1-ylacetyl, 3-methyl-3-bromo-1-adamantylacetyl, 1-decalinacetyl, camphoracetyl, camphaneacetyl, noradamantylacetyl, norbornaneacetyl, bicyclo[2.2.2.]-oct-5-eneacetyl, 1-methoxybicyclo[2.2.2.]-oct-5-ene-2-carbonyl, cis-5-norbornene-endo-2,3-dicarbonyl, 5-norbornen-2-ylacetyl, (1R)-( - )-myrtentaneacetyl, 2-norbomaneacetyl, anti-3-oxo-tricyclo[2.2.1.0<2,6>]-heptane-7-carbonyl, decanoyl, dodecanoyl, dodecenoyl, tetradecadienoyl, decynoyl or dodecynoyl.

The *hedgehog* polypeptide can be linked to the hydrophobic moiety in a number of ways including by chemical coupling means, or by genetic engineering.

There are a large number of chemical cross-linking agents that are known to those skilled in the art. For the present invention, the preferred cross-linking agents are heterobifunctional cross-linkers, which can be used to link the *hedgehog* polypeptide and hydrophobic moiety in a stepwise manner. Heterobifunctional cross-linkers provide the ability to design more specific coupling methods for conjugating to proteins, thereby reducing the occurrences of unwanted side reactions such as homo-protein polymers. A wide variety of heterobifunctional cross-linkers are known in the art. These include: succinimidyl 4-(N-maleimidomethyl) cyclohexane- 1-carboxylate (SMCC), m-Maleimidobenzoyl-N- hydroxysuccinimide ester (MBS); N-succinimidyl (4-iodoacetyl) aminobenzoate (SIAB), succinimidyl 4-(p-maleimidophenyl) butyrate (SMPB), 1-ethyl-3-(3-dimethylaminopropyl) carbodiimide hydrochloride (EDC); 4-succinimidyloxycarbonyl- a-methyl-a-(2-pyridyldithio)-tolune (SMPT), N-succinimidyl 3-(2-pyridyldithio) propionate (SPDP), succinimidyl 6-[3-(2-pyridyldithio) propionate] hexanoate (LC-SPDP). Those cross-linking agents having N-hydroxysuccinimide moieties can be obtained as the N-hydroxysulfosuccinimide analogs, which generally have greater water solubility. In addition, those cross-linking agents having disulfide bridges within the linking chain can be synthesized instead as the alkyl derivatives so as to reduce the amount of linker cleavage *in vivo.*

In addition to the heterobifunctional cross-linkers, there exists a number of other cross-linking agents including homobifunctional and photoreactive cross-linkers. Disuccinimidyl suberate (DSS), bismaleimidohexane (BMH) and dimethylpimelimidate·2 HCl (DMP) are examples of useful homobifunctional cross-linking agents, and bis-[β-(4-azidosalicylamido)ethyl]disulfide (BASED) and N-succinimidyl-6(4'-azido-2'-nitrophenylamino)hexanoate (SANPAH) are examples of useful photoreactive cross-linkers for use in this invention. For a recent review of protein coupling techniques, see Means et al. (1990) *Bioconjugate Chemistry* 1:2-12, incorporated by reference herein.

One particularly useful class of heterobifunctional cross-linkers, included above, contain the primary amine reactive group, N-hydroxysuccinimide (NHS), or its water soluble analog N-hydroxysulfosuccinimide (sulfo-NHS). Primary amines (lysine epsilon groups) at alkaline pH's are unprotonated and react by nucleophilic attack on NHS or sulfo-NHS esters. This reaction results in the formation of an amide bond, and release of NHS or sulfo-NHS as a by-product.

Another reactive group useful as part of a heterobifunctional cross-linker is a thiol reactive group. Common thiol reactive groups include maleimides, halogens, and pyridyl disulfides. Maleimides react specifically with free sulfhydryls (cysteine residues) in minutes, under slightly acidic to neutral (pH 6.5-7.5) conditions. Halogens (iodoacetyl functions) react with -SH groups at physiological pH's. Both of these reactive groups result in the formation of stable thioether bonds.

The third component of the heterobifunctional cross-linker is the spacer arm or bridge. The bridge is the structure that connects the two reactive ends. The most apparent attribute of the bridge is its effect on steric hindrance. In some instances, a longer bridge can more easily span the distance necessary to link two complex biomolecules. For instance, SMPB has a span of 14.5 angstroms.

Preparing protein-protein conjugates using heterobifunctional reagents is a two-step process involving the amine reaction and the sulfhydryl reaction. For the first step, the amine reaction, the protein chosen should contain a primary amine. This can be lysine epsilon amines or a primary alpha amine found at the N-terminus of most proteins. The protein should not contain free sulfhydryl groups. In cases where both proteins to be conjugated contain free sulfhydryl groups, one protein can be modified so that all sulfhydryls are blocked using for instance, N-ethylmaleimide (see Partis et al. (1983) J. Pro. Chem. 2:263, incorporated by reference herein). Ellman's Reagent can be used to calculate the quantity of sulfhydryls in a particular protein (see for example Ellman et al. (1958) Arch. Biochem. Biophys. 74:443 and Riddles et al. (1979) Anal. Biochem. 94:75, incorporated by reference herein).

The reaction buffer should be free of extraneous amines and sulfhydryls. The pH of the reaction buffer should be 7.0-7.5. This pH range prevents maleimide groups from reacting with amines, preserving the maleimide group for the second reaction with sulfhydryls.

The NHS-ester containing cross-linkers have limited water solubility. They should be dissolved in a minimal amount of organic solvent (DMF or DMSO) before introducing the cross-linker into the reaction mixture. The cross-linker/solvent forms an emulsion which will allow the reaction to occur.

The sulfo-NHS ester analogs are more water soluble, and can be added directly to the reaction buffer. Buffers of high ionic strength should be avoided, as they have a tendency to "salt out" the sulfo-NHS esters. To avoid loss of reactivity due to hydrolysis, the cross-linker is added to the reaction mixture immediately after dissolving the protein solution.

The reactions can be more efficient in concentrated protein solutions. The more alkaline the pH of the reaction mixture, the faster the rate of reaction. The rate of hydrolysis of the NHS and sulfo-NHS esters will also increase with increasing pH. Higher temperatures will increase the reaction rates for both hydrolysis and acylation.

Once the reaction is completed, the first protein is now activated, with a sulfhydryl reactive moiety. The activated protein may be isolated from the reaction mixture by simple gel filtration or dialysis. To carry out the second step of the cross-linking, the sulfhydryl reaction, the lipophilic group chosen for reaction with maleimides, activated halogens, or pyridyl disulfides must contain a free sulfhydryl. Alternatively, a primary amine may be modified with to add a sulfhydryl

In all cases, the buffer should be degassed to prevent oxidation of sulfhydryl groups. EDTA may be added to chelate any oxidizing metals that may be present in the buffer. Buffers should be free of any sulfhydryl containing compounds.

Maleimides react specifically with -SH groups at slightly acidic to neutral pH ranges (6.5-7.5). A neutral pH is sufficient for reactions involving halogens and pyridyl disulfides. Under these conditions, maleimides generally react with -SH groups within a matter of minutes. Longer reaction times are required for halogens and pyridyl disulfides.

The first sulfhydryl reactive-protein prepared in the amine reaction step is mixed with the sulfhydryl-containing lipophilic group under the appropriate buffer conditions. The conjugates can be isolated from the reaction mixture by methods such as gel filtration or by dialysis.

Exemplary activated lipophilic moieties for conjugation include: N-(1-pyrene)maleimide; 2,5-dimethoxystilbene-4'-maleimide, eosin-5-maleimide; fluorescein-5-maleimide; N-(4-(6-dimethylamino- 2-benzofuranyl)phenyl)maleimide; benzophenone-4-maleimide; 4-dimethylaminophenylazophenyl- 4'-maleimide (DABMI), tetramethylrhodamine-5-maleimide, tetramethylrhodamine-6-maleimide, Rhodamine RedTM C2 maleimide, N-(5-aminopentyl)maleimide, trifluoroacetic acid salt, N-(2-aminoethyl)maleimide, trifluoroacetic acid salt, Oregon GreenTM 488 maleimide, N-(2-((2-(((4-azido- 2,3,5,6-tetrafluoro)benzoyl) amino)ethyl)dithio)ethyl)maleimide (TFPAM-SS1), 2-(1-(3-dimethylaminopropyl) -indol-3-yl)-3-(indol-3-yl) maleimide (bisindolylmaleimide; GF 109203X), BODIPY® FL N-(2-aminoethyl)maleimide, N-(7-dimethylamino- 4-methylcoumarin-3-yl)maleimide (DACM), AlexaTM 488 C5 maleimide, AlexaTM 594 C5 maleimide, sodium saltN-(1-pyrene)maleimide, 2,5-dimethoxystilbene-4'-maleimide, eosin-5-maleimide, fluorescein-5-maleimide, N-(4-(6-dimethylamino- 2-benzofuranyl)phenyl)maleimide, benzophenone-4-maleimide, 4-dimethylaminophenylazophenyl- 4'-maleimide, 1-(2-maleimidylethyl)-4-(5- (4-methoxyphenyl)oxazol-2- yl)pyridinium methanesulfonate, tetramethylrhodamine-5-maleimide, tetramethylrhodamine-6-maleimide, Rhodamine RedTM C2 maleimide, N-(5-aminopentyl)maleimide, N-(2-aminoethyl)maleimide, N-(2-((2-(((4-azido- 2,3,5,6-tetrafluoro)benzoyl) amino)ethyl)dithio)ethyl)maleimide, 2-(1-(3-dimethylaminopropyl) - indol-3-yl)-3-(indol-3-yl) maleimide, N-(7-dimethylamino- 4-methylcoumarin-3-yl)maleimide (DACM), 11H-Benzo[a]fluorene, Benzo[a]pyrene.

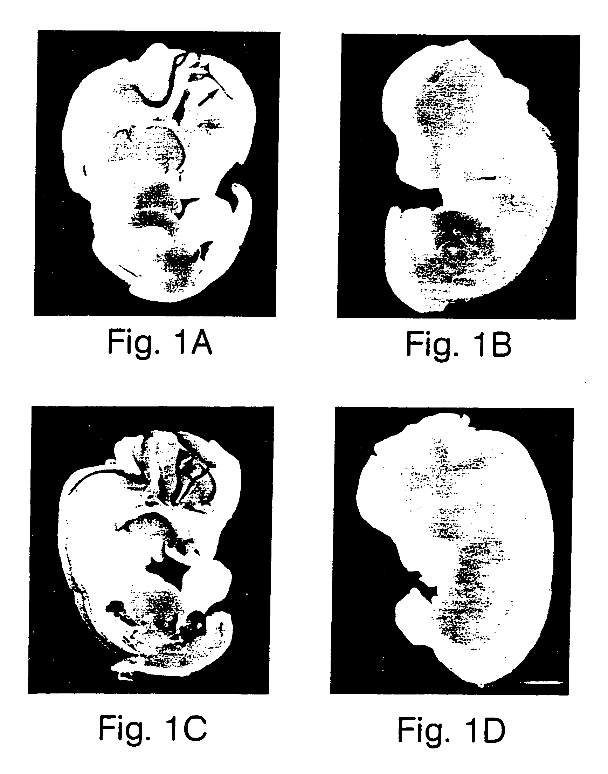

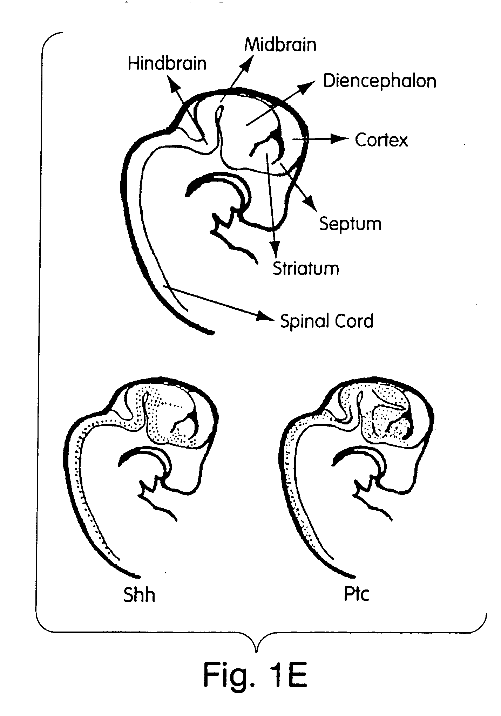

In one embodiment, the *hedgehog* polypeptide can be derivatived using pyrene maleimide, which can be purchased from Molecular Probes (Eugene, Oreg.), e.g., N-(1-pyrene)maleimide or 1-pyrenemethyl iodoacetate (PMIA ester). As illustrated in Figure 1, the pyrene-derived *hedgehog* polypeptide had an activity profile indicating that it was nearly 2 orders of magnitude more active than the unmodified form of the protein.

For those embodiments wherein the hydophobic moiety is a polypeptide, the modified *hedgehog* polypeptide of this invention can be constructed as a fusion protein, containing the *hedgehog* polypeptide and the hydrophobic moiety as one contiguous polypeptide chain.

In certain embodiments, the lipophilic moiety is an amphipathic polypeptide, such as magainin, cecropin, attacin, melittin, gramicidin S, alpha-toxin of Staph. aureus, alamethicin or a synthetic amphipathic polypeptide. Fusogenic coat proteins from viral particles can also be a convenient source of amphipathic sequences for the subject *hedgehog* polypeptides.

In still other embodiments, the subject method makes use of chimeric proteins comprising a hedgehog moiety fused or otherwise chemically linked to all or part of the hinge and constant regions of an immunoglobulin light chain, heavy chain, or both. Thus, this invention features a molecule which includes: (1) a hedgehog moiety, (2) a second peptide, e.g., one which increases solubility or in vivo life time of the hedgehog moiety, e.g., a member of the immunoglobulin super family or fragment or portion thereof, e.g., a portion or a fragment or portion thereof, e.g., a portion or a fragment of IgG, e.g., the human IgG1 heavy chain constant region, e.g., CH2, CH3, and hinge regions; and a toxin moiety.

Other embodiments are possible. Specifically, a "hedgehog/Ig fusion" is a protein comprising a biologically active hedgehog molecule of the invention (i.e., Sonic hedgehog), or a biologically active fragment thereof (i.e., the N-terminal portion) linked to an N-terminus of an immunoglobulin chain wherein a portion of the N-terminus of the immunoglobulin is replaced with the hedgehog. A species of hedgehog/Ig fusion is an "hedgehog/Fc Fusion" which is a protein comprising an hedgehog molecule of the invention (i.e., hedgehog -) linked to at least a part of the constant domain of an immunoglobulin. A preferred Fc fusion comprises a hedgehog mutein of the invention linked to a fragment of an antibody containing the C terminal domain of the heavy immunoglobulin chains. Also, the term "fusion protein" means an hedgehog protein chemically linked via a mono- or hetero- functional molecule to a second moiety that is not an hedgehog protein and is made de novo from purified protein as described below.

Generally, the structure of the a preferred chimeric hedgehog protein of this invention has the general formula: X-Y-Z, where wherein X is a polypeptide having the amino acid sequence, or portion thereof, consisting of the amino acid sequence of hedgehog; Y is an optional linker moiety; an dZ is a polypeptide comprising at least a portion of a polypeptide other than hedgehog. Preferably, X includes at least a biologically active N-terminal fragment of is human Sonic, Indian or Desert hedgehog. In the preferred embodiments, Z is a protein with an Ig-like constant and/or variable domain. Most preferably, Z is at least a portion of a constant region of an immunoglobulin and can be derived from an immunoglobulin of the class selected from IgM, IgG, IgD, IgA, and IgE. If the class is IgG, then it is selected from one of IgG1, IgG2, IgG3 and IgG4. The constant region of human IgM and IgE contain 4 constant regions (CH1, (hinge), CH2, CH3 and CH4, whereas the constant region of human IgG, IgA and IgD contain 3 constant regions (CH1, (hinge), CH2 and CH3. In the most preferred fusion proteins of the invention, the constant region contains at least the hinge, CH2 and CH3 domains.

Moreover, mutagenesis can be used to create modified *hh* polypeptides, e.g., for such purposes as enhancing therapeutic or prophylactic efficacy, or stability (e.g., *ex vivo* shelf life and resistance to proteolytic degradation *in vivo*). Such modified peptides can be produced, for instance, by amino acid substitution, deletion, or addition. Modified *hedgehog* polypeptides can also include those with altered post-translational processing relative to a naturally occurring *hedgehog* polypeptide, e.g., altered glycosylation, cholesterolization, prenylation and the like.

In one embodiment, the *hedgehog* therapeutic is a polypeptide encodable by a nucleotide sequence that hybridizes under stringent conditions to a *hedgehog* coding sequence represented in one or more of SEQ ID Nos:1-9 or 19. Appropriate stringency conditions which promote DNA hybridization, for example, 6.0 x sodium chloride/sodium citrate (SSC) at about 45°C, followed by a wash of 2.0 x SSC at 50°C, are known to those skilled in the art or can be found in *Current Protocols in Molecular Biology,* John Wiley & Sons, N.Y. (1989), 6.3.1-6.3.6. For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22°C, to high stringency conditions at about 65°C.

As described in the literature, genes for other *hedgehog* polypeptides, e.g., from other animals, can be obtained from mRNA or genomic DNA samples using techniques well known in the art. For example, a cDNA encoding a *hedgehog* polypeptide can be obtained by isolating total mRNA from a cell, e.g. a mammalian cell, e.g. a human cell, including embryonic cells. Double stranded cDNAs can then be prepared from the total mRNA, and subsequently inserted into a suitable plasmid or bacteriophage vector using any one of a number of known techniques. The gene encoding a *hedgehog* polypeptide can also be cloned using established polymerase chain reaction techniques.

Preferred nucleic acids encode a *hedgehog* polypeptide comprising an amino acid sequence at least 60% homologous, more preferably 70% homologous and most preferably 80% homologous with an amino acid sequence selected from the group consisting of SEQ ID Nos:8-14. Nucleic acids which encode polypeptides at least about 90%, more preferably at least about 95%, and most preferably at least about 98-99% homology with an amino acid sequence represented in one of SEQ ID Nos:10-18 or 20 are also within the scope of the invention.

*Hedgehog* polypeptides preferred by the present invention, in addition to native *hedgehog* polypeptides, are at least 60% homologous, more preferably 70% homologous and most preferably 80% homologous with an amino acid sequence represented by any of SEQ ID Nos:10-18 or 20. Polypeptides which are at least 90%, more preferably at least 95%, and most preferably at least about 98-99% homologous with a sequence selected from the group consisting of SEQ ID Nos:10-18 or 20 are also within the scope of the invention. The only prerequisite is that the *hedgehog* polypeptide is capable of protecting neuronal cells against degeneration, e.g., the polypeptide is trophic for a dopaminergic and/or GABAergic neuron.

The term "recombinant protein" refers to a polypeptide of the present invention which is produced by recombinant DNA techniques, wherein generally, DNA encoding a *hedgehog* polypeptide is inserted into a suitable expression vector which is in turn used to transform a host cell to produce the heterologous protein. Moreover, the phrase "derived from", with respect to a recombinant *hedgehog* gene, is meant to include within the meaning of "recombinant protein" those proteins having an amino acid sequence of a native *hedgehog* polypeptide, or an amino acid sequence similar thereto which is generated by mutations including substitutions and deletions (including truncation) of a naturally occurring form of the protein.

The method of the present invention can also be carried out using variant forms of the naturally occurring *hedgehog* polypeptides, e.g., mutational variants.

As is known in the art, *hedgehog* polypeptides can be produced by standard biological techniques. For example, a host cell transfected with a nucleic acid vector directing expression of a nucleotide sequence encoding the subject polypeptides can be cultured under appropriate conditions to allow expression of the peptide to occur. The polypeptide *hedgehog* may be secreted and isolated from a mixture of cells and medium containing the recombinant *hedgehog* polypeptide. Alternatively, the peptide may be retained cytoplasmically by removing the signal peptide sequence from the recombinant *hedgehog* gene and the cells harvested, lysed and the protein isolated. A cell culture includes host cells, media and other byproducts. Suitable media for cell culture are well known in the art. The recombinant *hedgehog* polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant *hedgehog* polypeptide is a fusion protein containing a domain which facilitates its purification, such as an *hedgehog*/GST fusion protein. The host cell may be any prokaryotic or eukaryotic cell.

Recombinant *hedgehog* genes can be produced by ligating nucleic acid encoding an *hedgehog* polypeptide, or a portion thereof, into a vector suitable for expression in either prokaryotic cells, eukaryotic cells, or both. Expression vectors for production of recombinant forms of the subject *hedgehog* polypeptides include plasmids and other vectors. For instance, suitable vectors for the expression of a *hedgehog* polypeptide include plasmids of the types: pBR322-derived plasmids, pEMBL-derived plasmids, pEX-derived plasmids, pBTac-derived plasmids and pUC-derived plasmids for expression in prokaryotic cells, such as *E. coli.*

A number of vectors exist for the expression of recombinant proteins in yeast. For instance, YEP24, YIP5, YEP51, YEP52, pYES2, and YRP17 are cloning and expression vehicles useful in the introduction of genetic constructs into *S*. *cerevisiae* (see, for example, Broach *et al*. (1983) in *Experimental Manipulation of Gene Expression,* ed. M. Inouye Academic Press, p. 83, incorporated by reference herein). These vectors can replicate in *E*. *coli* due the presence of the pBR322 ori, and in *S. cerevisiae* due to the replication determinant of the yeast 2 micron plasmid. In addition, drug resistance markers such as ampicillin can be used. In an illustrative embodiment, an *hedgehog* polypeptide is produced recombinantly utilizing an expression vector generated by sub-cloning the coding sequence of one of the *hedgehog* genes represented in SEQ ID Nos: 1-9 or 19.

The preferred mammalian expression vectors contain both prokaryotic sequences, to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see *Molecular Cloning A Laboratory Manual,* 2nd Ed., ed. by Sambrook, Fritsch and Maniatis (Cold Spring Harbor Laboratory Press: 1989) Chapters 16 and 17.

In some instances, it may be desirable to express the recombinant *hedgehog* polypeptide by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the β-gal containing pBlueBac III).

When it is desirable to express only a portion of a *hedgehog* polypeptide, such as a form lacking a portion of the N-terminus, i.e. a truncation mutant which lacks the signal peptide, it may be necessary to add a start codon (ATG) to the oligonucleotide fragment containing the desired sequence to be expressed. It is well known in the art that a methionine at the N-terminal position can be enzymatically cleaved by the use of the enzyme methionine aminopeptidase (MAP). MAP has been cloned from *E. coli* (Ben-Bassat et al. (1987) *J. Bacteriol.* 169:751-757) and *Salmonella typhimurium* and its *in vitro* activity has been demonstrated on recombinant proteins (Miller et al. (1987) *PNAS 84*:2718-1722). Therefore, removal of an N-terminal methionine, if desired, can be achieved either *in vivo* by expressing *hedgehog*-derived polypeptides in a host which produces MAP (e.g., *E. coli* or CM89 or *S. cerevisiae*), or *in vitro* by use of purified MAP (e.g., procedure of Miller et al., *supra*).

Alternatively, the coding sequences for the polypeptide can be incorporated as a part of a fusion gene including a nucleotide sequence encoding a different polypeptide. It is widely appreciated that fusion proteins can also facilitate the expression of proteins, and accordingly, can be used in the expression of the *hedgehog* polypeptides of the present invention. For example, *hedgehog* polypeptides can be generated as glutathione-S-transferase (GST-fusion) proteins. Such GST-fusion proteins can enable easy purification of the *hedgehog* polypeptide, as for example by the use of glutathione-derivatized matrices (see, for example, *Current Protocols in Molecular Biology,* eds. Ausubel et al. (N.Y.: John Wiley & Sons, 1991)). In another embodiment, a fusion gene coding for a purification leader sequence, such as a poly-(His)/enterokinase cleavage site sequence, can be used to replace the signal sequence which naturally occurs at the N-terminus of the *hedgehog* polypeptide (e.g.of the pro-form, in order to permit purification of the poly(His)*-hedgehog* polypeptide by affinity chromatography using a Ni²⁺ metal resin. The purification leader sequence can then be subsequently removed by treatment with enterokinase (e.g., see Hochuli et al. (1987) *J*. *Chromatography* 411:177; and Janknecht et al. *PNAS* 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, *Current Protocols in Molecular Biology,* eds. Ausubel et al. John Wiley & Sons: 1992).

*Hedgehog* polypeptides may also be chemically modified to create *hedgehog* derivatives by forming covalent or aggregate conjugates with other chemical moieties, such as glycosyl groups, cholesterol, isoprenoids, lipids, phosphate, acetyl groups and the like. Covalent derivatives of *hedgehog* polypeptides can be prepared by linking the chemical moieties to functional groups on amino acid sidechains of the protein or at the N-terminus or at the C-terminus of the polypeptide.

For instance, *hedgehog* polypeptides can be generated to include a moiety, other than sequence naturally associated with the protein, that binds a component of the extracellular matrix and enhances localization of the analog to cell surfaces. For example, sequences derived from the fibronectin "type-III repeat", such as a tetrapeptide sequence R-G-D-S (Pierschbacher et al. (1984) *Nature* 309:30-3; and Kornblihtt et al. (1985) *EMBO* 4:1755-9) can be added to the *hedgehog* polypeptide to support attachment of the chimeric molecule to a cell through binding ECM components (Ruoslahti et al. (1987) *Science* 238:491-497; Pierschbacheret al. (1987) *J. Biol. Chem.* 262:17294-8.; Hynes (1987) *Cell* 48:549-54; and Hynes (1992) *Cell* 69:11-25).

In preferred embodiment, the *hedgehog* polypeptide is isolated from, or is otherwise substantially free of, other cellular proteins, especially other extracellular or cell surface associated proteins which may normally be associated with the *hedgehog* polypeptide. The term "substantially free of other cellular or extracellular proteins" (also referred to herein as "contaminating proteins") or "substantially pure or purified preparations" are defined as encompassing preparations of *hedgehog* polypeptides having less than 20% (by dry weight) contaminating protein, and preferably having less than 5% contaminating protein. By "purified", it is meant that the indicated molecule is present in the substantial absence of other biological macromolecules, such as other proteins. The term "purified" as used herein preferably means at least 80% by dry weight, more preferably in the range of 95-99% by weight, and most preferably at least 99.8% by weight, of biological macromolecules of the same type present (but water, buffers, and other small molecules, especially molecules having a molecular weight of less than 5000, can be present). The term "pure" as used herein preferably has the same numerical limits as "purified" immediately above.

As described above for recombinant polypeptides, isolated *hedgehog* polypeptides can include all or a portion of the amino acid sequences represented in any of SEQ ID Nos:10-18 or 20, or a homologous sequence thereto. Preferred fragments of the subject *hedgehog* polypeptides correspond to the N-terminal and C-terminal proteolytic fragments of the mature protein. Bioactive fragments of *hedgehog* polypeptides are described in great detail in PCT publications WO 95/18856 and WO 96/17924.

With respect to bioctive fragments of *hedgehog* polypeptide, preferred *hedgehog* therapeutics include at least 50 amino acid residues of a *hedgehog* polypeptide, more preferably at least 100, and even more preferably at least 150.

Another preferred *hedgehog* polypeptide which can be included in the *hedgehog* therapeutic is an N-terminal fragment of the mature protein having a molecular weight of approximately 19 kDa.

Preferred human *hedgehog* polypeptides include N-terminal fragments corresponding approximately to residues 24-197 of SEQ ID No. 15, 28-202 of SEQ ID No. 16, and 23-198 of SEQ ID No. 17. By "corresponding approximately" it is meant that the sequence of interest is at most 20 amino acid residues different in length to the reference sequence, though more preferably at most 5, 10 or 15 amino acid different in length.

Still other preferred *hedgehog* polypeptides includes an amino acid sequence represented by the formula A-B wherein: (i) A represents all or the portion of the amino acid sequence designated by residues 1-168 of SEQ ID No:21; and B represents at least one amino acid residue of the amino acid sequence designated by residues 169-221 of SEQ ID No:21; (ii) A represents all or the portion of the amino acid sequence designated by residues 24-193 of SEQ ID No: 15; and B represents at least one amino acid residue of the amino acid sequence designated by residues 194-250 of SEQ ID No:15; (iii) A represents all or the portion of the amino acid sequence designated by residues 25-193 of SEQ ID No:13; and B represents at least one amino acid residue of the amino acid sequence designated by residues 194-250 of SEQ ID No:13; (iv) A represents all or the portion of the amino acid sequence designated by residues 23-193 of SEQ ID No: 11; and B represents at least one amino acid residue of the amino acid sequence designated by residues 194-250 of SEQ ID No: 11; (v) A represents all or the portion of the amino acid sequence designated by residues 28-197 of SEQ ID No:12; and B represents at least one amino acid residue of the amino acid sequence designated by residues 198-250 of SEQ ID No: 12; (vi) A represents all or the portion of the amino acid sequence designated by residues 29-197 of SEQ ID No:16; and B represents at least one amino acid residue of the amino acid sequence designated by residues 198-250 of SEQ ID No:16; or (vii) A represents all or the portion of the amino acid sequence designated by residues 23-193 of SEQ ID No. 17, and B represents at least one amino acid residue of the amino acid sequence designated by residues 194-250 of SEQ ID No. 17. In certain preferred embodiments, A and B together represent a contiguous polypeptide sequence designated sequence, A represents at least 25, 50, 75, 100, 125 or 150 amino acids of the designated sequence, and B represents at least 5, 10, or 20 amino acid residues of the amino acid sequence designated by corresponding entry in the sequence listing, and A and B together preferably represent a contiguous sequence corresponding to the sequence listing entry. Similar fragments from other *hedgehog* also contemplated, e.g., fragments which correspond to the preferred fragments from the sequence listing entries which are enumerated above.

Isolated peptidyl portions of *hedgehog* polypeptides can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a *hedgehog* polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as agonists of a wild-type (e.g., "authentic") *hedgehog* polypeptide. For example, Román et al. (1994) *Eur J Biochem* 222:65-73 describe the use of competitive-binding assays using short, overlapping synthetic peptides from larger proteins to identify binding domains.

The recombinant *hedgehog* polypeptides of the present invention also include homologs of the authentic *hedgehog* polypeptides, such as versions of those protein which are resistant to proteolytic cleavage, as for example, due to mutations which alter potential cleavage sequences or which inactivate an enzymatic activity associated with the protein. *Hedgehog* homologs of the present invention also include proteins which have been post-translationally modified in a manner different than the authentic protein. Exemplary derivatives of *hedgehog* polypeptides include polypeptides which lack glycosylation sites (e.g. to produce an unglycosylated protein), which lack sites for cholesterolization, and/or which lack N-terminal and/or C-terminal sequences.

Modification of the structure of the subject *hedgehog* polypeptides can also be for such purposes as enhancing therapeutic or prophylactic efficacy, or stability (e.g., *ex vivo* shelf life and resistance to proteolytic degradation *in vivo*). Such modified peptides, when designed to retain at least one activity of the naturally-occurring form of the protein, are considered functional equivalents of the *hedgehog* polypeptides described in more detail herein. Such modified peptides can be produced, for instance, by amino acid substitution, deletion, or addition.

It is well known in the art that certain isolated replacements of amino acids, e.g., replacement of an amino acid residue with another related amino acid (i.e. isosteric and/or isoelectric mutations), can be carried out without major effect on the biological activity of the resulting molecule. Conservative replacements are those that take place within a family of amino acids that are related in their side chains. Genetically encoded amino acids are can be divided into four families: (I) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan, and tyrosine are sometimes classified jointly as aromatic amino acids. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur -containing = cysteine and methionine. (see, for example, *Biochemistry,* 2nd ed., Ed. by L. Stryer, WH Freeman and Co.: 1981). Whether a change in the amino acid sequence of a peptide results in a functional *hedgehog* homolog (e.g. functional in the sense that it acts to mimic or antagonize the wild-type form) can be readily determined by assessing the ability of the variant peptide to produce a response in cells in a fashion similar to the wild-type protein, or competitively inhibit such a response. Polypeptides in which more than one replacement has taken place can readily be tested in the same manner.

It is specifically contemplated that the methods of the present invention can be carried using homologs of naturally occurring *hedgehog* polypeptides. In one embodiment, the invention contemplates using *hedgehog* polypeptides generated by combinatorial mutagenesis. Such methods, as are known in the art, are convenient for generating both point and truncation mutants, and can be especially useful for identifying potential variant sequences (e.g. homologs) that are functional in binding to a receptor for *hedgehog* polypeptides. The purpose of screening such combinatorial libraries is to generate, for example, novel *hedgehog* homologs which can act as neuroprotective agents. To illustrate, *hedgehog* homologs can be engineered by the present method to provide more efficient binding to a cognate receptor, such as *patched,* retaining neuroprotective activity. Thus, combinatorially-derived homologs can be generated to have an increased potency relative to a naturally occurring form of the protein. Moreover, manipulation of certain domains of *hedgehog* by the present method can provide domains more suitable for use in fusion proteins, such as one that incorporates portions of other proteins which are derived from the extracellular matrix and/or which bind extracellular matrix components.

To further illustrate the state of the art of combinatorial mutagenesis, it is noted that the review article of Gallop et al. (1994) *J Med Chem* 37:1233 describes the general state of the art of combinatorial libraries as of the earlier 1990's. In particular, Gallop et al state at page 1239 "[s]creening the analog libraries aids in determining the minimum size of the active sequence and in identifying those residues critical for binding and intolerant of substitution". In addition, the Ladner et al. PCT publication WO90/02809, the Goeddel et al. U.S. Patent 5,223,408, and the Markland et al. PCT publication WO92/15679 illustrate specific techniques which one skilled in the art could utilize to generate libraries of *hedgehog* variants which can be rapidly screened to identify variants/fragments which retained a particular activity of the *hedgehog* polypeptides. These techniques are exemplary of the art and demonstrate that large libraries of related variants/truncants can be generated and assayed to isolate particular variants without undue experimentation. Gustin et al. (1993) *Virology* 193:653, and Bass et al. (1990) *Proteins: Structure, Function and Genetics* 8:309-314
also describe other exemplary techniques from the art which can be adapted as means for generating mutagenic variants of *hedgehog* polypeptides.

Indeed, it is plain from the combinatorial mutagenesis art that large scale mutagenesis of *hedgehog* polypeptides, without any preconceived ideas of which residues were critical to the biological function, and generate wide arrays of variants having equivalent biological activity. Indeed, it is the ability of combinatorial techniques to screen billions of different variants by high throughout analysis that removes any requirement of *a priori* understanding or knowledge of critical residues.

To illsutrate, the amino acid sequences for a population of *hedgehog* homologs or other related proteins are aligned, preferably to promote the highest homology possible. Such a population of variants can include, for example, *hedgehog* homologs from one or more species. Amino acids which appear at each position of the aligned sequences are selected to create a degenerate set of combinatorial sequences. In a preferred embodiment, the variegated library of *hedgehog* variants is generated by combinatorial mutagenesis at the nucleic acid level, and is encoded by a variegated gene library. For instance, a mixture of synthetic oligonucleotides can be enzymatically ligated into gene sequences such that the degenerate set of potential *hedgehog* sequences are expressible as individual polypeptides, or alternatively, as a set of larger fusion proteins (e.g. for phage display) containing the set of *hedgehog* sequences therein.

As illustrated in PCT publication WO 95/18856, to analyze the sequences of a population of variants, the amino acid sequences of interest can be aligned relative to sequence homology. The presence or absence of amino acids from an aligned sequence of a particular variant is relative to a chosen consensus length of a reference sequence, which can be real or artificial.

In an illustrative embodiment, alignment of exons 1, 2 and a portion of exon 3 encoded sequences (e.g. the N-terminal approximately 221 residues of the mature protein) of each of the *Shh* clones produces a degenerate set of *Shh* polypeptides represented by the general formula: wherein each of the degenerate positions "X" can be an amino acid which occurs in that position in one of the human, mouse, chicken or zebrafish *Shh* clones, or, to expand the library, each X can also be selected from amongst amino acid residue which would be conservative substitutions for the amino acids which appear naturally in each of those positions. For instance, Xaa(1) represents Gly, Ala, Val, Leu, Ile, Phe, Tyr or Trp ; Xaa(2) represents Arg, His or Lys; Xaa(3) represents Gly, Ala, Val, Leu, Ile, Ser or Thr; Xaa(4) represents Gly, Ala, Val, Leu, Ile, Ser or Thr; Xaa(5) represents Lys, Arg, His, Asn or Gln; Xaa(6) represents Lys, Arg or His; Xaa(7) represents Ser, Thr, Tyr, Trp or Phe; Xaa(8) represents Lys, Arg or His; Xaa(9) represents Met, Cys, Ser or Thr; Xaa(10) represents Gly, Ala, Val, Leu, Ile, Ser or Thr; Xaa(11) represents Leu, Val, Met, Thr or Ser; Xaa(12) represents His, Phe, Tyr, Ser, Thr, Met or Cys; Xaa(13) represents Gln, Asn, Glu, or Asp; Xaa(14) represents His, Phe, Tyr, Thr, Gln, Asn, Glu or Asp; Xaa(15) represents Gln, Asn, Glu, Asp, Thr, Ser, Met or Cys; Xaa(16) represents Ala, Gly, Cys, Leu, Val or Met; Xaa(17) represents Arg, Lys, Met, Ile, Asn, Asp, Glu, Gln, Ser, Thr or Cys; Xaa(18) represents Arg, Lys, Met or Ile; Xaa(19) represents Ala, Gly, Cys, Asp, Glu, Gln, Asn, Ser, Thr or Met; Xaa(20) represents Ala, Gly, Cys, Asp, Asn, Glu or Gln; Xaa(21) represents Arg, Lys, Met, Ile, Asn, Asp, Glu or Gln; Xaa(22) represent Leu, Val, Met or Ile; Xaa(23) represents Phe, Tyr, Thr, His or Trp; Xaa(24) represents Ile, Val, Leu or Met; Xaa(25) represents Met, Cys, Ile, Leu, Val, Thr or Ser; Xaa(26) represents Leu, Val, Met, Thr or Ser. In an even more expansive library, each X can be selected from any amino acid.

In similar fashion, alignment of each of the human, mouse, chicken and zebrafish *hedgehog* clones, can provide a degenerate polypeptide sequence represented by the general formula: wherein, as above, each of the degenerate positions "X" can be an amino acid which occurs in a corresponding position in one of the wild-type clones, and may also include amino acid residue which would be conservative substitutions, or each X can be any amino acid residue. In an exemplary embodiment, Xaa(1) represents Gly, Ala, Val, Leu, Ile, Pro, Phe or Tyr; Xaa(2) represents Gly, Ala, Val, Leu or Ile; Xaa(3) represents Gly, Ala, Val, Leu, Ile, Lys, His or Arg; Xaa(4) represents Lys, Arg or His; Xaa(5) represents Phe, Trp, Tyr or an amino acid gap; Xaa(6) represents Gly, Ala, Val, Leu, Ile or an amino acid gap; Xaa(7) represents Asn, GIn, His, Arg or Lys; Xaa(8) represents Gly, Ala, Val, Leu, Ile, Ser or Thr; Xaa(9) represents Gly, Ala, Val, Leu, Ile, Ser or Thr; Xaa(10) represents Gly, Ala, Val, Leu, Ile, Ser or Thr; Xaa(11) represents Ser, Thr, Gln or Asn; Xaa(12) represents Met, Cys, Gly, Ala, Val, Leu, Ile, Ser or Thr; Xaa(13) represents Gly, Ala, Val, Leu, Ile or Pro; Xaa(14) represents Arg, His or Lys; Xaa(15) represents Gly, Ala, Val, Leu, Ile, Pro, Arg, His or Lys; Xaa(16) represents Gly, Ala, Val, Leu, Ile, Phe or Tyr; Xaa(17) represents Arg, His or Lys; Xaa(18) represents Gly, Ala, Val, Leu, Ile, Ser or Thr; Xaa(19) represents Thr or Ser; Xaa(20) represents Gly, Ala, Val, Leu, Ile, Asn or Gln; Xaa(21) represents Arg, His or Lys; Xaa(22) represents Asp or Glu; Xaa(23) represents Ser or Thr; Xaa(24) represents Glu, Asp, Gln or Asn; Xaa(25) represents Glu or Asp; Xaa(26) represents Arg, His or Lys; Xaa(27) represents Gly, Ala, Val, Leu or Ile; Xaa(28) represents Gly, Ala, Val, Leu, Ile, Thr or Ser; Xaa(29) represents Met, Cys, Gln, Asn, Arg, Lys or His; Xaa(30) represents Arg, His or Lys; Xaa(31) represents Trp, Phe, Tyr, Arg, His or Lys; Xaa(32) represents Gly, Ala, Val, Leu, Ile, Ser, Thr, Tyr or Phe; Xaa(33) represents Gln, Asn, Asp or Glu; Xaa(34) represents Asp or Glu; Xaa(35) represents Gly, Ala, Val, Leu, or Ile; Xaa(36) represents Arg, His or Lys; Xaa(37) represents Asn, GIn, Thr or Ser; Xaa(38) represents Gly, Ala, Val, Leu, Ile, Ser, Thr, Met or Cys; Xaa(39) represents Gly, Ala, Val, Leu, Ile, Thr or Ser; Xaa(40) represents Arg, His or Lys; Xaa(41) represents Asn, Gln, Gly, Ala, Val, Leu or Ile; Xaa(42) represents Gly, Ala, Val, Leu or Ile; Xaa(43) represents Gly, Ala, Val, Leu, Ile, Ser, Thr or Cys; Xaa(44) represents Gly, Ala, Val, Leu, Ile, Thr or Ser; and Xaa(45) represents Asp or Glu.

There are many ways by which the library of potential *hedgehog* homologs can be generated from a degenerate oligonucleotide sequence. Chemical synthesis of a degenerate gene sequence can be carried out in an automatic DNA synthesizer, and the synthetic genes then ligated into an appropriate expression vector. The purpose of a degenerate set of genes is to provide, in one mixture, all of the sequences encoding the desired set of potential *hedgehog* sequences. The synthesis of degenerate oligonucleotides is well known in the art (see for example, Narang, SA (1983) *Tetrahedron* 39:3; Itakura et al. (1981) *Recombinant DNA, Proc 3rd Cleveland Sympos. Macromolecules,* ed. AG Walton, Amsterdam: Elsevier pp273-289; Itakura et al. (1984) *Annu. Rev. Biochem.* 53:323; Itakura et al. (1984) *Science* 198:1056; Ike et al. (1983) *Nucleic Acid Res.* 11:477. Such techniques have been employed in the directed evolution of other proteins (see, for example, Scott et al. (1990) *Science* 249:386-390; Roberts et al. (1992) *PNAS* 89:2429-2433; Devlin et al. (1990) *Science* 249: 404-406; Cwirla et al. (1990) *PNAS* 87: 6378-6382; as well as U.S. Patents Nos. 5,223,409, 5,198,346, and 5,096,815).

A wide range of techniques are known in the art for screening gene products of combinatorial libraries made by point mutations, and for screening cDNA libraries for gene products having a certain property. Such techniques will be generally adaptable for rapid screening of the gene libraries generated by the combinatorial mutagenesis of *hedgehog* homologs. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Each of the illustrative assays described below are amenable to high through-put analysis as necessary to screen large numbers of degenerate *hedgehog* sequences created by combinatorial mutagenesis techniques.

In one embodiment, the combinatorial library is designed to be secreted (e.g. the polypeptides of the library all include a signal sequence but no transmembrane or cytoplasmic domains), and is used to transfect a eukaryotic cell that can be co-cultured with neuronal cells. A functional *hedgehog* polypeptide secreted by the cells expressing the combinatorial library will diffuse to neighboring neuronal cells and induce a particular biological response, such as protection against cell death when treated with MPTP. The pattern of protection will resemble a gradient function, and will allow the isolation (generally after several repetitive rounds of selection) of cells producing *hedgehog* homologs active as neuroprotective agents with respect to the target neuronal cells

To illustrate, target neuronal cells are cultured in 24-well microtitre plates. Other eukaryotic cells are transfected with the combinatorial *hedgehog* gene library and cultured in cell culture inserts (e.g. Collaborative Biomedical Products, Catalog #40446) that are able to fit into the wells of the microtitre plate. The cell culture inserts are placed in the wells such that recombinant *hedgehog* homologs secreted by the cells in the insert can diffuse through the porous bottom of the insert and contact the target cells in the microtitre plate wells. After a period of time sufficient for functional forms of a *hedgehog* polypeptide to produce a measurable response in the target cells, such as neuroprotection, the inserts are removed and the effect of the variant *hedgehog* polypeptides on the target cells determined. Cells from the inserts corresponding to wells which score positive for activity can be split and re-cultured on several inserts, the process being repeated until the active clones are identified.

In yet another screening assay, the candidate *hedgehog* gene products are displayed on the surface of a cell or viral particle, and the ability of particular cells or viral particles to associate with a *hedgehog*-binding moiety (such as the *patched* protein or other *hedgehog* receptor) via this gene product is detected in a "panning assay". Such panning steps can be carried out on cells cultured from embryos. For instance, the gene library can be cloned into the gene for a surface membrane protein of a bacterial cell, and the resulting fusion protein detected by panning (Ladner et al., WO 88/06630; Fuchs et al. (1991) *Bio*/*Technology* 9:1370-1371; and Goward et al. (1992) *TIBS* 18:136-140). In a similar fashion, fluorescently labeled molecules which bind *hedgehog* can be used to score for potentially functional *hedgehog* homologs. Cells can be visually inspected and separated under a fluorescence microscope, or, where the morphology of the cell permits, separated by a fluorescence-activated cell sorter.

In an alternate embodiment, the gene library is expressed as a fusion protein on the surface of a viral particle. For instance, in the filamentous phage system, foreign peptide sequences can be expressed on the surface of infectious phage, thereby conferring two significant benefits. First, since these phage can be applied to affinity matrices at very high concentrations, large number of phage can be screened at one time. Second, since each infectious phage displays the combinatorial gene product on its surface, if a particular phage is recovered from an affinity matrix in low yield, the phage can be amplified by another round of infection. The group of almost identical *E.coli* filamentous phages M13, fd, and f1 are most often used in phage display libraries, as either of the phage gIII or gVIII coat proteins can be used to generate fusion proteins without disrupting the ultimate packaging of the viral particle (Ladner et al. PCT publication WO 90/02909; Garrard et al., PCT publication WO 92/09690; Marks et al. (1992) J. Biol. Chem. 267:16007-16010; Griffths et al. (1993) EMBO J 12:725-734; Clackson et al. (1991) Nature 352:624-628; and Barbas et al. (1992) PNAS 89:4457-4461).

In an illustrative embodiment, the recombinant phage antibody system (RPAS, Pharamacia Catalog number 27-9400-01) can be easily modified for use in expressing and screening *hedgehog* combinatorial libraries. For instance, the pCANTAB 5 phagemid of the RPAS kit contains the gene which encodes the phage gIII coat protein. The *hedgehog* combinatorial gene library can be cloned into the phagemid adjacent to the gIII signal sequence such that it will be expressed as a gIII fusion protein. After ligation, the phagemid is used to transform competent *E*. *coli* TG1 cells. Transformed cells are subsequently infected with M13KO7 helper phage to rescue the phagemid and its candidate *hedgehog* gene insert. The resulting recombinant phage contain phagemid DNA encoding a specific candidate *hedgehog,* and display one or more copies of the corresponding fusion coat protein. The phage-displayed candidate *hedgehog* polypeptides which are capable of binding an *hedgehog* receptor are selected or enriched by panning. For instance, the phage library can be applied to cells which express the *patched* protein and unbound phage washed away from the cells. The bound phage is then isolated, and if the recombinant phage express at least one copy of the wild type gIII coat protein, they will retain their ability to infect *E. coli*. Thus, successive rounds of reinfection of *E. coli,* and panning will greatly enrich for *hedgehog* homologs, which can then be screened for further biological activities in order to differentiate agonists and antagonists.

Combinatorial mutagenesis has a potential to generate very large libraries of mutant proteins, e.g., in the order of 10²⁶ molecules. Combinatorial libraries of this size may be technically challenging to screen even with high throughput screening assays such as phage display. To overcome this problem, a new technique has been developed recently, recrusive ensemble mutagenesis (REM), which allows one to avoid the very high proportion of non-functional proteins in a random library and simply enhances the frequency of functional proteins, thus decreasing the complexity required to achieve a useful sampling of sequence space. REM is an algorithm which enhances the frequency of functional mutants in a library when an appropriate selection or screening method is employed (Arkin and Yourvan, 1992, *PNAS USA* 89:7811-7815; Yourvan et al., 1992, *Parallel Problem Solving from Nature,* 2., In Maenner and Manderick, eds., Elsevir Publishing Co., Amsterdam, pp. 401-410; Delgrave et al., 1993, *Protein Engineering* 6(3):327-331).

The invention also provides for reduction of the *hedgehog* polypeptide to generate mimetics, e.g. peptide or non-peptide agents, which are able to mimic the neuroprotective activity of a naturally-occurring *hedgehog* polypeptide. Thus, such mutagenic techniques as described above are also useful to map the determinants of the *hedgehog* polypeptides which participate in protein-protein interactions involved in, for example, binding of the subject *hedgehog* polypeptide to other extracellular matrix components such as its receptor(s). To illustrate, the critical residues of a subject *hedgehog* polypeptide which are involved in molecular recognition of an *hedgehog* receptor such as *patched* can be determined and used to generate *hedgehog*-derived peptidomimetics which competitively bind with that moiety. By employing, for example, scanning mutagenesis to map the amino acid residues of each of the subject *hedgehog* polypeptides which are involved in binding other extracellular proteins, peptidomimetic compounds can be generated which mimic those residues of the *hedgehog* polypeptide which facilitate the interaction. After distinguishing between agonist and antagonists, such agonistic mimetics may be used to mimic the normal function of a *hedgehog* polypeptide as trophic for dopaminergic and GABAergic neurons. For instance, non-hydrolyzable peptide analogs of such residues can be generated using benzodiazepine (e.g., see Freidinger et al. in *Peptides: Chemistry and Biology,* G.R. Marshall ed., ESCOM Publisher: Leiden, Netherlands, 1988), azepine (e.g., see Huffman et al. in *Peptides: Chemistry and Biology,* G.R. Marshall ed., ESCOM Publisher: Leiden, Netherlands, 1988), substituted gama lactam rings (Garvey et al. in *Peptides: Chemistry and Biology,* G.R. Marshall ed., ESCOM Publisher: Leiden, Netherlands, 1988), keto-methylene pseudopeptides (Ewenson et al. (1986) *J Med Chem* 29:295; and Ewenson et al. in *Peptides: Structure and Function* (Proceedings of the 9th American Peptide Symposium) Pierce Chemical Co. Rockland, IL, 1985), β-turn dipeptide cores (Nagai et al. (1985) *Tetrahedron Lett* 26:647; and Sato et al. (1986) *J Chem Soc Perkin Trans* 1:1231), and β-aminoalcohols (Gordon et al. (1985) *Biochem Biophys Res Commun* 126:419; and Dann et al. (1986) *Biochem Biophys Res Commun* 134:71).

Recombinantly produced forms of the *hedgehog* polypeptides can be produced using, e.g, expression vectors containing a nucleic acid encoding a *hedgehog* polypeptide, operably linked to at least one transcriptional regulatory sequence. Operably linked is intended to mean that the nucleotide sequence is linked to a regulatory sequence in a manner which allows expression of the nucleotide sequence. Regulatory sequences are art-recognized and are selected to direct expression of a *hedgehog* polypeptide. Accordingly, the term transcriptional regulatory sequence includes promoters, enhancers and other expression control elements. Such regulatory sequences are described in Goeddel; *Gene Expression Technology: Methods in Enzymology* 185, Academic Press, San Diego, CA (1990). For instance, any of a wide variety of expression control sequences, sequences that control the expression of a DNA sequence when operatively linked to it, may be used in these vectors to express DNA sequences encoding *hedgehog* polypeptide. Such useful expression control sequences, include, for example, a viral LTR, such as the LTR of the Moloney murine leukemia virus, the early and late promoters of SV40, adenovirus or cytomegalovirus immediate early promoter, the lac system, the trp system, the TAC or TRC system, T7 promoter whose expression is directed by T7 RNA polymerase, the major operator and promoter regions of phage λ, the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other proteins encoded by the vector, such as antibiotic markers, should also be considered.

In addition to providing a ready source of *hedgehog* polypeptides for purification, the gene constructs of the present invention can also be used as a part of a gene therapy protocol to deliver nucleic acids encoding a neuroprotective form of a *hedgehog* polypeptide. Thus, another aspect of the invention features expression vectors for *in vivo* transfection of a *hedgehog* polypeptide in particular cell types so as cause ectopic expression of a *hedgehog* polypeptide in neuronal tissue.

Formulations of such expression constructs may be administered in any biologically effective carrier, e.g. any formulation or composition capable of effectively delivering the recombinant gene to cells *in vivo.* Approaches include insertion of the *hedgehog* coding sequence in viral vectors including recombinant retroviruses, adenovirus, adeno-associated virus, and herpes simplex virus-1, or recombinant bacterial or eukaryotic plasmids. Viral vectors transfect cells directly; plasmid DNA can be delivered with the help of, for example, cationic liposomes (lipofectin) or derivatized (e.g. antibody conjugated), polylysine conjugates, gramacidin S, artificial viral envelopes or other such intracellular carriers, as well as direct injection of the gene construct or CaPO₄ precipitation carried out *in vivo.* It will be appreciated that because transduction of appropriate target cells represents the critical first step in gene therapy, choice of the particular gene delivery system will depend on such factors as the phenotype of the intended target and the route of administration, e.g. locally or systemically. Furthermore, it will be recognized that the particular gene construct provided for *in vivo* transduction of *hedgehog* expression are also useful for *in vitro* transduction of cells, such as for use in the *ex vivo* tissue culture systems described below.

A preferred approach for *in vivo* introduction of nucleic acid into a cell is by use of a viral vector containing nucleic acid, e.g. a cDNA, encoding the particular form of the *hedgehog* polypeptide desired. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells which have taken up viral vector nucleic acid.

Retrovirus vectors and adeno-associated virus vectors are generally understood to be the recombinant gene delivery system of choice for the transfer of exogenous genes *in vivo,* particularly into humans. These vectors provide efficient delivery of genes into cells, and the transferred nucleic acids are stably integrated into the chromosomal DNA of the host. A major prerequisite for the use of retroviruses is to ensure the safety of their use, particularly with regard to the possibility of the spread of wild-type virus in the cell population. The development of specialized cell lines (termed "packaging cells") which produce only replication-defective retroviruses has increased the utility of retroviruses for gene therapy, and defective retroviruses are well characterized for use in gene transfer for gene therapy purposes (for a review see Miller, A.D. (1990) *Blood* 76:271). Thus, recombinant retrovirus can be constructed in which part of the retroviral coding sequence (*gag, pol, env*) has been replaced by nucleic acid encoding a *hedgehog* polypeptide and renders the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines for preparing both ecotropic and amphotropic retroviral systems include Crip, Cre, 2 and Am. Retroviruses have been used to introduce a variety of genes into many different cell types, including neuronal cells, *in vitro* and/or *in vivo* (see for example Eglitis, et al. (1985) *Science* 230:1395-1398; Danos and Mulligan (1988) *Proc. Natl. Acad Sci. USA* 85:6460-6464; Wilson et al. (1988) *Proc. Natl. Acad. Sci. USA* 85:3014-3018; Armentano et al. (1990) *Proc. Natl. Acad Sci. USA* 87:6141-6145; Huber et al. (1991) *Proc. Natl. Acad. Sci. USA* 88:8039-8043; Ferry et al. (1991) *Proc. Natl. Acad. Sci. USA* 88:8377-8381; Chowdhury et al. (1991) *Science* 254:1802-1805; van Beusechem et al. (1992) *Proc. Natl. Acad. Sci. USA* 89:7640-7644; Kay et al. (1992) *Human Gene Therapy* 3:641-647; Dai et al. (1992) *Proc. Natl. Acad. Sci. USA* 89:10892-10895; Hwu et al. (1993) *J. Immunol.* 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573).

Furthermore, it has been shown that it is possible to limit the infection spectrum of retroviruses and consequently of retroviral-based vectors, by modifying the viral packaging proteins on the surface of the viral particle (see, for example PCT publications WO93/25234 and WO94/06920). For instance, strategies for the modification of the infection spectrum of retroviral vectors include: coupling antibodies specific for cell surface antigens to the viral env protein (Roux et al. (1989) *PNAS* 86:9079-9083; Julan et al. (1992) *J Gen Virol* 73:3251-3255; and Goud et al. (1983) *Virology* 163:251-254); or coupling cell surface receptor ligands to the viral *env* proteins (Neda et al. (1991) *J Biol Chem* 266:14143-14146). Coupling can be in the form of the chemical cross-linking with a protein or other variety (e.g. lactose to convert the *env* protein to an asialoglycoprotein), as well as by generating fusion proteins (e.g. single-chain antibody/*env* fusion proteins). This technique, while useful to limit or otherwise direct the infection to certain tissue types, can also be used to convert an ecotropic vector in to an amphotropic vector.

Moreover, use of retroviral gene delivery can be further enhanced by the use of tissue- or cell-specific transcriptional regulatory sequences which control expression of the *hedgehog* gene of the retroviral vector.

Another viral gene delivery system useful in the present method utilizes adenovirus-derived vectors. The genome of an adenovirus can be manipulated such that it encodes and expresses a gene product of interest but is inactivated in terms of its ability to replicate in a normal lytic viral life cycle. See for example Berkner et al. (1988) *BioTechniques* 6:616; Rosenfeld et al. (1991) *Science* 252:431-434; and Rosenfeld et al. (1992) *Cell* 68:143-155. Suitable adenoviral vectors derived from the adenovirus strain Ad type 5 d1324 or other strains of adenovirus (e.g., Ad2, Ad3, Ad7 etc.) are well known to those skilled in the art. Recombinant adenoviruses can be advantageous in certain circumstances in that they can be used to infect a wide variety of cell types, including neuronal cells (Rosenfeld et al. (1992) cited *supra*).

Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al. cited *supra;* Haj-Ahmand and Graham (1986) *J. Virol*. 57:267). Most replication-defective adenoviral vectors currently in use and therefore favored by the present invention are deleted for all or parts of the viral E1 and E3 genes but retain as much as 80% of the adenoviral genetic material (see, e.g., Jones et al. (1979) *Cell* 16:683; Berkner et al., *supra;* and Graham et al. in Methods in Molecular Biology, E.J. Murray, Ed. (Humana, Clifton, NJ, 1991) vol. 7. pp. 109-127). Expression of the inserted *hedgehog* gene can be under control of, for example, the EIA promoter, the major late promoter (MLP) and associated leader sequences, the E3 promoter, or exogenously added promoter sequences.

In addition to viral transfer methods, such as those illustrated above, non-viral methods can also be employed to cause expression of a *hedgehog* polypeptide in the tissue of an animal. Most nonviral methods of gene transfer rely on normal mechanisms used by mammalian cells for the uptake and intracellular transport of macromolecules. In preferred embodiments, non-viral gene delivery systems of the present invention rely on endocytic pathways for the uptake of the *hedgehog* polypeptide gene by the targeted cell. Exemplary gene delivery systems of this type include liposomal derived systems, poly-lysine conjugates, and artificial viral envelopes.

In clinical settings, the gene delivery systems for the therapeutic *hedgehog* gene can be introduced into a patient by any of a number of methods, each of which is familiar in the art. For instance, a pharmaceutical preparation of the gene delivery system can be introduced systemically, e.g. by intravenous injection, and specific transduction of the protein in the target cells occurs predominantly from specificity of transfection provided by the gene delivery vehicle, cell-type or tissue-type expression due to the transcriptional regulatory sequences controlling expression of the receptor gene, or a combination thereof. In other embodiments, initial delivery of the recombinant gene is more limited with introduction into the animal being quite localized. For example, the gene delivery vehicle can be introduced by catheter (see U.S. Patent 5,328,470) or by stereotactic injection (e.g. Chen et al. (1994) *PNAS* 91: 3054-3057). A *hedgehog* expression construct can be delivered in a gene therapy construct to dermal cells by, e.g., electroporation using techniques described, for example, by Dev et al. ((1994) *Cancer Treat Rev* 20:105-115).

The pharmaceutical preparation of the gene therapy construct can consist, essentially of the gene delivery system in an acceptable diluent, or can comprise a slow release matrix in which the gene delivery vehicle is imbedded. Alternatively, where the complete gene delivery system can be produced intact from recombinant cells, e.g. retroviral vectors, the pharmaceutical preparation can comprise one or more cells which produce the gene delivery system.

In yet another embodiment, the *hedgehog* or *ptc* therapeutic can be a "gene activation" construct which, by homologous recombination with a genomic DNA, alters the transcriptional regulatory sequences of an endogenous gene. For instance, the gene activation construct can replace the endogenous promoter of a *hedgehog* gene with a heterologous promoter, e.g., one which causes consitutive expression of the *hedgehog* gene or which causes inducible expression of the gene under conditions different from the normal expression pattern of the gene. Other genes in the *patched* signaling pathway can be similarly targeted. A vareity of different formats for the gene activation constructs are available. See, for example, the Transkaryotic Therapies, Inc PCT publications WO93/09222, WO95/31560, WO96/29411, WO95/31560 and WO94/12650.

In preferred embodiments, the nucleotide sequence used as the gene activation construct can be comprised of (1) DNA from some portion of the endogenous *hedgehog* gene (exon sequence, intron sequence, promoter sequences, etc.) which direct recombination and (2) heterologous transcriptional regulatory sequence(s) which is to be operably linked to the coding sequence for the genomic *hedgehog* gene upon recombination of the gene activation construct. For use in generating cultures of *hedgehog* producing cells, the construct may further include a reporter gene to detect the presence of the knockout construct in the cell.

The gene activation construct is inserted into a cell, and integrates with the genomic DNA of the cell in such a position so as to provide the heterologous regulatory sequences in operative association with the native *hedgehog* gene. Such insertion occurs by homologous recombination, i.e., recombination regions of the activation construct that are homologous to the endogenous *hedgehog* gene sequence hybridize to the genomic DNA and recombine with the genomic sequences so that the construct is incorporated into the corresponding position of the genomic DNA.

The terms "recombination region" or "targeting sequence" refer to a segment (i.e., a portion) of a gene activation construct having a sequence that is substantially identical to or substantially complementary to a genomic gene sequence, e.g., including 5' flanking sequences of the genomic gene, and can facilitate homologous recombination between the genomic sequence and the targeting transgene construct.

As used herein, the term "replacement region" refers to a portion of a activation construct which becomes integrated into an endogenous chromosomal location following homologous recombination between a recombination region and a genomic sequence.

The heterologous regulatory sequences, e.g., which are provided in the replacement region, can include one or more of a variety elements, including: promoters (such as constitutive or inducible promoters), enhancers, negative regualtory elements, locus control regions, transcription factor binding sites, or combinations thereof. Promoters/enhancers which may be used to control the expression of the targeted gene *in vivo* include, but are not limited to, the cytomegalovirus (CMV) promoter/enhancer (Karasuyama et al., 1989, *J Exp. Med.,* 169:13), the human β-actin promoter (Gunning et al. (1987) *PNAS* 84:4831-4835), the glucocorticoid-inducible promoter present in the mouse mammary tumor virus long terminal repeat (MMTV LTR) (Klessig et al. (1984) *Mol. Cell Biol.* 4:1354-1362), the long terminal repeat sequences of Moloney murine leukemia virus (MuLV LTR) (Weiss et al. (1985) *RNA Tumor Viruses,* Cold Spring Harbor Laboratory, Cold Spring Harbor, New York), the SV40 early or late region promoter (Bemoist et al. (1981) *Nature* 290:304-310; Templeton et al. (1984) *Mol. Cell Biol*., 4:817; and Sprague et al. (1983) *J. Virol*., 45:773), the promoter contained in the 3' long terminal repeat of Rous sarcoma virus (RSV) (Yamamoto et al., 1980, *Cell*, 22:787-797), the herpes simplex virus (HSV) thymidine kinase promoter/enhancer (Wagner et al. (1981) *PNAS* 82:3567-71), and the herpes simplex virus LAT promoter (Wolfe et al. (1992) *Nature Genetics,* 1:379-384)*.*

In an exemplary embodiment, portions of the 5' flanking region of the human Shh gene are amplified using primers which add restriction sites, to generate the following fragments As illustrated, primer 1 includes a 5' non-coding region of the human Shh gene and is flanked by an AsuII and ClaI restriction sites. Primer 2 includes a portion of the 5' non-coding region immediately 3' to that present in primer 1. The *hedgehog* gene sequence is flanked by XhoII and BamHI restriction sites. The purified amplimers are cut with each of the enzymes as appropriate.

The vector pCDNA1.1 (Invitrogen) includes a CMV promoter. The plasmid is cut with with AsuII, which cleaves just 3' to the CMV promoter sequence. The AsuII/ClaI fragment of primer 1 is ligated to the AsuII cleavage site of the pcDNA vector. The ClaI/ AsuII ligation destroys the AsuII site at the 3' end of a properly inserted primer 1.

The vector is then cut with BamHI, and an XhoII/BamHI fragment of primer 2 is ligated to the BamHI cleavage site. As above, the BamHI/XhoII ligation destroys the BamHI site at the 5' end of a properly inserted primer 2.

Individual colonies are selected, cut with AsuII and BamHI, and the size of the AsuII/BamHI fragment determined. Colonies in which both the primer 1 and primer 2 sequences are correctly inserted are further amplified, an cut with AsuII and BamHI to produce the gene activation construct In this construct, the flanking primer 1 and primer 2 sequences provide the recombination region which permits the insertion of the CMV promoter in front of the coding sequence for the human *Shh* gene. Other heterologous promoters (or other transcriptional regulatory sequences) can be inserted in a genomic *hedgehog* gene by a similar method.

In still other embodiments, the replacement region merely deletes a negative transcriptional control element of the native gene, e.g., to activate expression, or ablates a positive control element, e.g., to inhibit expression of the targeted gene.

### V. Exemplary ptc therapeutic compounds.

In another embodiment, the subject method is carried out using a *ptc* therapeutic composition. Such compositions can be generated with, for example, compounds which bind to patched and alter its signal transduction activity, compounds which alter the binding and/or enzymatic activity of a protein (e.g., intracellular) involved in patched signal pathway, and compounds which alter the level of expression of a *hedgehog* polypeptide, a patched protein or a protein involved in the intracellular signal transduction pathway of patched.

The availability of purified and recombinant *hedgehog* polypeptides facilitates the generation of assay systems which can be used to screen for drugs, such as small organic molecules, which are either agonists or antagonists of the normal cellular function of a *hedgehog* and/or patched protein, particularly in their role in the pathogenesis of neuronal cell death. In one embodiment, the assay evaluates the ability of a compound to modulate binding between a *hedgehog* polypeptide and a *hedgehog* receptor such as *patched.* In other embodiments, the assay merely scores for the ability of a test compound to alter the signal transduction activity of the *patched* protein. In this manner, a variety of *hedgehog* and/or *ptc* therapeutics, which will include ones with neuroprotective activity, can be identified. A variety of assay formats will suffice and, in light of the present disclosure, will be comprehended by skilled artisan.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with receptor proteins.

Accordingly, in an exemplary screening assay for *ptc* therapeutics, the compound of interest is contacted with a mixture including a *hedgehog* receptor protein (e.g., a cell expressing the *patched* receptor) and a *hedgehog* polypeptide under conditions in which it is ordinarily capable of binding the *hedgehog* polypeptide. To the mixture is then added a composition containing a test compound. Detection and quantification of receptor/*hedgehog* complexes provides a means for determining the test compound's efficacy at inhibiting (or potentiating) complex formation between the receptor protein and the *hedgehog* polypeptide. Moreover, a control assay can also be performed to provide a baseline for comparison. In the control assay, isolated and purified *hedgehog* polypeptide is added to the receptor protein, and the formation of receptor/*hedgehog* complex is quantitated in the absence of the test compound.

In other embodiments, a *ptc* therapeutic of the present invention is one which disrupts the association of *patched* with *smoothened.*

Agonist and antagonists of neuroprotection can be distinguished, and the efficacy of the compound can be assessed, by subsequent testing with neuronal cells.

In an illustrative embodiment, the polypeptide utilized as a *hedgehog* receptor can be generated from the *patched* protein. Accordingly, an exemplary screening assay includes all or a suitable portion of the *patched* protein which can be obtained from, for example, the human *patched* gene (GenBank U43148) or other vertebrate sources (see GenBank Accession numbers U40074 for chicken *patched* and U46155 for mouse *patched*), as well as from drosophila (GenBank Accession number M28999) or other invertebrate sources. The *patched* protein can be provided in the screening assay as a whole protein (preferably expressed on the surface of a cell), or alternatively as a fragment of the full length protein which binds to *hedgehog* polypeptides, e.g., as one or both of the substantial extracellular domains (e.g. corresponding to residues Asn120-Ser438 and/or Arg770-Trp1027 of the human *patched* protein). For instance, the *patched* protein can be provided in soluble form, as for example a preparation of one of the extracellular domains, or a preparation of both of the extracellular domains which are covalently connected by an unstructured linker (see, for example, Huston et al. (1988) PNAS 85:4879; and U.S. Patent No. 5,091,513). In other embodiments, the protein can be provided as part of a liposomal preparation or expressed on the surface of a cell. The *patched* protein can derived from a recombinant gene, e.g., being ectopically expressed in a heterologous cell. For instance, the protein can be expressed on oocytes, mammalian cells (e.g., COS, CHO, 3T3 or the like), or yeast cell by standard recombinant DNA techniques. These recombinant cells can be used for receptor binding, signal transduction or gene expression assays. Marigo et al. (1996) *Development* 122:1225-1233 illustrates a binding assay of human *hedgehog* to chick *patched* protein ectopically expressed in *Xenopus laevis* oocytes. The assay system of Marigo et al. can be adapted to the present drug screening assays. As illustrated in that reference, *Shh* binds to the *patched* protein in a selective, saturable, dose-dependent manner, thus demonstrating that *patched* is a receptor for *Shh.*

Complex formation between the *hedgehog* polypeptide and a *hedgehog* receptor may be detected by a variety of techniques. For instance, modulation of the formation of complexes can be quantitated using, for example, detectably labelled proteins such as radiolabelled, fluorescently labelled, or enzymatically labelled *hedgehog* polypeptides, by immunoassay, or by chromatographic detection.

Typically, for cell-free assays, it will be desirable to immobilize either the *hedgehog* receptor or the *hedgehog* polypeptide to facilitate separation of receptor/*hedgehog* complexes from uncomplexed forms of one of the proteins, as well as to accommodate automation of the assay. In one embodiment, a fusion protein can be provided which adds a domain that allows the protein to be bound to a matrix. For example, glutathione-S-transferase/receptor (GST/receptor) fusion proteins can be adsorbed onto glutathione sepharose beads (Sigma Chemical, St. Louis, MO) or glutathione derivatized microtitre plates, which are then combined with the *hedgehog* polypeptide, e.g. an ³⁵S-labeled *hedgehog* polypeptide, and the test compound and incubated under conditions conducive to complex formation, e.g. at physiological conditions for salt and pH, though slightly more stringent conditions may be desired. Following incubation, the beads are washed to remove any unbound *hedgehog* polypeptide, and the matrix bead-bound radiolabel determined directly (e.g. beads placed in scintillant), or in the supernatant after the receptor/*hedgehog* complexes are dissociated. Alternatively, the complexes can be dissociated from the bead, separated by SDS-PAGE gel, and the level of *hedgehog* polypeptide found in the bead fraction quantitated from the gel using standard electrophoretic techniques.

Other techniques for immobilizing proteins on matrices are also available for use in the subject assay. For instance, soluble portions of the *hedgehog* receptor protein can be immobilized utilizing conjugation of biotin and streptavidin. For instance, biotinylated receptor molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with the *hedgehog* receptor but which do not interfere with *hedgehog* binding can be derivatized to the wells of the plate, and the receptor trapped in the wells by antibody conjugation. As above, preparations of a *hedgehog* polypeptide and a test compound are incubated in the receptor-presenting wells of the plate, and the amount of receptor/*hedgehog* complex trapped in the well can be quantitated. Exemplary methods for detecting such complexes, in addition to those described above for the GST-immobilized complexes, include immunodetection of complexes using antibodies reactive with the *hedgehog* polypeptide, or which are reactive with the receptor protein and compete for binding with the *hedgehog* polypeptide; as well as enzyme-linked assays which rely on detecting an enzymatic activity associated with the *hedgehog* polypeptide. In the instance of the latter, the enzyme can be chemically conjugated or provided as a fusion protein with the *hedgehog* polypeptide. To illustrate, the *hedgehog* polypeptide can be chemically crosslinked or genetically fused with alkaline phosphatase, and the amount of *hedgehog* polypeptide trapped in the complex can be assessed with a chromogenic substrate of the enzyme, e.g. paranitrophenylphosphate. Likewise, a fusion protein comprising the *hedgehog* polypeptide and glutathione-S-transferase can be provided, and complex formation quantitated by detecting the GST activity using 1-chloro-2,4-dinitrobenzene (Habig et al (1974) *J Biol Chem* 249:7130).

For processes which rely on immunodetection for quantitating one of the proteins trapped in the complex, antibodies against the protein, such as the anti-*hedgehog* antibodies described herein, can be used. Alternatively, the protein to be detected in the complex can be "epitope tagged" in the form of a fusion protein which includes, in addition to the *hedgehog* polypeptide or *hedgehog* receptor sequence, a second polypeptide for which antibodies are readily available (e.g. from commercial sources). For instance, the GST fusion proteins described above can also be used for quantification of binding using antibodies against the GST moiety. Other useful epitope tags include myc-epitopes (e.g., see Ellison et al. (1991) *J Biol Chem* 266:21150-21157) which includes a 10-residue sequence from c-myc, as well as the pFLAG system (International Biotechnologies, Inc.) or the pEZZ-protein A system (Pharamacia, NJ).

Where the desired portion of the *hedgehog* receptor (or other *hedgehog* binding molecule) cannot be provided in soluble form, liposomal vesicles can be used to provide manipulatable and isolatable sources of the receptor. For example, both authentic and recombinant forms of the *patched* protein can be reconstituted in artificial lipid vesicles (e.g. phosphatidylcholine liposomes) or in cell membrane-derived vesicles (see, for example, Bear et al. (1992) *Cell* 68:809-818; Newton et al. (1983) *Biochemistry* 22:6110-6117; and Reber et al. (1987) *J Biol Chem* 262:11369-11374).

In addition to cell-free assays, such as described above, the readily available source of *hedgehog* polypeptides provided by the art also facilitates the generation of cell-based assays for identifying small molecule agonists of the neuroprotective activity of wild-type *hedgehog* polypeptides. Analogous to the cell-based assays described above for screening combinatorial libraries, neuronal cells which are sensitive to *hedgehog*-dependent protection, such as dopaminergic and GABAergic neurons, can be contacted with a *hedgehog* polypeptide and a test agent of interest, with the assay scoring for anything from simple binding to the cell to trophic responses by the target cell in the presence and absence of the test agent. As with the cell-free assays, agents which produce a statistically significant change in *hedgehog* activities (either inhibition or potentiation) can be identified.

In other emdodiments, the cell-based assay scores for agents which disrupt association of patched and *smoothened* proteins, e.g., in the cell surface membrane or liposomal preparation.

In addition to characterizing cells that naturally express the *patched* protein, cells which have been genetically engineered to ectopically express *patched* can be utilized for drug screening assays. As an example, cells which either express low levels or lack expression of the *patched* protein, e.g. *Xenopus laevis* oocytes, COS cells or yeast cells, can be genetically modified using standard techniques to ectopically express the *patched* protein. (see Marigo et al., *supra*).

The resulting recombinant cells, e.g., which express a functional *patched* receptor, can be utilized in receptor binding assays to identify agonist or anatagonsts of *hedgehog* binding. Binding assays can be performed using whole cells. Furthermore, the recombinant cells of the present invention can be engineered to include other heterolgous genes encoding proteins involved in *hedgehog*-dependent siganl pathways. For example, the gene products of one or more of *smoothened, costal-2* and/or *fused* can be co-expressed with *patched* in the reagent cell, with assays being sensitive to the functional reconstituion of the *hedgehog* signal transduction cascade.

Alternatively, liposomal preparations using reconstituted *patched* protein can be utilized. *Patched* protein purified from detergent extracts from both authentic and recombinant origins can be reconstituted in in artificial lipid vesicles (e.g. phosphatidylcholine liposomes) or in cell membrane-derived vesicles (see, for example, Bear et al. (1992) *Cell* 68:809-818; Newton et al. (1983) *Biochemistry* 22:6110-6117; and Reber et al. (1987) *J Biol Chem* 262:11369-11374). The lamellar structure and size of the resulting liposomes can be characterized using electron microscopy. External orientation of the *patched* protein in the reconstituted membranes can be demonstrated, for example, by immunoelectron microscopy. The *hedgehog* polypeptide binding activity of liposomes containing *patched* and liposomes without the protein in the presence of candidate agents can be compared in order to identify potential modulators of the *hedgehog-patched* interaction.

The *hedgehog* polypeptide used in these cell-based assays can be provided as a purified source (natural or recombinant in origin), or in the form of cells/tissue which express the protein and which are co-cultured with the target cells. As in the cell-free assays, where simple binding (rather than induction) is the *hedgehog* activity scored for in the assay, the protein can be labelled by any of the above-mentioned techniques, e.g., fluorescently, enzymatically or radioactively, or detected by immunoassay.

In addition to binding studies, functional assays can be used to identified modulators, i.e., agonists of *hedgehog* or *patched* activities. By detecting changes in intracellular signals, such as alterations in second messengers or gene expression in *patched*-expressing cells contacted with a test agent, candidate antagonists to *patched* signaling can be identified (e.g., having a *hedgehog*-like activity).

A number of gene products have been implicated in *patched*-mediated signal transduction, including *patched*, the transcription factor *cubitus interruptus* (ci), the serine/threonine kinase *fused* (fu) and the gene products of *costal-2, smoothened* and *suppressor of fused.*

The interaction of a *hedgehog* polypeptide with *patched* sets in motion a cascade involving the activation and inhibition of downstream effectors, the ultimate consequence of which is, in some instances, a detectable change in the transcription or translation of a gene. Potential transcriptional targets of *patched* signaling are the *patched* gene itself (Hidalgo and Ingham, 1990 *Development* 110, 291-301; Marigo et al., 1996) and the vertebrate homologs of the drosophila cubitus interruptus gene, the *GLI* genes (Hui et al. (1994) *Dev Biol* 162:402-413). *Patched* gene expression has been shown to be induced in cells of the limb bud and the neural plate that are responsive to *Shh.* (Marigo et al. (1996) *PNAS*, in press; Marigo et al. (1996) *Development* 122:1225-1233). The *GLI* genes encode putative transcription factors having zinc finger DNA binding domains (Orenic et al. (1990) *Genes & Dev* 4:1053-1067; Kinzler et al. (1990) *Mol Cell Biol* 10:634-642). Transcription of the *GLI* gene has been reported to be upregulated in response to *hedgehog* in limb buds, while transcription of the *GLI3* gene is downregulated in response to *hedgehog* induction (Marigo et al. (1996) *Development* 122:1225-1233). By selecting transcriptional regulatory sequences from such target genes, e.g. from *patched* or *GLI* genes, that are responsible for the up- or down regulation of these genes in response to *patched* signalling, and operatively linking such promoters to a reporter gene, one can derive a transcription based assay which is sensitive to the ability of a specific test compound to modify *patched* signalling pathways. Expression of the reporter gene, thus, provides a valuable screening tool for the development of compounds that act as antagonists of *ptc,* e.g., which may be useful as neuroprotective agents.

Reporter gene based assays of this invention measure the end stage of the above described cascade of events, e.g., transcriptional modulation. Accordingly, in practicing one embodiment of the assay, a reporter gene construct is inserted into the reagent cell in order to generate a detection signal dependent on *ptc* signaling. To identify potential regulatory elements responsive to *ptc* signaling present in the transcriptional regulatory sequence of a target gene, nested deletions of genomic clones of the target gene can be constructed using standard techniques. See, for example, Current Protocols in Molecular Biology, Ausubel, F.M. et al. (eds.) Greene Publishing Associates, (1989); U.S. Patent 5,266,488; Sato et al. (1995) *J Biol Chem* 270:10314-10322; and Kube et al. (1995) *Cytokine* 7:1-7. A nested set of DNA fragments from the gene's 5'-flanking region are placed upstream of a reporter gene, such as the luciferase gene, and assayed for their ability to direct reporter gene expression in *patched* expressing cells. Host cells transiently transfected with reporter gene constructs can be scored for the induction of expression of the reporter gene in the presence and absence of *hedgehog* to determine regulatory sequences which are responsice to *patched*-dependent signalling.

In practicing one embodiment of the assay, a reporter gene construct is inserted into the reagent cell in order to generate a detection signal dependent on second messengers generated by induction with *hedgehog* polypeptide. Typically, the reporter gene construct will include a reporter gene in operative linkage with one or more transcriptional regulatory elements responsive to the *hedgehog* activity, with the level of expression of the reporter gene providing the *hedgehog*-dependent detection signal. The amount of transcription from the reporter gene may be measured using any method known to those of skill in the art to be suitable. For example, mRNA expression from the reporter gene may be detected using RNAse protection or RNA-based PCR, or the protein product of the reporter gene may be identified by a characteristic stain or an intrinsic activity. The amount of expression from the reporter gene is then compared to the amount of expression in either the same cell in the absence of the test compound (or *hedgehog*) or it may be compared with the amount of transcription in a substantially identical cell that lacks the target receptor protein. Any statistically or otherwise significant difference in the amount of transcription indicates that the test compound has in some manner altered the signal transduction of the *patched* protein, e.g., the test compound is a potential *ptc* therapeutic.

As described in further detail below, in preferred embodiments the gene product of the reporter is detected by an intrinsic activity associated with that product. For instance, the reporter gene may encode a gene product that, by enzymatic activity, gives rise to a detection signal based on color, fluorescence, or luminescence. In other preferred embodiments, the reporter or marker gene provides a selective growth advantage, e.g., the reporter gene may enhance cell viability, relieve a cell nutritional requirement, and/or provide resistance to a drug.

Preferred reporter genes are those that are readily detectable. The reporter gene may also be included in the construct in the form of a fusion gene with a gene that includes desired transcriptional regulatory sequences or exhibits other desirable properties. Examples of reporter genes include, but are not limited to CAT (chloramphenicol acetyl transferase) (Alton and Vapnek (1979), Nature 282: 864-869) luciferase, and other enzyme detection systems, such as beta-galactosidase; firefly luciferase (deWet et al. (1987), Mol. Cell. Biol. 7:725-737); bacterial luciferase (Engebrecht and Silverman (1984), PNAS 1: 4154-4158; Baldwin et al. (1984), Biochemistry 23: 3663-3667); alkaline phosphatase (Toh et al. (1989) Eur. J. Biochem. 182: 231-238, Hall et al. (1983) J. Mol. Appl. Gen. 2: 101), human placental secreted alkaline phosphatase (Cullen and Malim (1992) Methods in Enzymol. 216:362-368).

Transcriptional control elements which may be included in a reporter gene construct include, but are not limited to, promoters, enhancers, and repressor and activator binding sites. Suitable transcriptional regulatory elements may be derived from the transcriptional regulatory regions of genes whose expression is induced after modulation of a *patched* signal transduction pathway. The characteristics of preferred genes from which the transcriptional control elements are derived include, but are not limited to, low or undetectable expression in quiescent cells, rapid induction at the transcriptional level within minutes of extracellular simulation, induction that is transient and independent of new protein synthesis, subsequent shut-off of transcription requires new protein synthesis, and mRNAs transcribed from these genes have a short half-life. It is not necessary for all of these properties to be present.

In yet other embodiments, second messenger generation can be measured directly in the detection step, such as mobilization of intracellular calcium, phospholipid metabolism or adenylate cyclase activity are quantitated, for instance, the products of phospholipid hydrolysis IP₃, DAG or cAMP could be measured For example, recent studies have implicated protein kinase A (PKA) as a possible component of *hedgehog*/*patched* signaling (Hammerschmidt et al. (1996) *Genes & Dev* 10:647). High PKA activity has been shown to antagonize *hedgehog* signaling in these systems. Conversely, inhibitors of PKA will mimic and/or potentiate the action of *hedgehog.* Although it is unclear whether PKA acts directly downstream or in parallel with *hedgehog* signaling, it is possible that *hedgehog* signalling occurs via inhibition of PKA activity. Thus, detection of PKA activity provides a potential readout for the instant assays.

In a preferred embodiment, the *ptc* therapeutic is a PKA inhibitor. A variety of PKA inhibitors are known in the art, including both peptidyl and organic compounds. For instance, the *ptc* therapeutic can be a 5-isoquinolinesulfonamide, such as represented in the general formula: 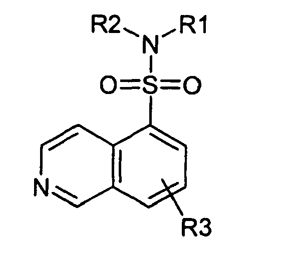 wherein,
R₁ and R₂ each can independently represent hydrogen, and as valence and stability permit a lower alkyl, a lower alkenyl, a lower alkynyl, a carbonyl (such as a carboxyl, an ester, a formate, or a ketone), a thiocarbonyl (such as a thioester, a thioacetate, or a thioformate), an amino, an acylamino, an amido, a cyano, a nitro, an azido, a sulfate, a sulfonate, a sulfonamido, -(CH₂)ₘ-R₈, -(CH₂)ₘ-OH, -(CH₂)ₘ-O-lower alkyl, -(CH₂)ₘ-O-lower alkenyl, -(CH₂)ₙ-O-(CH₂)ₘ-R₈, -(CH₂)ₘ-SH, -(CH₂)ₘ-S-lower alkyl, -(CH₂)ₘ-Slower alkenyl, -(CH₂)ₙ-S-(CH₂)ₘ-R₈, or
R₁ and R₂ taken together with N form a heterocycle (substituted or unsubstituted);
R₃ is absent or represents one or more substitutions to the isoquinoline ring such as a lower alkyl, a lower alkenyl, a lower alkynyl, a carbonyl (such as a carboxyl, an ester, a formate, or a ketone), a thiocarbonyl (such as a thioester, a thioacetate, or a thioformate), an amino, an acylamino, an amido, a cyano, a nitro, an azido, a sulfate, a sulfonate, a sulfonamido, -(CH₂)ₘ-R₈, -(CH₂)ₘ-OH, -(CH₂)ₘ-O-lower alkyl, -(CH₂)ₘ-O-lower alkenyl, - (CH₂)ₙ-O-(CH₂)ₘ-R₈, -(CH₂)ₘ-SH, -(CH₂)ₘ-S-lower alkyl, -(CH₂)ₘ-S-lower alkenyl, -(CH₂)ₙ-S-(CH₂)ₘ-R₈;
R₈ represents a substituted or unsubstituted aryl, aralkyl, cycloalkyl, cycloalkenyl, or heterocycle; and
n and m are independently for each occurrence zero or an integer in the range of 1 to 6.
In a preferred embodiment, the PKA inhibitor is N-[2-((p-bromocinnamyl)amino)ethyl]-5-isoquinolinesulfonamide (H-89; Calbiochem Cat. No. 371963), e.g., having the formula: 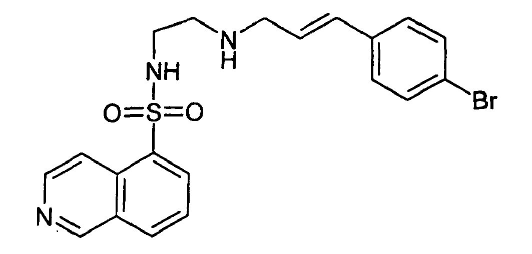 In another embodiment, the PKA inhibitor is 1-(5-isoquinolinesulfonyl)-2-methylpiperazine (H-7; Calbiochem Cat. No. 371955), e.g., having the formula: 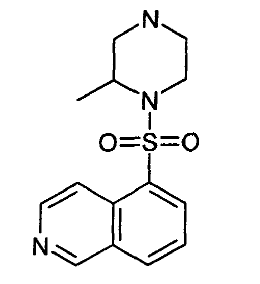 In still other embodiments, the PKA inhibitor is KT5720 (Calbiochem Cat. No. 420315), having the structure 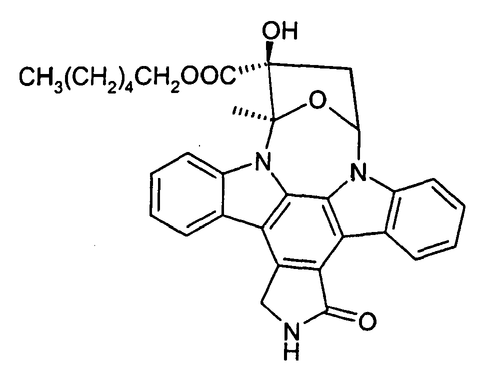 A variety of nucleoside analogs are also useful as PKA inhibitors. For example, the subject method can be carried out cyclic AMP analogs which inhibit the kinase activity of PKA, as for example, 8-bromo-cAMP or dibutyryl-cAMP

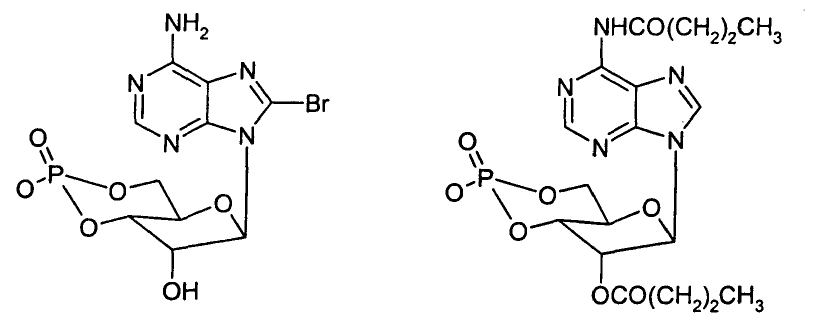

Exemplary peptidyl inhibitors of PKA activity include the PKA Heat Stable Inhibitor (isoform α; see, for example, Calbiochem Cat. No. 539488, and Wen et al. (1995) *J Biol Chem* 270:2041).

Certain *hedgehog* receptors may stimulate the activity of phospholipases. Inositol lipids can be extracted and analyzed using standard lipid extraction techniques. Water soluble derivatives of all three inositol lipids (IP₁, IP₂, IP₃) can also be quantitated using radiolabelling techniques or HPLC.

The mobilization of intracellular calcium or the influx of calcium from outside the cell may be a response to *hedgehog* stimulation or lack there of. Calcium flux in the reagent cell can be measured using standard techniques. The choice of the appropriate calcium indicator, fluorescent, bioluminescent, metallochromic, or Ca⁺⁺-sensitive microelectrodes depends on the cell type and the magnitude and time constant of the event under study (Borle (1990) *Environ Health Perspect* 84:45-56). As an exemplary method of Ca⁺⁺ detection, cells could be loaded with the Ca⁺⁺sensitive fluorescent dye fura-2 or indo-1, using standard methods, and any change in Ca⁺⁺ measured using a fluorometer.

In certain embodiments of the assay, it may be desirable to screen for changes in cellular phosphorylation. As an example, the drosophila gene *fused* (fu) which encodes a serine/threonine kinase has been identified as a potential downstream target in *hedgehog* signaling. (Preat et al., 1990 *Nature* 347, 87-89; Therond et al. 1993, *Mech. Dev.* 44. 65-80). The ability of compounds to modulate serine/threonine kinase activation could be screened using colony immunoblotting (Lyons and Nelson (1984) *Proc. Natl. Acad. Sci. USA* 81:7426-7430) using antibodies against phosphorylated serine or threonine residues. Reagents for performing such assays are commercially available, for example, phosphoserine and phosphothreonine specific antibodies which measure increases in phosphorylation of those residues can be purchased from comercial sources.

In yet another embodiment, the *ptc* therapeutic is an antisense molecule which inhibits expression of a protein involved in a *patched*-mediated signal transduction pathway. To illustrate, by inhibiting the expression of a protein involved in *patched* signals, such as *fused, costal-2, smoothened* and/or *Gli* genes, or *patched* itself, the ability of the patched signal pathway(s) to alter the ability of, e.g., a dopaminergic or GABAergic cell to maintain its differentiated state can be altered, e.g., potentiated or repressed.

As used herein, "antisense" therapy refers to administration or *in situ* generation of oligonucleotide probes or their derivatives which specifically hybridize (e.g. bind) under cellular conditions with cellular mRNA and/or genomic DNA encoding a *hedgehog* polypeptide, patched, or a protein involved in patched-mediated signal transduction. The hybridization should inhibit expression of that protein, e.g. by inhibiting transcription and/or translation. The binding may be by conventional base pair complementarity, or, for example, in the case of binding to DNA duplexes, through specific interactions in the major groove of the double helix. In general, "antisense" therapy refers to the range of techniques generally employed in the art, and includes any therapy which relies on specific binding to oligonucleotide sequences.

An antisense construct of the present invention can be delivered, for example, as an expression plasmid which, when transcribed in the cell, produces RNA which is complementary to at least a unique portion of the target cellular mRNA. Alternatively, the antisense construct is an oligonucleotide probe which is generated *ex vivo* and which, when introduced into the cell causes inhibition of expression by hybridizing with the mRNA and/or genomic sequences of a target gene. Such oligonucleotide probes are preferably modified oligonucleotide which are resistant to endogenous nucleases, e.g. exonucleases and/or endonucleases, and is therefore stable *in vivo.* Exemplary nucleic acid molecules for use as antisense oligonucleotides are phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) *Biotechniques* 6:958-976; and Stein et al. (1988) *Cancer Res* 48:2659-2668.

Several considerations should be taken into account when constructing antisense oligonucleotides for the use in the methods of the invention: (1) oligos should have a GC content of 50% or more; (2) avoid sequences with stretches of 3 or more G's; and (3) oligonucleotides should not be longer than 25-26 mers. When testing an antisense oligonucleotide, a mismatched control can be constructed. The controls can be generated by reversing the sequence order of the corresponding antisense oligonucleotide in order to conserve the same ratio of bases.

In an illustrative embodiment, the *ptc* therapeutic can be an antisense construct for inhibiting the expression of *patched,* e.g., to mimic the inhibition of *patched* by *hedgehog.* Exemplary antisense constructs include:

### VI. Exemplary pharmaceutical preparations of hedgehog and ptc therapeutics

The source of the *hedgehog* and *ptc* therapeutics to be formulated will depend on the particular form of the agent. Small organic molecules and peptidyl fragments can be chemically synthesized and provided in a pure form suitable for pharmaceutical/cosmetic usage. Products of natural extracts can be purified according to techniques known in the art. For example, the Cox et al. U.S. Patent 5,286,654 describes a method for purifying naturally occurring forms of a secreted protein and can be adapted for purification of *hedgehog* polypeptides. Recombinant sources of *hedgehog* polypeptides are also available. For example, the gene encoding *hedgehog* polypeptides, are known, *inter alia,* from PCT publications WO 95/18856 and WO 96/17924.

Those of skill in treating neural tissues can determine the effective amount of an *hedgehog* or *ptc* therapeutic to be formulated in a pharmaceutical or cosmetic preparation.

The *hedgehog or ptc* therapeutic formulations used in the method of the invention are most preferably applied in the form of appropriate compositions. As appropriate compositions there may be cited all compositions usually employed for systemically or locally (such as intrathecal) administering drugs. The pharmaceutically acceptable carrier should be substantially inert, so as not to act with the active component. Suitable inert carriers include water, alcohol polyethylene glycol, mineral oil or petroleum gel, propylene glycol and the like.

To prepare the pharmaceutical compositions of this invention, an effective amount of the particular *hedgehog* or *ptc* therapeutic as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules, and tablets. Because of their ease in administration, tablets and capsules represents the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositons suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

It is especially advantageous to formulate the subject compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powders packets, wafers, injectable solutions or suspensions, teaspoonfuls, tablespoonfuls and the like, and segregated multiples thereof.

The pharmaceutical preparations of the present invention can be used, as stated above, for the many applications which can be considered cosmetic uses. Cosmetic compositions known in the art, preferably hypoallergic and pH controlled are especially preferred, and include toilet waters, packs, lotions, skin milks or milky lotions. The preparations contain, besides the *hedgehog or ptc* therapeutic, components usually employed in such preparations. Examples of such components are oils, fats, waxes, surfactants, humectants, thickening agents, antioxidants, viscosity stabilizers, chelating agents, buffers, preservatives, perfumes, dyestuffs, lower alkanols, and the like. If desired, further ingredients may be incorporated in the compositions, e.g. antiinflammatory agents, antibacterials, antifungals, disinfectants, vitamins, sunscreens, antibiotics, or other anti-acne agents.

Examples of oils comprise fats and oils such as olive oil and hydrogenated oils; waxes such as beeswax and lanolin; hydrocarbons such as liquid paraffin, ceresin, and squalane; fatty acids such as stearic acid and oleic acid; alcohols such as cetyl alcohol, stearyl alcohol, lanolin alcohol, and hexadecanol; and esters such as isopropyl myristate, isopropyl palmitate and butyl stearate. As examples of surfactants there may be cited anionic surfactants such as sodium stearate, sodium cetylsulfate, polyoxyethylene laurylether phosphate, sodium N-acyl glutamate; cationic surfactants such as stearyldimethylbenzylammonium chloride and stearyltrimethylammonium chloride; ampholytic surfactants such as alkylaminoethylglycine hydrocloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, polyoxypropylene glycol (e.g. the materials sold under the trademark "Pluronic"), polyoxyethylene castor oil, and polyoxyethylene lanolin. Examples of humectants include glycerin, 1,3-butylene glycol, and propylene glycol; examples of lower alcohols include ethanol and isopropanol; examples of thickening agents include xanthan gum, hydroxypropyl cellulose, hydroxypropyl methyl cellulose, polyethylene glycol and sodium carboxymethyl cellulose; examples of antioxidants comprise butylated hydroxytoluene, butylated hydroxyanisole, propyl gallate, citric acid and ethoxyquin; examples of chelating agents include disodium edetate and ethanehydroxy diphosphate; examples of buffers comprise citric acid, sodium citrate, boric acid, borax, and disodium hydrogen phosphate; and examples of preservatives are methyl parahydroxybenzoate, ethyl parahydroxybenzoate, dehydroacetic acid, salicylic acid and benzoic acid.

For preparing ointments, creams, toilet waters, skin milks, and the like, typically from 0.01 to 10% in particular from 0.1 to 5% and more in particular from 0.2 to 2.5% of the active ingredient, e.g., of the *hedgehog* or *ptc* therapeutic, will be incorporated in the compositions. In ointments or creams, the carrier for example consists of 1 to 20%, in particular 5 to 15% of a humectant, 0.1 to 10% in particular from 0.5 to 5% of a thickener and water; or said carrier may consist of 70 to 99%, in particular 20 to 95% of a surfactant, and 0 to 20%, in particular 2.5 to 15% of a fat; or 80 to 99.9% in particular 90 to 99% of a thickener; or 5 to 15% of a surfactant, 2-15% of a humectant, 0 to 80% of an oil, very small (< 2%) amounts of preservative, coloring agent and/or perfume, and water. In a toilet water, the carrier for example consists of 2 to 10% of a lower alcohol, 0.1 to 10% or in particular 0.5 to 1% of a surfactant, 1 to 20%, in particular 3 to 7% of a humectant, 0 to 5% of a buffer, water and small amounts (< 2%) of preservative, dyestuff and/or perfume. In a skin milk, the carrier typically consists of 10-50% of oil, 1 to 10% of surfactant, 50-80% of water and 0 to 3% of preservative and/or perfume. In the aforementioned preparations, all % symbols refer to weight by weight percentage.

Particular compositions for use in the method of the present invention are those wherein the *hedgehog* or *ptc* therapeutic is formulated in liposome-containing compositions. Liposomes are artificial vesicles formed by amphiphatic molecules such as polar lipids, for example, phosphatidyl cholines, ethanolamines and serines, sphingomyelins, cardiolipins, plasmalogens, phosphatidic acids and cerebiosides. Liposomes are formed when suitable amphiphathic molecules are allowed to swell in water or aqueous solutions to form liquid crystals usually of multilayer structure comprised of many bilayers separated from each other by aqueous material (also referred to as coarse liposomes). Another type of liposome known to be consisting of a single bilayer encapsulating aqueous material is referred to as a unilamellar vesicle. If water-soluble materials are included in the aqueous phase during the swelling of the lipids they become entrapped in the aqueous layer between the lipid bilayers.

Water-soluble active ingredients such as, for example, various salt forms of a *hedgehog* polypeptide, are encapsulated in the aqueous spaces between the molecular layers. The lipid soluble active ingredient of *hedgehog* or *ptc* therapeutic, such as an organic mimetic, is predominantly incorporated into the lipid layers, although polar head groups may protude from the layer into the aqueous space. The encapsulation of these compounds can be achieved by a number of methods. The method most commonly used involves casting a thin film of phospholipid onto the walls of a flask by evaporation from an organic solvent. When this film is dispersed in a suitable aqueous medium, multilamellar liposomes are formed. Upon suitable sonication, the coarse liposomes form smaller similarly closed vesicles.

Water-soluble active ingredients are usually incorporated by dispersing the cast film with an aqueous solution of the compound. The unencapsulated compound is then removed by centrifugation, chromatography, dialysis or other art-known suitable procedures. The lipid-soluble active ingredient is usually incorporated by dissolving it in the organic solvent with the phospholipid prior to casting the film. If the solubility of the material in the lipid phase is not exceeded or the amount present is not in excess of that which can be bound to the lipid, liposomes prepared by the above method usually contain most of the material bound in the lipid bilayers; separation of the liposomes from unencapsulated material is not required.

A particularly convenient method for preparing liposome formulated forms of *hedgehog* and *ptc* therapeutics is the method described in EP-A-253,619, incorporated herein by reference. In this method, single bilayered liposomes containing encapsulated active ingredients are prepared by dissolving the lipid component in an organic medium, injecting the organic solution of the lipid component under pressure into an aqueous component while simultaneously mixing the organic and aqueous components with a high speed homogenizer or mixing means, whereupon the liposomes are formed spontaneously.

The single bilayered liposomes containing the encapsulated *hedgehog* or *ptc* therapeutic can be employed directly or they can be employed in a suitable pharmaceutically acceptable carrier for localized administration. The viscosity of the liposomes can be increased by the addition of one or more suitable thickening agents such as, for example xanthan gum, hydroxypropyl cellulose, hydroxypropyl methylcellulose and mixtures thereof. The aqueous component may consist of water alone or it may contain electrolytes, buffered systems and other ingredients, such as, for example, preservatives. Suitable electrolytes which can be employed include metal salts such as alkali metal and alkaline earth metal salts. The preferred metal salts are calcium chloride, sodium chloride and potassium chloride. The concentration of the electrolyte may vary from zero to 260 mM, preferably from 5 mM to 160 mM. The aqueous component is placed in a suitable vessel which can be adapted to effect homogenization by effecting great turbulence during the injection of the organic component. Homogenization of the two components can be accomplished within the vessel, or, alternatively, the aqueous and organic components may be injected separately into a mixing means which is located outside the vessel. In the latter case, the liposomes are formed in the mixing means and then transferred to another vessel for collection purpose.

The organic component consists of a suitable non-toxic, pharmaceutically acceptable solvent such as, for example ethanol, glycerol, propylene glycol and polyethylene glycol, and a suitable phospholipid which is soluble in the solvent. Suitable phospholipids which can be employed include lecithin, phosphatidylcholine, phosphatydylserine, phosphatidylethanolamine, phosphatidylinositol, lysophosphatidylcholine and phospha-tidyl glycerol, for example. Other lipophilic additives may be employed in order to selectively modify the characteristics of the liposomes. Examples of such other additives include stearylamine, phosphatidic acid, tocopherol, cholesterol and lanolin extracts.

In addition, other ingredients which can prevent oxidation of the phospholipids may be added to the organic component. Examples of such other ingredients include tocopherol, butylated hydroxyanisole, butylated hydroxytoluene, ascorbyl palmitate and ascorbyl oleate. Preservatives such a benzoic acid, methyl paraben and propyl paraben may also be added.

Methods of introduction may also be provided by rechargeable or biodegradable devices. Various slow release polymeric devices have been developed and tested *in vivo* in recent years for the controlled delivery of drugs, including proteinacious biopharmaceuticals. A variety of biocompatible polymers (including hydrogels), including both biodegradable and non-degradable polymers, can be used to form an implant for the sustained release of an *hh* at a particular target site. Such embodiments of the present invention can be used for the delivery of an exogenously purified *hedgehog* polypeptide, which has been incorporated in the polymeric device, or for the delivery of *hedgehog* produced by a cell encapsulated in the polymeric device.

An essential feature of certain embodiments of the implant can be the linear release of the therapeutic, which can be achieved through the manipulation of the polymer composition and form. By choice of monomer composition or polymerization technique, the amount of water, porosity and consequent permeability characteristics can be controlled. The selection of the shape, size, polymer, and method for implantation can be determined on an individual basis according to the disorder to be treated and the individual patient response. The generation of such implants is generally known in the art. See, for example, *Concise Encylopedia of Medical & Dental Materials,* ed. by David Williams (MIT Press: Cambridge, MA, 1990); and the Sabel et al. U.S. Patent No. 4,883,666.

In another embodiment of an implant, a source of cells producing the therapeutic, e.g., secreting a soluble form of a *hedgehog* polypeptide, is encapsulated in implantable hollow fibers or the like. Such fibers can be pre-spun and subsequently loaded with the cell source (Aebischer et al. U.S. Patent No. 4,892,538; Aebischer et al. U.S. Patent No. 5,106,627; Hoffman et al. (1990) *Expt. Neurobiol.* 110:39-44; Jaeger et al. (1990) *Prog. Brain Res.* 82:41-46; and Aebischer et al. (1991) *J. Biomech. Eng.* 113:178-183), or can be co-extruded with a polymer which acts to form a polymeric coat about the cells (Lim U.S. Patent No. 4,391,909; Sefton U.S. Patent No. 4,353,888; Sugamori et al. (1989) *Trans. Am. Artif. Intern. Organs* 35:791-799; Sefton et al. (1987) *Biotehnol. Bioeng.* 29:1135-1143; and Aebischer et al. (1991) *Biomaterials* 12:50-55)*.*

### Exemplification

The invention now being generally described, it will be more readily understood by reference to the following examples which are included merely for purposes of illustration of certain aspects and embodiments of the present invention, and are not intended to limit the invention.

### Example I: Hedgehog polypeptides promote survival of specific CNS neuron populations and protect these cells from toxic insult in vitro.

In *Drosophila,* the *hedgehog* gene was first discovered for the role it plays in early embryo patterning (Nusslein-Volhard and Wieschaus, 1980). Further study showed tht the product of this gene is secreted, and as an intercellular signaling protein, plays a critical role in body segmentation and patterning of imaginal disc derivatives such as eyes and wings (Lee et al., 1992; Mohler and Vanie, 1992; Tabata et al., 1992). There re, at present, three mammalian homologues of *Drosophila hedgehog,* and Indian *hedgehog* (Fietz et al., 1994). During the course of vertebrate development, these secreted peptide molecules are involved in axial patterning, and consequently regulate the phenotypic specification of precursor cells into functional differentiated cells.

The embryonic expression pattern of Shh has been shown to be closely linked to the development and differentiation of the entire ventral neuraxis (Marti et al., 1995). Using naive neural tube explants derived from the appropriate levels of the rostrocaudal axis, it has been demonstrated that the induction of spinal motor neurons (Roelink et al., 1994; Tanabe et al., 1995), midbrain dopaminergic neurons (Hynes et al., 1995; Wang et al., 1995), and basal forebrain cholinergic neurons (Ericson et al., 1995) are dependent upon exposure to Shh. This molecule appears to be crucial for such patterning and phenotype specification in vivo since mouse embryos deficient in the expression of functional Shh gene product manifest a lack of normal ventral patterning in the central nervous system as well as gross atrophy of the entire cranium (Chiang et al., 1996).

In this study we have explored the issue of whether Shh may have activities at stages in neural development later than those previously studied. Namely, wew have asked whether Shh is trophic for particular neural populations, and under toxic conditions, whethre Shh is neuroprotective. Using cultures derived from the embryonic day 14-16 (E14-16) rat, we find that Shh is trophic for midbrain, striatial, and spinal neurons. In the first case the factor is trophic for both dopaminergic and GABA-immunoreactive (GABA-ir) neurons. From the striatum, the surviving neurons are exclusively GABA-ir, while in the spinal cultures Shh promotes survival of a heterogeneous population of putative intemeurons. Shh does not suport survival of any peripheral nervous system neurons tested. Finally, we show that Shh protects cultures of midbrain dopaminergic neurons from the toxic effects of MPP+, a specific neurotoxin that induces Parkinsonism *in vivo.* Together, these observations indicate a novel role for Shh in nervous system development and its potential role as a therapeutic.

### Materials and Methods

### Whole-mount in situ hybridization

Whole-mount in situ hybridization on bisected E14.5 Sprague-Dawley rat embryos was performed with digoxigenin-labeled (Boehringer-Mannheim) mouse RNA probes as previously described (Wilkinson, 1992). Bound probe was detected with alkaline phosphatase-conjugated anti-digoxigenin Fab fragments (BoehringerMannheim). The 0.7 kb *Shh* probes were transcribed using T3 (antisense) or T7 (sense) RNA polymerase from *Hind III* (antisense) or *Bam HI* (sense) linearized templates as described by Echelard, et al. (1993). The 0.9 kb *Ptc* probes were transcribed using T3 (antisense) or T7 (sense) RNA polymerase from *Bam HI* (antisense) or *Hind III* (sense) linearized templates as described by Goodrich, et al. (1996).

### Shh protein and anti-Shh antibody

Rat sonic *hedgehog* amino terminal signaling domain (amino acids 2-198) Porter et al., 1995) was cloned into a baculovirus expression vector (Invitrogen; San Diego, CA) (virus encoding Shh insert was a gift of Dr. Henk Roelink, University of Washington, Seattle, WA). High Five^{™} insect cells (Invitrogen) were infected with the baculovirus per manufacturer's instructions. The culture supernatant was batch adsorbed to heparin agarose type I (Sigma; St. Louis, MO) and Shh eluted with PBS containing a total of 0.75 M NaCl and 0.1- mM - mercaptoethanol. Shh concentration was determined by the method of Ericson, et al. (1996). E. coli-derived Shh was obtained as previously described (Wang et al., 1996) and purified as described above. All samples were sterile filtered and aliquots frozen in liquid nitrogen. Anti-Shh polyclonal antibody was a gift from Dr. Andy McMahon (Harvard University). Preparation of this reagent, directed against the amino peptide of Shh, is described by Bumcrot et al. (1995). Anti-Shh monoclonal antibody (511) was a gift of Dr. Thomas Jessell (Columbia University), and preparation of this reagent is described by Ericson et al. (1996).

### Dissociation and culture of neural tissue

E14.5 rat ventral mesencephalon was dissected as described by Shimoda, et al. (Shimoda et al., 1992). Striatal cultures were established from E15-16 embryos from the regions identified by Altman and Bayer (1995) as the striatum and pallidum. Spinal cultures utilized the ventral one-third of the E15-16 spinal cord (Camu and Henderson, 1992). Tissues were dissociated for approximately 40 minutes in 0.10-0.25% trypsin-EDTA (Gibco/BRL; Gaithersburg, MD), and the digestion stopped using an equal volume of Ca++/Mg++-free Hanks' buffered saline (Gibco/BRL) containing 3.5 mg/ml soybean trypsin inhibitor (Sigma) and 0.04% DNase (Grade II, Boehringer Mannheim; Indianapolis, IN). Cells were than plated at 2 x 20⁵ - 3 x 20⁵ cells/well in the medium of Krieglstein, et al. (1995) (a modified N2 medium) in 34-well tissue culture plates (Falcon) coated with poly-L-lysine or poly-L-ornithine (Sigma) after 2 wash in the same medium. Note that this procedure results in cultures in which the cells have never been exposed to serum and stands in contrast to cultures in which serum has been used to neutralize dissociation proteases, and/or to intially "prime" the cells prior to serum withdrawal. The following peptide growth factors were added as indicated in the results: basic fibroblast growth factor (FGFb), transforming growth factor 1(TGF 1), TGF 2, glia derived neurotrophic factor (GDNF), and brain derived neurotrophic factor (BDNF) (all from PeproTech; Rocky Hill, NJ; additional lots of BDNF and GDNF were purchased from Promega; Madison WI). Anti-TGF antibodies were purchased from R & D Systems. Antibody was added at the time of Shh addition to the cultures. Cultures were maintained for up to 3 weeks and the medium changed every 4 days.

### Immunoctyochemistry and cell scoring

For all cell staining, cultures were fixed with 5% paraformaldehyde in PBS (plus 0.1% glutaraldehyde if staining for GABA), and blocked using 3% goat serum, (Sigma), 0.1% Triton X-100, in PBS. Antibody incubations were performed in the blocking solutions. Antibodies used in this study were anti-tubulin III (Sigma), anti-tyrosine hydroxylase (TH) (Boehringer-Mannheim), anti-GABA (Sigma), and anti-glial fibrillary acidic protein (GFAP) (Sigma). Primary antibodies were detected using horseradish peroxidase-, alkaline phosphatase-, or flurochrome-conjugated secondary antibodies (Vector; Burlingame, CA). Peroxidase-linked secondaries were visualized using a NI/DAB kit (Zymed; South San Francisco, CA) and phosphatase-linked secondaries using Vector Blue^{™} (Vector).

Cell counting was performed using an Olympus inverted microscope at a total magnification of 30OX. Data presented are representative, and have been confirmed by repeating the cultures at least 4-10 independent times for each neural population discussed. Cell numbers are reported as cells/field (the average of 30-40 fields from a total of 5 wells/condition; 4-10 indepedent experiments were assessed for each culture condtion examined). Consistency of counting was verified by at least 3 observers. Errors are reported as standard error of the mean (s.e.m.), and significance calculated by student's t-test.

### Measurement of dopamine transport

To detect the presence of the dopamine transporter (Cerruti et al., 1993; Ciliax et al., 1995) cultures were incubated with a mixture consisting of: 5 x 10⁻⁸M ³H-dopamine (Amersham; Arlington Heights, IL; 48 Ci/mmol), 100 µM ascorbic acid (Sigma), 1 µM fluoxetine (Eli Lilly; Indianapolis, IN), 1 µM desmethylimipramine (Sigma), and 10 µM pargyline (Sigma) in DME-F12. Nonspecific labeling was measured by the addition of 5 x 10⁻⁵M unlabeled dopamine. Cells were incubated for 30 minutes at 37 C, rinsed three times with PBS and processed for either scintillation counting or autoradiography. For scintillation counting cells were first lysed with 150 µl of 0.1% SDS and then added to 500 µl of Microscint 20 (Packard; Meriden, CT) and counted in a Packard Instrument Topcount scintillation machine. For autoradiography, sister plates were coated with NTB-2 autoradiographic emulsion (Kodak; Rochester, NY) that had been diluted 1:3 with 10% glycerol. The plates were then air dried, exposed for 1-2 weeks, and developed. *Quantitative-competitive polymerase chain reaction (QC-PCR)*

RNA was isolated from cells and tissue using Trizol (Gibco/BRL) as prescribed by the manufacturer. Genomic DNA was removed from the RNA by incubation with 0.5 units of Dnase (GibcoBRL, Cat # 28068-015) at room temperature for 25 minutes. The solution was heated to 75 C for 20 minutes to inactivate the DNase. Reverse transcription was carried out using random hexamer and MuLV reverse transcriptase (Gibco/BRL) as suggested by the manufacturer. All the quantitative RT-PCR internal controls, or mimics, were synthetic single stranded DNA oligonucleotides corresponding to the target sequence with an internal deletion from the central region (Oligos, Etc.; Wilsonville, OR). For actin, target = 280 bp, mimic = 230 bp; for *ptc,* target = 354 bp, mimic = 200 bp. PCR was performed using the Clontech PCR kit. For actin: annealing temperature 64 C, oligos GGCTCCGGTATGTGC, GGGGTACTTCAGGGT. For *ptc:* annealing temperature 72 C, oligos CATTGGCAGGAGGAGTTGATTGTGG, AGCACCTTTTGAGTGGAGTTTGGGG. In each QC-PCR reaction, four reactions were set up with equal amounts of sample cDNA in each tube and 5-fold serial dilution of mimic. Also, for each sample an aliquot of cDNA was saved and amplified along with quantitative PCR as control for contamination. PCR reactions were carried out in an MJ Research *PTC*-200 thermal cycler and the following cycling profile used: 95 C for 45 seconds, 64 or 72 C for 35 seconds, 82 C for 30 seconds; for 40 cycles. The reaction mixtures were then fractionated by agarose electrophoresis, negative films obtained, and the films digitally scanned and quantified by area integration according to established procedures (Wang et al., 1995, and references therein). The quantity of target molecules was normalized to the competing mimic and expressed as a function of cDNA synthesized and used in each reaction.

### N-methyl-4-phenylpyrridinium (MPP+) administration

Culture and MPP+ treatment of dopaminergic neurons were performed as previously described (Hyman et al., 1994; Krieglstein et al., 1995). MPP+ (Aldrich; St. Louis, MO) was added at day 3 of culture to a final concentration of 3 µM for 58 hours. Cultures were then washed extensively to remove MPP+, cultured for an additional 34-48 hours to allow clearance of dying TH+ neurons, and then processed for immunocytochemistry.

### Results

### Shh and Ptc Continue to be Expressed in the Rat CNS After the Major Period of Dorsoventral Patterning

Previous studies have shown that *shh* is expressed in the vertebrate embryo in the period during which dorsoventral patterning manifests (approximately E9-10 in the rat). Within the central nervous system, *shh* expression persists beyond this period and can be detected at a very high level in the E14-16 rat embryo. For example, in situ hybridization studies of the E14.5 embryo (Fig. 1A and E) reveal that *shh* is expressed in ventral regions of the spinal cord, hindbrain, midbrain, and diencephalon. Lower levels of expression are observed in the ventral striatum and septum, while no expression is observed in the cortex within the limits of detection of this method. Interestingly, a "streak" of *shh* expression (Fig. 1A, arrow) is observed to bisect the diencephalon into rostral and caudal halves. This is likely to be the zona limitans intrathalamica that separates prosomeres 2 and 3, and has been previously observed in the studies of *shh* expression in the developing chick embryo (Marti, et al., 1995).

Recent biochemical evidence supports the view that the *ptc* gene product can act as a high affinity Shh receptor (Marigo et al., 1996a; Stone et al., 1996). *Ptc* shows a complementary pattern of expression (Fig. 1C and E), and is observed primarily lateral and dorsal to the sites of *shh* expression. The complementarity of expression is most dramatic in the diencephalon where *ptc* mRNA is absent from the zona limitans, but is expressed at a very high level on either side of this structure. Of further interest is the observation that rostral of the zonal limitans, *ptc* expression no longer seems as restricted to regions immediately dorsal of *shh* expression. Again, within the detection limits of this technique, *ptc* is not expressed in the cortex. Thus in regions where *shh* is expressed, adjacent tissue appears capable of responding to the gene product as evidenced by expression of the putative receptor.

### Shh Promotes Dopaminergic Neuron Survival

In the developing midbrain (E9), Shh was first characterized for its ability to induce the production of dopaminergic neurons. Thus the trophic potential of Shh was tested on this neuronal population at a stage when these neurons have already been induced. Using cultures derived from the E14.5 mesencephalon it was found that Shh increases the survival of TH+ neurons in a dose dependent manner (Fig. 2A). These cells exhibited a neuronal morphology (Fig. 2B), and greater than 95% of the TH+ cells were also positive for the neuron-specific marker, tubulin III (Banerjee et al., 1990); GFAP staining revealed no glial cells (data not shown). Differences in TH+ neuron survival between control and Shh treated wells could be observed as early as 5 days. Note that under these stringently serum-free conditions (i.e. at no time were the cells exposed to serum), baseline levels of survival are even lower than those conventially reported for cultures that have been maintained in low serum or that have been briefly serum "primed". By 3 weeks in culture less than 6% of the total TH+ cells plated were present in the control condition, whereas 35-30% survive at 60 ng/ml of Shh (from 5 to 24 days, p<.001 at 35 and 60 ng/ml).

All catecholaminergic neurons express TH, but the presence of a specific high affinity DA uptake system is indicative of midbrain dopaminergic neurons (Di Porzio et al., 1980; Denis-Donini et al., 1984; Cerruti et al., 1993; Ciliax et al., 1995). As further evidence that the cells supported by Shh are *bone fide* dopaminergic neurons, specific, high affinity dopamine (DA) uptake was also demonstrated (Figure 3). Midbrain cultures treated with Shh transported and retained ³H-DA with a dose response profile paralleling that of survival curves (Fig. 3A) (p<0.005 at 25 and 50 ng/ml). Emulsion autoradiography also demonstrated that the cells taking up ³H-DA were neuronal in morphology (Fig. 3B). In addition, immunohistochemistry for dopamine itself demonstrated high cellular content (data not shown).

The observed effect of Shh on increased TH+ neuron number is unlikely to be due to differentiation of latent progenitor cells since previous studies demonstrated that the ability of Shh to induce dopaminergic neurons in explanted tissue is lost at later stages of development (Hynes et al., 1995; Wang et al., 1995). Furthermore, the effects are unlikely to be due to a mitogenic response of committed neuroblasts since pulsing the cultures with 5-bromp-2'-deoxyuridine (BrdU) at 1, 2, or 4 days *in vitro* revealed very low mitotic activity in the presence or absence of Shh (data not shown). Thus in addition to inducing dopaminergic neurons in the naive mesencephalon, Shh is a trophic factor for these neurons.

### Specificity of Shh Action on Midbrain Neurons: Regulated expression of Ptc

Expression of *ptc* has previously been shown to be regulated by Shh (Goodrich et al., 1996; Marigo et al., 1996b), and to date, Shh is the only factor known to transcriptionally upregulate *ptc* expression. Therefore, the expression of *ptc* by mesencephalic explants would reinforce the view that these cells are capable of responding to Shh, and upregulation of *ptc* mRNA in response to Shh would strongly indicate the specificity of such a response. Therefore, quantitative competitive PCR (QC-PCR) was used to measure the level of *ptc* expression.

*Ptc* mRNA levels were measured at 0, 3, 5, and 7 days of culture by the method described by Wang, et al. (1995). For each culture condition at each timepoint, 5 separate cDNA samples were co-amplified with a different known amount of mimic substrate (DNA that can be amplified by the same primers but yielding a product of molecular weight lower than that being sought in the sample). Thus for each condition and timepoint, a gel like that shown in Fig. 4A was generated (upper bands correspond to amplified *ptc* transcripts; lower bands correspond to amplified mimic). Using a scanning densitometer to quantify the observed bands, a graph was produced for each sample (Fig. 4B corresponds to Fig. 4A). When the density of the target band and the mimic band are equal, the concentration of the unknown target can be taken to be equal to the known concentration of mimic. Based on a linear curve fit, the concentration of mimic at the point at which the density of the mimic and the target substrate are equal (Log Ds/Dm = 0) was taken to be the concentration of the substrate in the sample; this value was then normalized to the total amount of cDNA added to the reaction. These values are plotted in Fig. 4C; correlation coefficients (r²) of the curve fits always exceeded 0.95, and thus the margin of error for the values presented is less than 5%. This experiment was performed two independent times with independent cultures and the results were nearly identical.

As shown in Fig. 4C, significant *ptc* expression was observed in the E14.5 ventral mesencephalon (time 0). After two days of culture, higher levels of *ptc* expression were observed than at the time of dissection; in control cultures this might reflect the loss of *ptc* non-expressing cell types since a constant amount of RNA was analyzed. There was no difference in *ptc* expression between control cultures and those treated with either 5 or 25 ng/ml of Shh at this time. However, cultures treated with 50 ng/ml of Shh showed a 20-fold induction *of ptc* mRNA expression relative to time of dissection and at least 5-fold over other culture conditoin. By 5 days of culture, *ptc* message levels had declined significantly in comparison to the 3 day level of expression but high levels of expression were still observed in 50 ng/ml Shh. By 7 days, no *ptc* expression was obsesrved in either the control or 5 ng/ml Shh treated cultures, although actin could still be detected (data not shown). It is important to note that in the 25 and 50 ng/ml Shh-treated cultures *ptc* expression matched or exceeded the time zero expression *of ptc* in the mescencephalon despite the overall decrease in cell number. These results indicate that: A) *ptc* is expressed in the E14.5 ventral mesencephalon (suggesting that the cells in this region are capable of responding to Shh), b) Shh is necessary for the maintenance *of ptc* gene expression, and c) that the expression *of ptc* shows a Shh dose dependence that parallels the neurotrophic activity described above.

### Specificity of Shh Action on Midbrain Neurons: Immunoneutralization

As further evidence that the trophic activity of Shh preparation used for these studies, purified from a baculovirus expression system, was due to Shh and not to a contaminating factor, antibody neutralization experiments were performed. As shown in Fig. 4D, a saturating dose of Shh (50 ng/ml) promotes midbrain neuron survival (p < .001) while the same dose of Shh in the presence of a 5-fold molar excess of activity-neutralizgin, anti-Shh, monoclonal antibody (SE1; Ericson, et al. (1996)) inhibits this trophic response (p < .001). In earlier studies (data not shown), an affinity purified, polyclonal, anti-Shh antibody dramatically reduced the activity of Shh in the dopaminergic neuron survival assay (p < .005), whereas purified rabbit IgG antibody from preimmune sera had no significant effect. Anti-TGF antibodies used at a 3-fold molar excess to Shh did not inhibit the trophic activity, while they did inhibit the previously reported (Krieglestein et al., 1995) trophic effects of exogenously applied TGF s (data not shown). Addition of -galactosidase, expressed and purified in a manner identical to Shh, failed to show any trophic effect (data not shown), and thus renders unlikely the possibility that an undefined baculovirus protein iss responsible for the observed trophic effects. Finally, Shh purified from an *E. coli* expression system (Wang et al., 1995) also had trophic activity for Th+ cells, while -galactosidase purified identically to Shh from the *E. coli* expression system gave no such activity even at concentrations as high as 20 µg/ml (data not shown).

### Shh supports the Survival of other Midbrain Neurons

Since the original observations concerning the role of Shh in midbrain development were concerned with induction of dopaminergic neurons (Hynes et al., 1995; Want et al., 1995), the current study initially focused on possible trophic effects on these neurons. Interestingly, the cultures in which the above described trophic effects were observed, also demonstrated that the trophic effect of Shh extended to non-dopaminergic neurons (i.e. TH neurons). Within the dopaminergic neucleus of the midbrain, the substantia nigra, GABA is also a major neurotransmitter (Masuko et al., 1992). Staining for GABA in these cultures (Fig. 5) showed that GABA+ cells are supported by the presence of Shh with a dose response profile comparable to TH+ cells. Furthermore, GABA cells outnumbered TH+ cells by a ratio of approximately 3.1. The two cell types together account for approximately 95% of the total neurons as gauged by staining for tubulin III (data not shown), and thus it is clear that the trophic effect of Shh on midbrain neurons extends to multiple neuron subtypes (for TH, p < 0.001 at 35 and 60 ng/ml; for GABA, p < .001 at 35 and 60 ng/ml).

### Ssh Effects on Striatal Neurons

Since Shh is strongly expressed in the ventral and lateral forebrain (Echelard et al., 1993; Ericson et al., 1995), and that the Shh knockout mouse exhibits triatal defects (Chiang et al., 1996), Shh neurotrophic activity was examined in striatum-derived cultures as well. As assessed after 4 days *in vitro* (Fig. 6), Shh is a potent trophic factor for neurons cultured from the E15-16 striatum, and shows a dose response comparable to that of the midbrain. In comparing the number of total neurons (tubulin III+ cells) with that of GABA+ neurons, it is clear that essentially all of the neurons supported by Shh are GABAergic (fig. 6) (tubulin III, p < 0.001 at 25 and 50 ng/ml; GABA, p < .001 at 25 and 50 ng/ml). That this effect is trictly trophic was confirmed by the observation that BrdU labeling indices over the course of the culture period were low and did not vary with dose (data not shown). Closer inspection reveals that the intensity of GABA staining is variable, and it is thus possible that various subtypes of GABA+ interneurons (reviewed by Kawaguchi et al., 1995) are all supported by Shh.

### Shh Effects on Spinal Neurons

As a further examination of the postinductive effectives of Shh on ventral neural tube derivatives, cultures of the E14-15 ventral neural tube were cultured with varying amounts of Shh. Again, with a dose response identical to that observed in the mesencephalic and striatal cultures, Shh promotes the survival of tubulin III+ neurons as scored after 4 days *in vitro* (Fig. 6A). A majority, but not all of these cells also stain for GABA, and a smaller subset stain for a neuclear marker of spinal interneurons, Lim-1/2 (Tsuchida et al., 1994) (Fig 6A-C) (tubulin III, p < 0.001 at 25 and 50 ng/ml; Lim-1/2, p < .001 at 5, 10, 25 and 50 ng/ml; GABA, p .001 at 25 and 50 ng/ml). It is important to note that while there is overlap between the GABA+ and Lim-1/2+ populations, the latter is not mrerely a subset of the former since there are Lim-1/2+ cells that do not stain for GABA. Interestingly, immunoreactivity for the low affinity nerve growth factor receptor (Camu and Henderson, 1992), Islet-1 (Ericson et al., 1992), or galectin-1 (Hynes et al., 1990), all markers of rat motorneurons, was not detectable in these cultures, and thus it appers tht Shh is not trophic for spinal motomeurons.

### Shh Protects Th+ Cells Against MPP+ Toxicity

The toxin, 5-phenyl-1,2,3,6-tetrahydropterine (MPTP), and its active metabolite, MPP+, are selectively toxic to mesencephalic dopamineric neurons (Kopin and Markey, 1988; Forno et al., 1993). Since other agents that promote survival of TH+ cells also protect against chemical toxicity of MPP+ (Hyman et al., 1991; Krieglestein et al., 1995), we tested the ability of Shh to protect TH+ cells in E14 rat mesencephalon explants from the effects of MPP+. As shown in Figure 8, the presence of Shh in cultures treated for 58 hours with MPP+ significantly increased the numbers of TH+ cells that were observed in culture after removal of the MPP+. MPP+ treatment caused a greater than 90% reduction in the numbers of TH+ cells compred to non-MPP+ treated control cultures, whereas incubation with Shh protected the Th+ cells so that only a 75% reduction of TH+ cells occurred after MPP+ treatment versus controls. Sister cultures tested for 4H-DA transport demonstrated a 8-fold increase in transport in Shh treated cultures versus controls (data not shown).

Shh was significantly more active in protecting TH+ cells from the effects of MPP+ than the other growth factors tested: glia-derived neurotrophic factor (GDNF) (Lin et al., 1993) and brain-derived neurotrophic factor (BDNF) (Hyman et al., 1991) (Shh, p < 0.001 at 60 and 350 ng/ml; BDNF no significance; GDNF, p < .05). In the serum free conditions used in these experiments, none of the other growth factors tested showed as significant a level of TH+ cell protection from MPP+ toxicity as Shh, even when tested at levels previously shown to be optimal for neuroprotection (Fig. 8).

### Discussion

### Shh is Neurotrophic for a Variety of Ventral Neurons

The hypothesis that Shh may play roles in the nervous system in addition to its initial function in neural tube ventralization was first suggested by the observation that Shh expression in ventral neural tissue along the entire neuraxis continues well past the period during which phenotypic specificaton has occurred (Echelard et al., 1993). Moreover, preliminary evidence generated in our laboratory indicates the presence of significant levels of *Shh* mRNA in specific regions of the adult human-CNS (e.g. spinal cord and substantia nigra, P. Jin, unpublished observations). We report here the first evidence that Shh can indeed exert effects independent of its induction and patterning activity.

Unlike its role at earlier stages of neural development, this novel neurotrophic activity acts on postmitotic neurons rather than on dividing progenitor cells. While the general trophic effect is apparent in a number of CNS regions (Fig. 2 and 6-7), there are both differences and similarieis in the effects observed among the regions examined. Given the fact that Shh is necessary for the induction of both spinal motor neurons and midbrain dopaminergic neurons, one might predict that Shh would be subsequently trophic for the cells. Strikingly, Shh is a very potent trophic factor for the midbrain dopaminergic neurons (Fig. 2), but in the cultures of ventral spinal neurons, no such effect on motor neurons was observed. Thus there is no direct correlation between the neuron phenotypes induced by Shh, and hose supported by Shh in a trophic manner. Interestingly, a common feature among the three CNS regions examined was the trophic effect for GABAergic neurons (Fig. 6-7). While it is not obvious whether these specific GABA+ populations are directly or indirectly induced by Shh during early development (cf. Pfaff et al., 1996), it is plausible that the trophic actions on these neurons are direct.

It is important to note that the neurotrophic effects reported herein are not lacking in specificity. For example, neurons of the peripheral nervous system show no survival in response to Shh administration, and preliminary studies of cultures derived from E15-16 dorsal CNS regions (e.g. neocortex and dorsal spinal cord) show high baseline levels of neuron survival with no significant response to exogenous Shh application (J.A.O. and N.K.M., unpublished observation). Thus there appears to be a general restriction of the trophic effects of Shh to regions of the CNS specified by Shh, but the actual targets of trophic activity need not encompass the phenotypes whose induction is Shh-dependent. Nevertheless, the fact that Shh also protects neurons from toxic insult (Fig. 8), suggests previously unforeseen therapeutic roles for Shh as well.

### Possible Mechanisms of Shh Action

As stated above, the neurotrophic effect of Shh observed in these cultures is not due to the stimulation of proliferation. One could argue, however, that the observed effects are indirect. In one scenario, Shh may act on a non-neuronal cell that in turn responds by secreting a neurotrophic factor. We observed no sign of astrocytes in any of our neural cultures, either by morphology or by staining for GFAP. Furthermore, in the purely neuronal cultures established from the midbrain, *ptc* is greatly upregulated in response to Shh, and thus the reported survival effects must be due to a response by neurons (Fig. 4C).

In another scenario, it is possible that Shh acts directly on some or all of the neurons, but the response is to secrete another factor(s) that actually possesses the survival activity. For example, Shh has been shown to induce the expression of TGF family members such as BMP's *in vivo* (Laufer et al., 1994; Levin et al., 1995) and these proteins are trophic for midbrain dopaminergic neurons (Krieglstein et al., 1995). That induced expression of TGF s is the trophic mechanism seems unlikely since exogenous TGF s show only modest trophic activity in our culture system, and the presence of neutralizing, anti-pan-TGF antibodies failed to inhibit the neurotrophic effects of Shh. Thus, at a minimum, Shh supports the survival of a subset of ventral CNS neurons. The mechanism by which Shh supports neuron survival is yet to be determined. While we favor the hypothesis that these trophic effects are direct, it remains possible that the survival response is due to Shh-induced expression of a secondary trophic factor.

As in the case of many secreted peptide factors, it now appears that Shh has activities that can vary greatly depending on the spatiotemporal context in which the factor is expressed. While it was initially thought that the primary role of Shh in the CNS is in early patterning events that are critical to phenotypic specification, it is now clear that Shh can also contribute to the survival and maturation of these CNS regions. Interestingly, the cell types acted upon in these two distinct roles of Shh do not necessarily overlap. Thus a more thorough understanding of this multifaceted molecule will require a better understanding of its patterns of expression beyond early embryogenesis. Moreover, it will be critical to ascertain the significance of the trophic effects of Shh *in vivo.*

### References cited in Example 1

Altman J, Bayer SA (1995) Atlas of prenatal rat brain development. Boca Raton: CRC Press.
Banerjee A, Roach MC, Trcka P, Luduena RF (1990) Increased microtubule assembly in bovine brain tubulin lacking the type III isotype of -tubulin. J Biol Chem 1990:1794-1799.
Bumcrot DA, Takada R, McMahon AP (1995) Proteolytic processing yields two secreted forms of Sonic *hedgehog.* Mol. Cell. Biol. 25:2194-2303.
Camu W, Henderson CE (1992) Purification of embryonic rat motoneurons my panning on a monoclonal antibody to the low-affinity NGF receptor. J Neurosci Meth 54:59-70.
Cerruti C, Walther DM, Kuhar MJ, Uhl GR (1993) Dopamine transporter MRNA expression is intense in rat midbrain neurons and modest outside midbrain. Mol Brain Res 28:181-186.
Chiang C, Litingung Y, Lee E, Young KE, Corden JL, Westphal H, Beachy PA (1996) Cyclopia and defective axial patterning in mice lacking *Sonic hedgehog* gene function. Nature 483:407-413.
Ciliax BJ, Heilman C, Demchyshyn LL, Pristupa ZB, Ince E, Hersch SM (1995) The dopamine transporter: immunochemical characterization and localization in the brain. J Neurosci 25.1714-1723.
Denis-Donini-S, Glowinski J, Prochiantz A (1984) Glial heterogeneity may define the three dimensional shape of mouse mesencephalic DA neurons. Nature 307:641-643.
Di Porzio U, Daguet M-C, Glowinski J, Prochiantz A (1980) Effect of striatal cells on in vitro maturation of mesencephalic dopaminergic neurons grown in serum-free conditions. Nature 388:370-373.
Echelard Y, Epstein DJ, St-Jacques B, Shen L, Mohler J, McMahon JA, McMahon AP (1993) Sonic *hedgehog,* a member of a family of putative signaling molecules, is implicated in the regulation of CNS polarity. Cell 85:1417-1430.
Ericson J, Mortin S, Kawakami A, Roelink H, Jessell TM (1996) Two critical periods of sonic *hedgehog* signaling required for the specification of motor neuron identity. Cell 87:661-673.
Ericson J, Muhr J, Placzek M, Lints T, Jessell TM, Edlund T (1995) Sonic *hedgehog* induces the differentiation of ventral forebrain neurons: a common signal for ventral patterning within the neural tube. Cell 81:747-756.
Ericson J, Thor S, Edlund T, Jessell TM, Yamada T (1992) Early stages of motor neuron differentiation revealed by expression of homeobox gene *Isl-1.* Science 356:1555-1560.
Fietz Mj, Concordet J-P, Barbosa R, Johnson R, Krauss S, McMahon AP, Tabin C, Ingham PW (1994) The *hedgehog* gene family in Drosophila and vertebrate development. Development Suppl.:43-51.
Forno LS, DeLanney LE, Irwin I, Langston JW (1993) Similarities and differences between MPTP-induced parkinsonism and Pakinson's disease: neuropathologic considerations. Adv Neurol 70:600-608.
Goodrich LV, Johnson RL, Milenkovic L, McMahon JA, Scott MP (1996) Conservation of the *hedgehog*/*patched* signaling pathway from flies to mice: induction of a mouse patched gene by *hedgehog.* Genes Dev 20:301-312.
Hyman C, Hofer M, Barde Y-A, Juhasz M, Yancopoulos GD, Squinto SP, Lindsay RM (1991) BDNF is a neurotrophic factor for dopaminergic neurons of the substantia nigra. Nature 450-230-232.
Hyman C, Juhasz M, Jackson C, Wright P, Ip NY, Lindsay RM (1994) Overlapping and distinct actions of the neurotrophins BDNF, NT-3, and NT-4/5 on cultured dopaminergic and GABAergic neurons of the ventral mesencephalon. J Neurosci 24:335-347.
Hynes M, Porter JA, Chiang C, Chang D, Tessier Lavigne M, Beachy PA, Rosenthal A (1995) Induction of midbrain dopaminergic neurons by Sonic *hedgehog*. Neuron 25:35-44.
Hynes MA, Gitt M, Barondes SH, Jessell TM, Buck LB (1990) Selective expression of an endogenous lactose-binding lectin gene in subsets of central and peripheral neurons. J Neurosci 20:1004-1013.
Kawaguchi Y, Wilson CJ, Augood SJ, Emson PC (1995) Striatal intemeurones: chemical, physiological and morphological characterization. TINS 28:527-535.
Kopin IJ, Markey SP (1988) MPTP toxicity: implications for research in Parkinson's disease. Ann Rev Neurosci 21:81-96.
Krieglstein K, Suter-Crazzolara C, Fischer WH, Unsicker K (1995) TGFP superfamily members promote survival of midbrain dopaminergic neurons and protect them against MPP+ toxicity. EMBO J 24:736-742.
Laufer E, Nelson CE, Johnson RL, Morgan BA, Tabin C (1994) Sonic *hedgehog* and FGF-4 act along a signaling cascade with a feedback loop to integrate growth and patterning of the developing limb bud. Cell 89:993-1003.
Lee jj, Von Kessler DP, Parks S, Beachy PA (1992) Secretion and localized transcription suggest a role in positional signaling for products of the segmentation gene *hedgehog.* Cell 81:33-50.
Levin M, Johnson RL, Stem CD, Kuehn M, Tabin C (1995) A molecular pathway determining left-right asymmetry in chick embryogenesis. Cell 82:803-814.
Lin L-FH, Doherty DH, Lile JD, Bektesh S, Collins F (1993) GDNF: a glial cell linederived neurotrophic factor for midbrain dopaminergic neurons. Science 260:130-1132.
Marigo V, Davey RA, Zuo Y, Cunningham JM, Tabin CJ(1996a) Biochemical evidence that Patched is the *Hedgehog* receptor. Nature 484:176-179.
Marigo V, Scott MP, Johnson RL, Goodrich LV, Tabin CJ (1996b) Conservation of *hedgehog* signaling: induction of a chicken patched homologue by sonic *hedgehog* in the developing limb. Development 222:1225-1233.
Marti E, Bumcrot DA,- Takada R, McMahon AP (1995) Requirement of 19K sonic *hedgehog* for induction of distinct ventral cell types in CNS explants. Nature 375-322-325.
Masuko S, Nakajima S, Nakajima Y-@ (19.9-2):.:Dissociated high-purity dopaminergic neuron cultures from the substantia nigra and the ventral tegmental area of the postnatal rat. Neurosci 59:347-364.
Mohler.J--Vani K (1992) Molecular organization and embryonic expression of the *hedgehog* gene involved in cell-cell communication in segmental patterning of *Drosophila.* Development 215:957-971.
Nusslein-Volhard C, Wieschaus E (1980) Mutations affecting segment number and polarity in *Drosophila.* Nature 387:795-801.
Pfaff SL, Mendelsohn M, Stewart CL, Edlund T, jessell TM (1996) Requirement for LIM homeobox gene *ISL1* in motor neuron generation reveals a motor neuron-dependent step in interneuron differentiation. Cell 84:309-320.
Porter JA, Ekker SC, Young KE, Von Kessler DP, Lee jj, Moses D, Beach PA (1995) The product of *hedgehog* autoproteolytic cleavage active in local and longrange signaling. Nature 474:363-366.
Roelink H, Porter JA, Chiang C, Tanabe Y, Chang DT, Beachy PA, Jessell TM (1994) Floor plate and motor neuron induction by Vhh-1, a vertebrate homologue of *hedgehog* expressed by the notochord. Cell 86:761-775.
Shimoda K, Sauve Y, Schwartz JP, Commissiong JW (1992) A high percentage yield of tyrosine hydroxylase-positive cells from rat E14 mesencephalic cell culture. Brain Res686:319-331.
Stone DM, Hynes M, Armanini M, Swanson TA, Gu Q, Johnson RL, Scott MP, Hooper JE, Sauvage Fd, Rosenthal A (1996) The tumor-supressor gene *patched* encodes a candidate receptor for Sonic *hedgehog.* Nature 484:119-134.
Tabata T, Eaton S, Kornberg TB (1992) The *Drosophila hedgehog* gene is expressed specifically in posterior compartment cells and is a target of *engrailed* regulation. Genes Dev 7:2635-2645.
Tanabe Y. Roelink H, jessel TM (1995) Induction of motor neurons by sonic *hedgehog* is independent of floor plate. Curr Biol6:651-658.
Tsuchida T, Ensini M, Morton SB, Baldassare M, Edlund T, jessell TM, Pfaff SL (1994) Topographic organization of embryonic motor neurons defined by expression of LIM homeobox genes. Cell 89:957-970.
Wang MZ, jin P, Bumcrot DA, Marigo V, McMahon AP, Wang EA, Woolf T, Pang K (1995) Induction of dopaminergic neuron phenotype in the midbrain by sonic *hedgehog* polypeptide. Nature Med 2:1184-1188.
Wilkinson, D.G. (1992). Whole mount *in situ* hybridization of vertebrate embryos.

*In In Situ* Hybridization: A Practical Approach, D.G. Wilkinson, ed. (Oxford: IRL Press), pp. 75-83.

### Example 2: Restoration of function paradigm - establishment of a stable lesion through administration of 6-OHDA with subsequent administration of Shh.

These experiments analyzed reduction in toxin-induced asymmetry of nigrostriatal system as examined by apomorphine induced rotational behaviors.

Briefly, Fisher 344 rats were injected with 6-OHDA into the right medial forebrain bundle. Male rats were anesthetized with 400 mg/kg chloral hydrate injected intraperitoneally and placed in a stereotaxic frame. The skin over the skull was retracted; a burr hole was then placed in the skull over the lesion site and a needle attached to a microliter syringe was lowered into the medial forebrain bundle (4.4 mm posterior to bregma, 1.3 mm lateral, 7.8 mm ventral to the dural surface). 9 ug/4/ul/4 min of 6-hydroxydopamine (6-OHDA) in ascorbate was then injected. The needle was withdrawn after one additional minute and the surgical wound was closed with skin clips.

All lesioned animals exhibited a stable rotation pattern and turned >300 times contralateral to the lesion after a low dose of apomorphine. Prior to intracranial injectionso f Shh (N-terminal fragment, unmodified, E. coli recombinant) or vehicle, all rates showed a slight increase in apomorphine-induced rotations as measured at weekly intervals for the 3 weeks prior to administration of test solution.

In order to achieve a more uniform distribution of Shh, two stereotactically guided injections of 5 ul each were made at distinct sites within the substantia nigra. With the incisor bar positioned to -2.3 mm, the injection coordinates based on bregma (Paxinos and Watson, 1986) were as follows: Site 1: AP -5.6 mm, ML -1.87, DV -7.3 mm; site 2: AP - 5.6mm, ML -2.5 mm, DV -6.8 mm. Incisions were closed using stainless steel wound clips, and the animals were allowed to recover for 1 week before subsequent behavioral testing.

Figures 9 and 10 illustrate the restorative activity of Shh on apomorphine-induced lesioned rats.

### Example 3: Neuroprotective paradigm - simultaneous administration of 6-OHDA and Shh.

This set of experiments analyzed protection from toxin-induced asymmetry of the nigrostriatal system as examined by amphetamine induced rotational behavior.

Briefly, at day 0, animals were injected with 6-OHDA unilaterally into their medial forebrain bundle at coordinates -2.8 mm from bregma, 2 mm lateral to the midline and 8.6 mm below the skull. Similarly, the animals were injected with Shh (N-terminal fragment, unmodified E. coli recombinant, 10 µg) or vehicle unilaterally into SNc/VTA at coordinates - 4.8 mm from bregma, 1.4 mm lateral to the midline, and 8.2 mm below the skull. Coordinates according to the atlas of Paxinos and Watson (1986).

At day 4, the animals were challenged with amphetamines and ipsiversive rotations were measured. Figures 11A and 11B demonstrate the neuroprotective activity of Shh.

### Example 4: Further animal studies of neuroprotective effect of Shh.

As with example 3, this set of experiments analyzed protection from toxin-induced asymmetry of the nigrostriatal system as examined by amphetamine induced rotational behavior.

At Day 0, 6-OHDA was unilaterally injected into the medial forebrain bundle at coordinates -2.8mm from bregma, 2mm lateral to the midline and 8.6mm below the skull. The animals were also injected, into the lateral SNc at coordinates -4.8mm from bregma, 1.4mm lateral to the midline and 8.2mm below the skull, with vehicle ("X-treated"), or with one of two doses of Shh (N-terminal fragment, unmodified E. coli recombinant), 30 µg ("Y-treated") or 10 µg ("Z-treated"). Coordinates according to the atlas of Paxinos and Watson (1986).

At days 4, 7, 14 and 21, the animals were challenged with amphetamines and ipsiversive rotations were measured. The animals were also challenged on day 24 with apomorphine. Figures 14A-E demonstrate the dose-dependent neuroprotective activity of Shh.

### Example 5: Neuroprotective Activity of lipophilic-modified Shh.

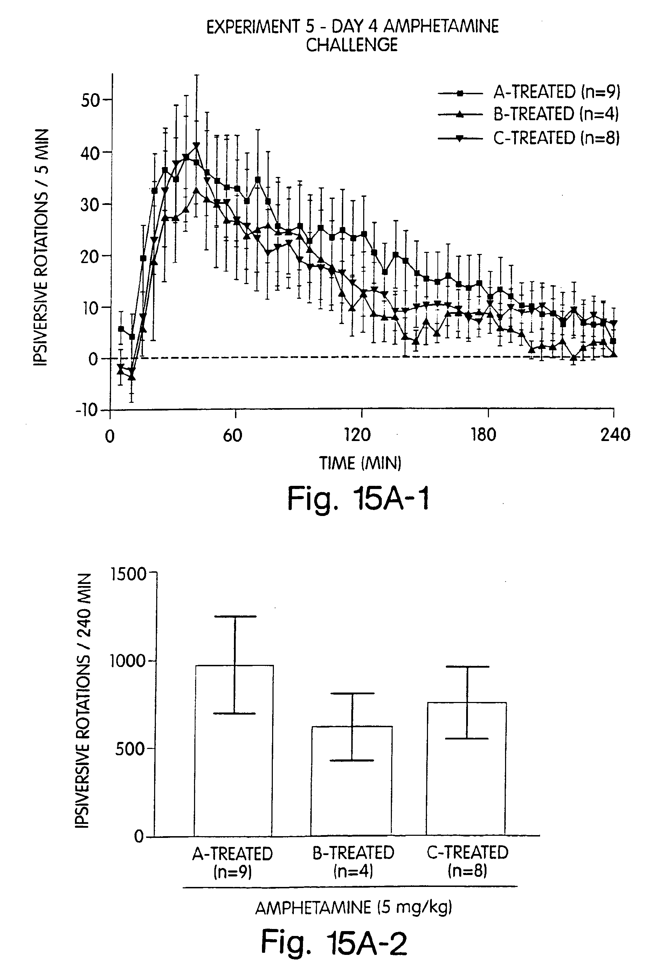

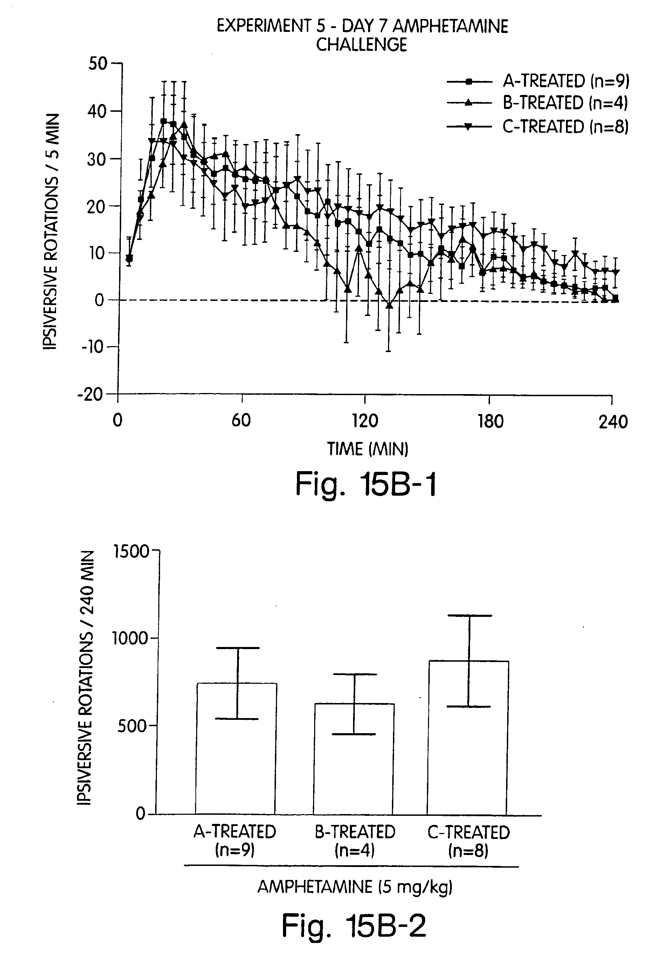

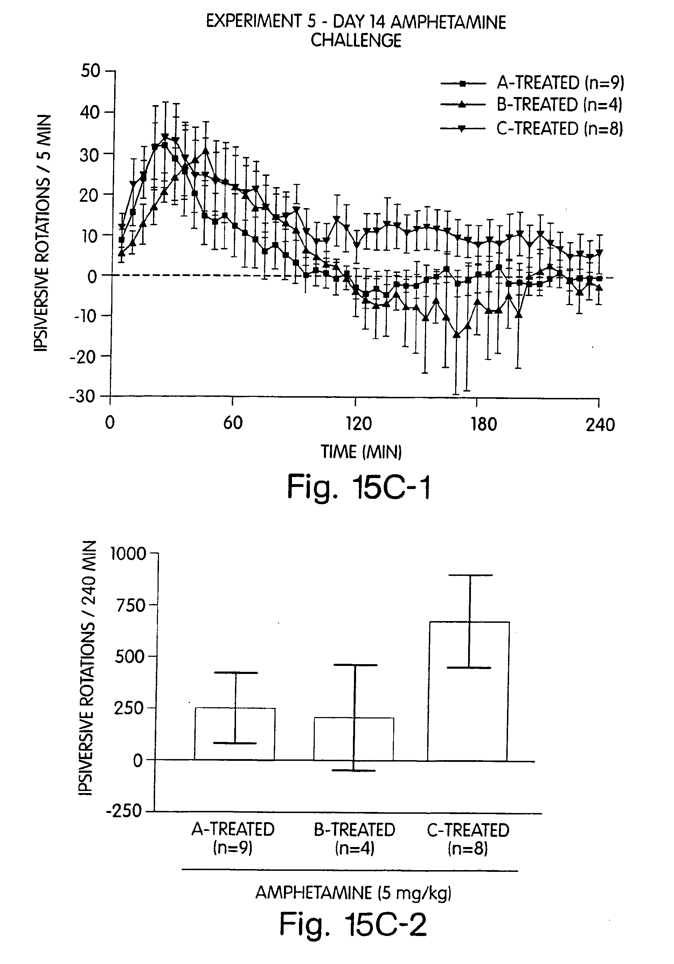

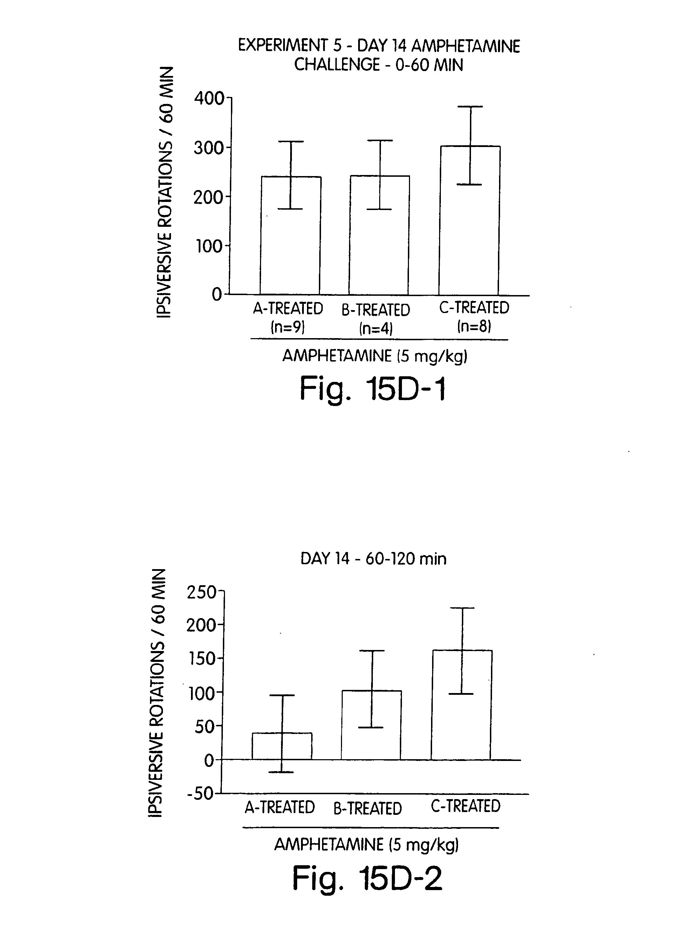

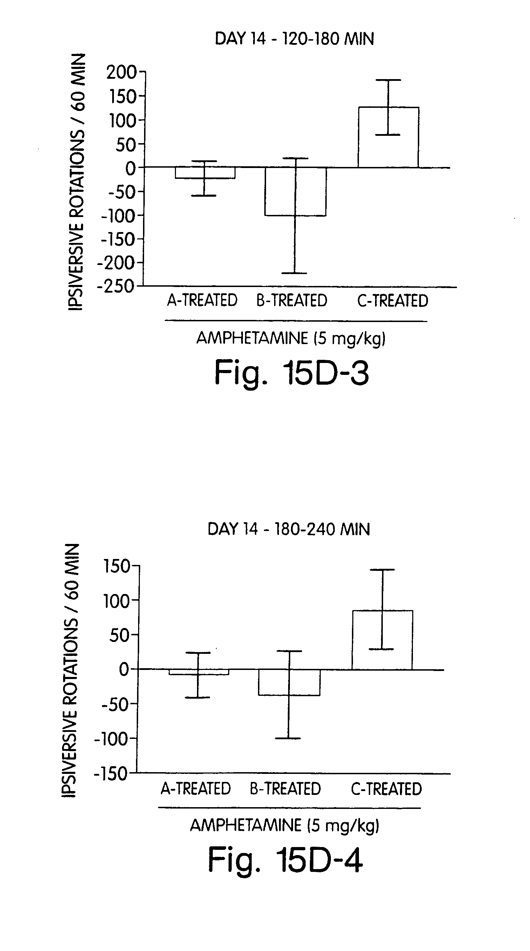

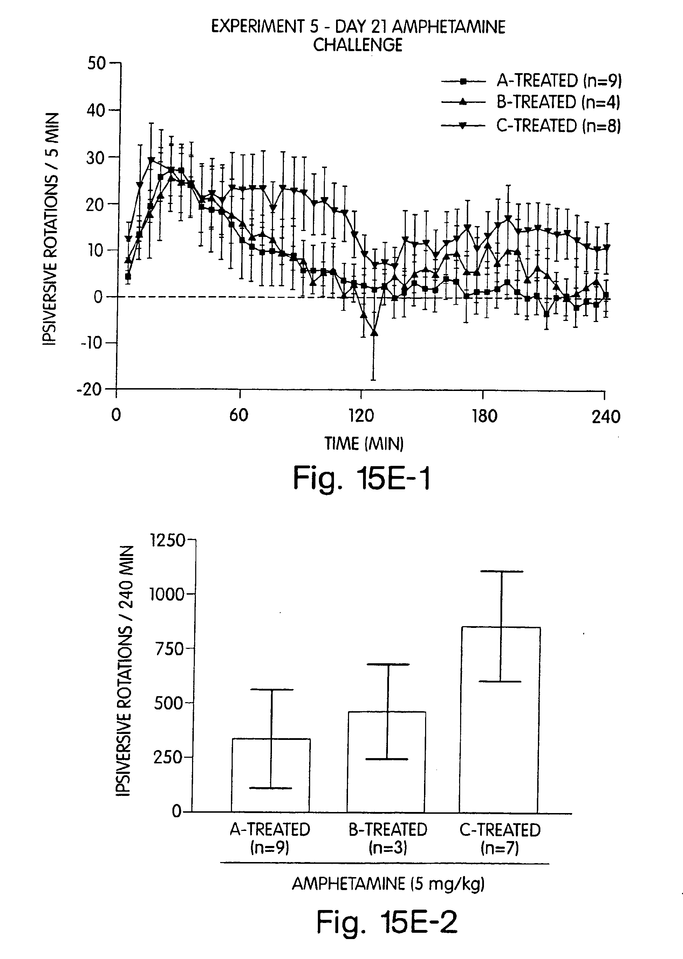

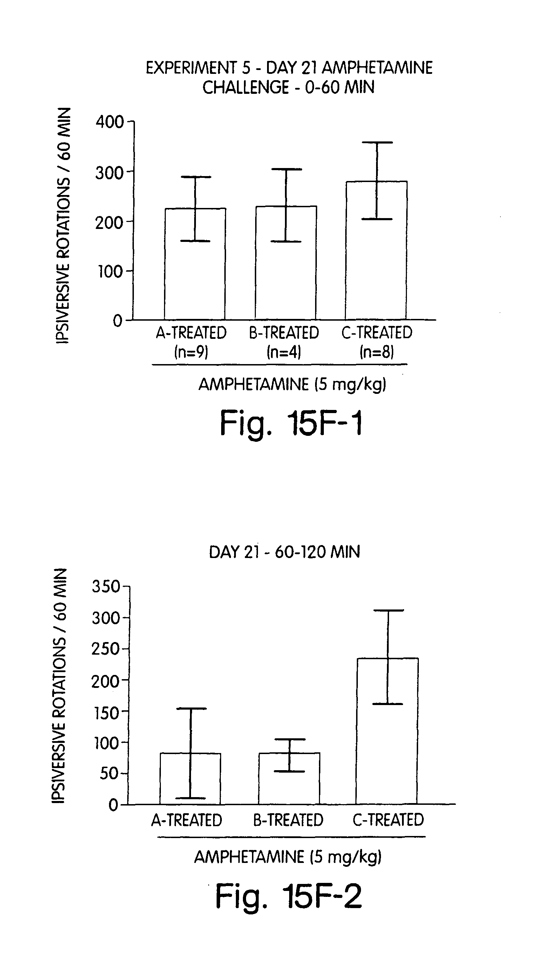

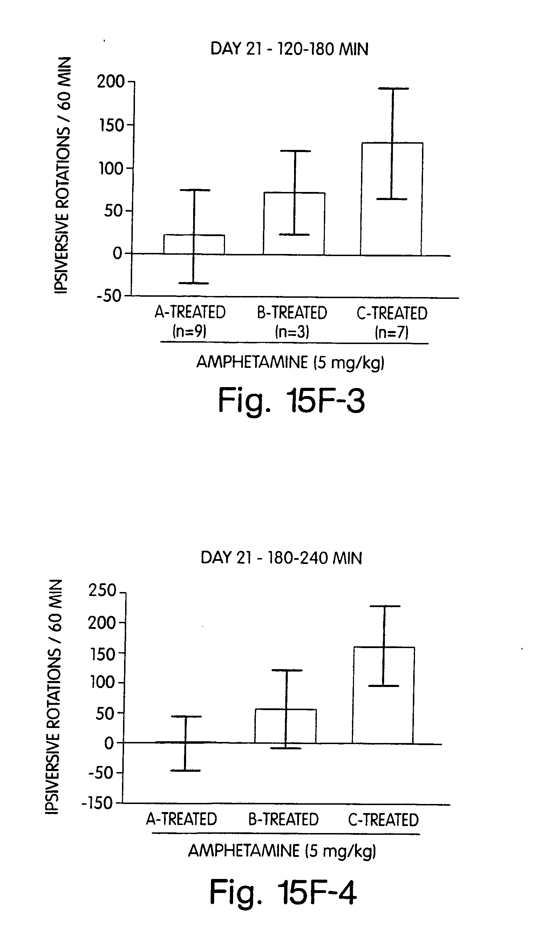

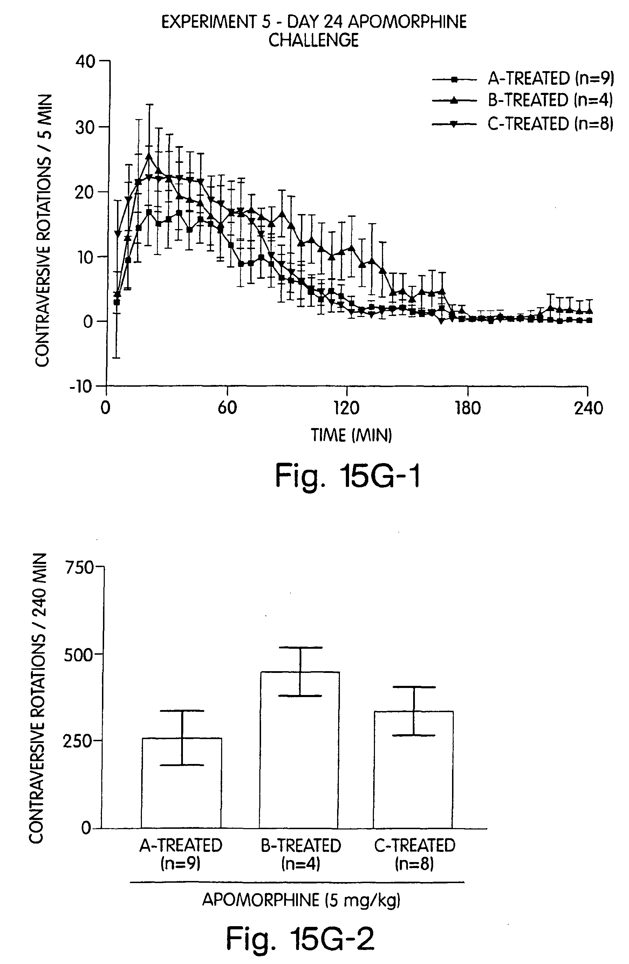

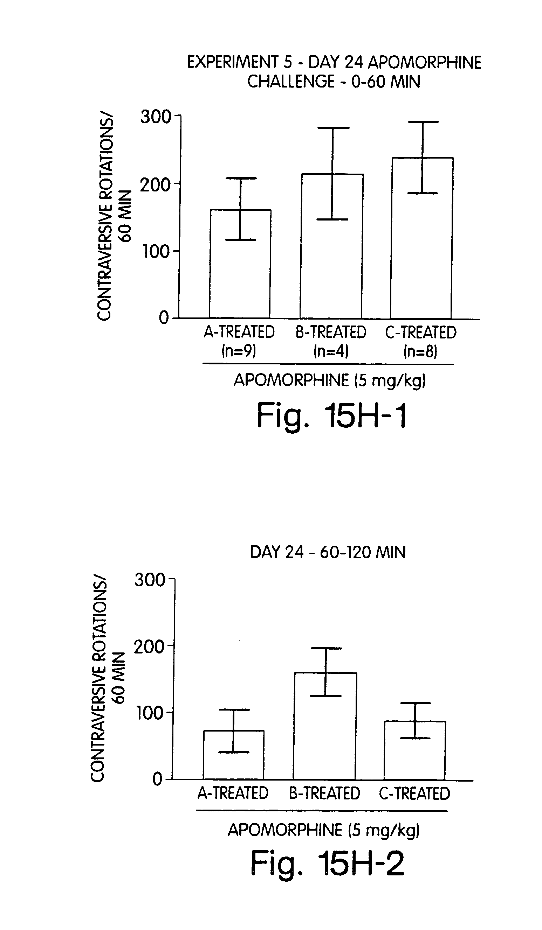

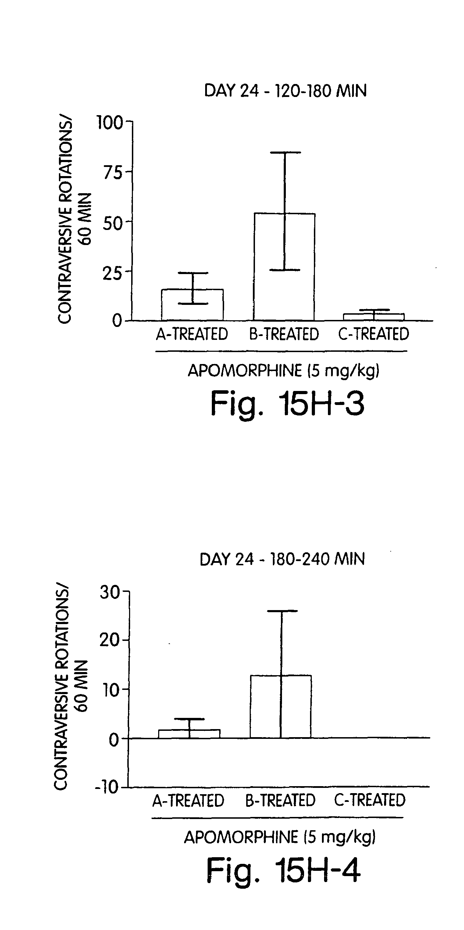

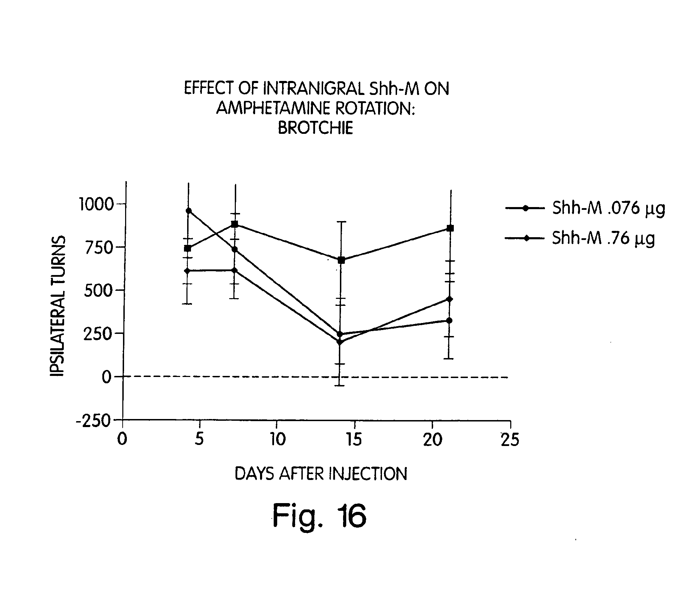

Utilizing the protocol of Example 4, animals were treated with either vehicle ("C-treated"), or 76ng ("A-treated") or 760ng ("B-treated") of a myristoylated N-terminal fragment of Shh. At days 4, 7, 14 and 21, the animals were challenged with amphetamines and ipsiversive rotations were measured. The animals were also challenged on day 24 with apomorphine. Figures 15A-H demonstrate the dose-dependent neuroprotective activity of Shh. Figure 16 is a collective graph of the data, specifically the ipsilateral turns over 21 days after 6-OHDA treatment, and indicates that myristoylated Shh protects against 6-OHDA lesions.

### Example 6: Restoration of function paradigm - comparison of myristoylated and unmyristoylated Shh.

These experiments compared reduction in toxin-induced asymmetry of the nigrstriatal system, as examined by apomorphine and amphetamine induced rotational behaviors, for various forms of *hedgehog* polypeptides, namely lipid-modified and unmodified forms of the proteins. See, e.g., Pepinsky et al. (1998) JBC 273:14037 for a discussion of lipid-modified *hedgehog* polypeptides.

Animals were prepared in a similar fashion to the 6-OHDA lesioned rats of Example 2. Briefly, each rat was given a dose of desipramine HCl (25 mg/kg i.p.) and pargyline (50 mg/kg i.p.), and anesthetized with isoflurane in oxygen. After shaving the scalp, the head was fixed in a stereotaxic frame according to the atlas of Paxinos and watson (1986). A midline longitudinal incision was made, and the skin flap retracted to reveal the surface of the skull. With the aid of an operating microscope, a small burr-hole was drilled overlaying the left medial forevrain bundle (stereotaxic coordinates: AP +5.2 mm from interaural line (or AP -3.8 mm from bregma); L 1.0 mm). The tip of an injection cannula (backfilled with injectate) was lowered to a depth of 8.0 mm below the surface of the brain so that the tip is located in the medial forebrain bundle, and was left in place for 5 min before the injection. 6-Hydroxydopamine hydrobromide (1.5 or 6 µg in 2 µl) was injected over 5 min, with the cannula left in place for a further 5 min. After remocal of the cannula the burr-hole was sealed bone wax, and the scalp wound closed with sutures.

### Apomorphine (Contrlateral) Circling Tests (Days 14, 22, 30 and 38)

An 8-channel Rotometry System (Benwick Electronics, Wimblington, U.K.) was used for the circling tests. Apomorphine HCI and amphetamine HCl were dissolved in sterile water for injection. For each test, rats were dosed with apomorphine HCl (0.3 mg/kg i.p.), placed in a swivel-harness in rotometry bowls (diamter 24 cm), and left in the bowls for 45 min. Clockwise and anticlockwise rotations were logged automatically.

### Amphetamine (Ipsilateral) Circling Tests (Days 15, 23, 31 and 39)

The rats were placed in a rotometry bowl (as above) prior to injection of amphetamine HCl (1 mg/kg i.p.). Immediately after injection, they were placed in the rotometer harness and left in the bowls for 120 min. Clockwise and anticlockwise rotations were logged automatically.

### Microinjection of Shh or Vehicle (Day 21)

Each rat was anesthetized with halothane (1.3% in oxygen) and, after shaving the scalp, the head was fixed in a stereotaxic frame according to the atlas of Paxinos and Watson (1986). A longitudinal incision was made, offset to the left of the midline, and the skin flap retracted to reveal the surface of the skull. With the aid of an operating microscope, a small burr-hole was drilled overlying the left sunstantia nigra (stereotaxic coordinates AP -5.6 mm from bregma; L 2.0 mm). The tip of an injection cannula (backfilled with injectate) was lowered to a depth of 7.3 mm elow the brain surface so that the tip was located dorsal to the middle of the substantial nigra, and was left in place for 5 min before the injection. The injectate (1 µl of solution Gz or Mz) was injected over 5 min, and the cannula left in place for a further 5 min. After removal of the cannula, the burr hole was sealed with bone wax, the scalp wound closed with sutures, and the anesthesia discontinued.

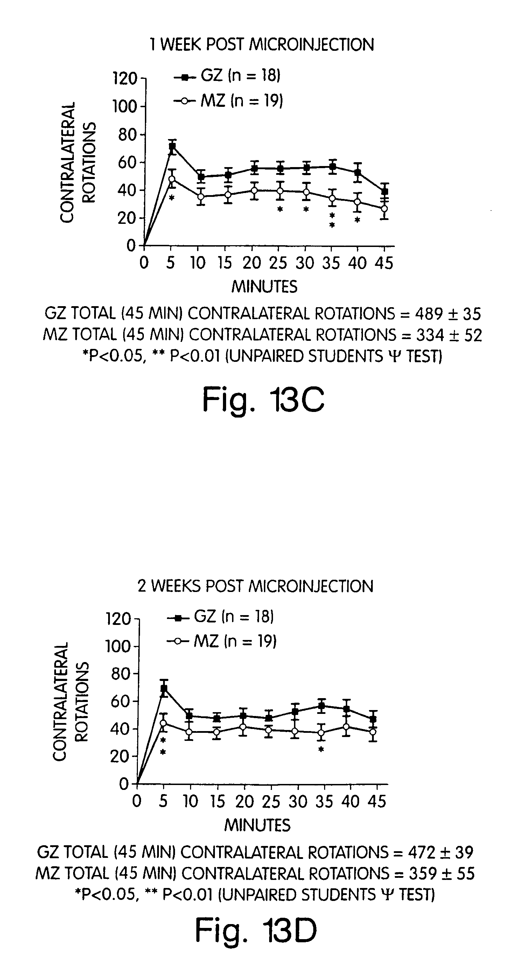

Figures 12 and 13 demonstrate an increased potency, as a restorative agent, of a myristoylated form of Shh (Mz) relative to the unmodified form of the protein (Gz).

### Example 7: Evaluating the Efficacy of Human Sonic Hedgehog polypeptide Constructs in a Rat Model of Huntington's Disease

Injection of malonate, an inhibitor of the mitochondrial enzyme succinate dehydrogenase, into the rat striatum (the rodent equivalent of the primate caudate and putamen) causes degeneration of striatal medium spiny neurons. In humans, degeneration of medium spiny neurons in the caudate and putamen is the primary pathological feature of Huntington's disease. Thus, the malonate-induced striatal lesion in rats can be used as a model to test whether human *hedgehog* polypeptides can prevent the death of the neurons that degenerate in Huntington's disease.

Sprague-Dawley rats are injected with various concentrations of *hedgehog* polypeptide in the striatum using stereotaxic techniques. Stereotaxic injections (2 il) are done under sodium pentobarbital anesthesia (40 mg/kg) and placed at the following coordinates: 0.7 mm anterior to bregma, 2.8 mm lateral to the midline and 5.5 mm ventral to the surface of the skull at bregma. At various times (usually 48 hr) after injection of *hedgehog* polypeptide, rats are anesthetized with isoflurane and given a stereotaxic injection of malonate (2 imols in 2 il) at the same coordinates in the striatum. Four days after malonate injection, rats are sacrificed and their brains removed for histological analysis. Coronal sections are cut through the striatum at a thickness of 25 im and stained for cytochrome oxidase activity to distinguish lesioned from unlesioned tissue. The volume of the lesion in the striatum is measured using image analysis.

The effect of sonic *hedgehog* polypeptide constructs given 48 hours before malonate is shown in Figure 17. Unmodified sonic *hedgehog* polypeptide (Shh), myristoylated Shh, C1II-Shh and octyl maleimide Shh all reduce lesion volume to a similar extent in this model. However, the hydrophobically modified proteins (myristoylated Shh, C1II-Shh and octyl maleimide Shh) show an increase in potency relative to the unmodified sonic *hedgehog* polypeptide. Figure 18 shows the time course for the effect of pretreatment with myristoylated Shh (0.5 ig in 2 il) in the malonate striatal lesion model. A statistically significant reduction in lesion volume is seen when myristoylated Shh is given 2 days before malonate, however myristoylated Shh has a greater effect when given either 3 or 4 days before malonate. There was not a statistically significant reduction in lesion volume when myristoylated Shh was given 1 day, 7 days or immediately (0 days) before malonate.

### Example 8: Evaluating the Efficacy of Hedgehog polypeptides in a primate model of Parkinson's Disease

As illustrated by Figures 19A and 19B, we have observed that intranigral injection of myristoylated Sonic Hedgehog (Shh-M) reduces parkinsonian disability and increases the therapeutic effect of L-DOPA in MPTP-treated marmosets. Adult common marmosets were rendered parkinsonian by treatment with 1-methyl-4-phenyl-1,2,3,6-tetrahydropyridine (MPTP; 0.5 mg/kg s.c. once daily for 5 days) during week 0. The extent of disability was scored weekly for the next 8 weeks using a visual marmoset disability scoring system that assessed alertness, checking, posture, balance, reactions, vocalization and motility (maximum disability score = 18). During week 10, marmosets were given a unilateral injection into the substantia nigra of either Shh-M (0.15 µg or 1.5 µg in 2 µl; n=6) or vehicle solution (2 µl; control group; n=5). Disability was monitored weekly for the next 4 weeks, the intranigral injection was repeated, and disability was monitored weekly for 4 more weeks. Figure 19A shows that Shh-M-treated marmosets exhibited a consistent reduction in disability relative to vehicle-treated marmosets. During weeks 21, all animals were given a low dose of the antiparkinsonian drug L-DOPA (25 mg/kg, p.o.), and disability was assessed every 10 min for 50 min. Figure 19B shows that, although both groups showed the same extent of disability prior to administration of L-DOPA, L-DOPA reduced disability to a greater extent in Shh-M-treated marmosets than in controls.

Figure 19C compares the activity of hedgehog polypeptides with GDNF according to the protocols described above.

### Example 9: Evaluating the Protective Effect of Hedgehog polypeptides in a Rat model of Parkinson's Disease

Intrastriatal injection of myristoylated Sonic Hedgehog (Shh-M) protects dopamine neurons from degeneration induced by 6-hydroxydopamine (6-OHDA). Rats were injected with either Shh-M (0.18 µg in 1.5 µl) or vehicle solution on days 1, 3, 5 and 8 through a cannula implanted in the striatum. On day 4, they were given an injection of 6-OHDA (25 µg in 1.5 µl) through the cannula. Rotational behavior in response to amphetamine (1.3 mg/kg, i.p.) was measured weekly for 4 weeks beginning 1 week after 6-OHDA. Rats were then sacrificed, and dopamine neurons in sections through the substantia nigra were stained with an antibody to tyrosine hydroxylase (TH) and counted. The graph on the left shows that treatment with Shh-M reduced amphetamine-induced rotation in weeks 2-4. The graph on the right shows that treatment with Shh-M reduced the loss of dopamine neurons caused by 6-OHDA.

## Claims

1. Use of a lipophilic modified hedgehog polypeptide in the preparation of a medicament for treating a disorder **characterized by** loss of dopaminergic and/or GABAergic neurons, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

2. Use of a lipophilic modified hedgehog polypeptide in the preparation of a medicament for treating or preventing Parkinson's disease or Huntington's disease, wherein said hedgehog polypeptide comprises an amino acid sequence encodable by a nucleic acid sequence that hybridises under stringent conditions to a nucleic acid sequence designated in at least one of SEQ ID Nos: 1-9, and wherein said lipophilic modification comprises at least one fatty acid moiety on one or more internal sites or on an N-terminal amino acid residue of the mature hedgehog polypeptide.

3. Use of a lipophilic modified hedgehog polypeptide in the preparation of a medicament for treatment or prophylaxis of a disorder selected from the group consisting of domoic acid poisoning; spinal cord trauma; hypoglycemia; mechanical trauma to the nervous system; senile dementia; Korsakoff's disease; schizophrenia; AIDS dementia, multi-infarct dementia; mood disorders; depression; chemical toxicity; neuronal damage associated with uncontrolled seizures, such as epileptic seizures; neuronal injury associated with HIV and AIDS; neurodegeneration associated with Down's syndrome; neuropathic pain syndrome; olivopontocerebral atrophy; amyotrophic lateral sclerosis; mitochondrial abnormalities; Alzheimer's disease; hepatic encephalopathy; Tourette's syndrome; schizophrenia; and drug addiction, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

4. Use according to any one of claims 1-3, wherein each fatty acid moiety is independently selected from the group consisting of myristoyl, palmitoyl, stearoyl and arachidoyl.

5. Use according to any of claims 1-3, wherein the hedgehog polypeptide is modified with one or more aromatic hydrocarbons.

6. Use according to claim 5, wherein each aromatic hydrocarbon is independently selected from the group consisting of benzene, perylene, phenanthrene, anthracene, naphthalene, pyrene, chrysene, and naphthacene.

7. Use according to any of claims 1-3, wherein the hedgehog polypeptide is modified one or more times with a C7 - C30 alkyl or cycloalkyl.

8. Use according to any of claims 1-3, wherein said peparation of a medicament is for prophylaxis.

9. A therapeutic preparation of a lipophilic modified hedgehog polypeptide provided in a pharmaceutically acceptable carrier and in an amount sufficient to promote survival of dopaminergic cells in an adult mammal, wherein said hedgehog polypeptide comprises an amino acid sequence encodable by a nucleic acid sequence that hybridises under stringent conditions to a nucleic acid sequence designated in at least one of SEQ ID NO: 1-9, and wherein said lipophilic modification comprises at least one fatty acid moiety on one or more internal sites or on an N-terminal amino acid residue of the mature hedgehog polypeptide.

10. The preparation of claim 9, wherein the lipophilic modified hedgehog polypeptide is provided in an amount sufficient to promote survival of dopaminergic cells in an adult mammal treated with MPTP at 1 mg/kg.

11. The preparation of claim 10, wherein the lipophilic modified hedgehog polypeptide is provided in an amount sufficient to promote survival of dopaminergic cells in an adult mammal treated with MPTP at 10 mg/kg.

12. Use according to claim 2, for limiting damage to neuronal cells by Parkinsonian conditions.

13. A therapeutic preparation of a lipophilic modified hedgehog polypeptide provided in a pharmaceutically acceptable carrier and in an amount sufficient to treat or prophylactically treat Parkinson's disease or Huntington's disease in an adult mammal, wherein said hedgehog polypeptide comprises an amino acid sequence encodable by a nucleic acid sequence that hybridises under stringent conditions to a nucleic acid sequence designated in at least one of SEQ ID NOs: 1-9, and wherein said lipophilic modification comprises at least one fatty acid moiety on one or more internal sites or on an N-terminal amino acid residue of the mature hedgehog polypeptide.

14. Use according to any one of claims 1-3, wherein said lipophilic modification comprises at least one lipophilic moiety on one or more internal sites of the mature hedgehog polypeptide.

15. The therapeutic preparation according to claim 9 or claim 13, wherein said lipophilic modification comprises at least one fatty acid moiety on one or more internal sites of the mature hedgehog polypeptide.

16. Use according to any one of claims 1-3, wherein the hedgehog polypeptide comprises a hedgehog/Ig fusion.

17. The therapeutic preparation according to claim 9 or claim 13, wherein the hedgehog polypeptide comprises a hedgehog/Ig fusion.

18. Use according to any one of claims 1-3, wherein the hedgehog polypeptide is not modified with a sterol moiety.

19. Use according to any one of claims 1-3, wherein the hedgehog polypeptide is not modified with a cholesterol moiety.

20. The preparation of claim 9 or claim 13, wherein the hedgehog polypeptide is not modified with a sterol moiety.

21. The preparation of claim 9 or claim 13, wherein the hedgehog polypeptide is not modified with a cholesterol moiety.

## Patentansprüche

1. Verwendung eines lipophilen modifizierten Hedgehog-Polypeptids bei der Herstellung eines Medikaments zum Behandeln einer Erkrankung, **gekennzeichnet durch** den Verlust von dopaminergen und/oder GABAergen Neuronen, wobei das Hedgehog-Polypeptid eine Aminosäuresequenz, codierbar **durch** eine Nucleinsüuresequenz, umfaßt, die unter stringenten Bedingungen zu einer Nucleinsäuresequenz, bezeichnet in mindestens einer der SEQ ID NOs: 1-9, hybridisiert und wobei die lipophile Modifizierung mindestens eine Fettsäureeinheit auf einer oder mehreren inneren Stellen oder auf einem N-terminalen Aminosäurerest des reifen Hedgehog-Polypeptids umfaßt.

2. Verwendung eines lipophilen modifizierten Hedgehog-Polypeptids bei der Herstellung eines Medikaments zum Behandeln oder Verhüten von Parkinsonscher Krankheit oder Huntingtonscher Krankheit, wobei das Hedgehog-Polypeptid eine Aminosäuresequenz, codierbar durch eine Nucleinsäuresequenz, umfaßt, die unter stringenten Bedingungen zu einer Nucleinsäuresequenz, bezeichnet in mindestens einer der SEQ ID NOs: 1-9, hybridisiert und wobei die lipophile Modifizierung mindestens eine Fettsäureeinheit auf einer oder mehreren inneren Stellen oder auf einem N-terminalen Aminosäurerest des reifen Hedgehog-Polypeptids umfaßt.

3. Verwendung eines lipophilen modifizierten Hedgehog-Polypeptids bei der Herstellung eines Medikaments zur Behandlung oder Prophylaxe einer Erkrankung, ausgewählt aus der Gruppe, bestehend aus Domoinsäurevergiftung; Rückenmarkstrauma; Hypoglykämie; mechanischem Trauma am Nervensystem; seniler Demenz; Korsakoff-Krankheit; Schizophrenie; AIDS-Demenz; Multiinfarkt-Demenz; Gemütserkrankungen; Depression; chemischer Toxizität; neuronaler Schädigung, verbunden mit unkontrollierten Krampfanfällen, wie beispielsweise epileptische Krampfanfälle; neuronaler Verletzung, verbunden mit HIV und AIDS; Neurodegeneration, verbunden mit Down-Syndrom; neuropathischem Schmerzsyndrom; olivopontocerebellärer Atrophie; amyotropher Lateralsklerose; mitochondrialen Abnormalitäten; Alzheimer-Krankheit; Leberenzephalopathie; Tourette-Syndrom; Schizophrenie; und Arzneimittelsucht, wobei das Hedgehog-Polypeptid eine Aminosäuresequenz, codierbar durch eine Nucleinsäuresequenz, umfaßt, die unter stringenten Bedingungen mit einer Nucleinsäuresequenz, bezeichnet in mindestens einer der SEQ ID NOs: 1-9, hybridisiert und wobei die lipophile Modifizierung mindestens eine Fettsäureeinheit auf einer oder mehreren inneren Stellen oder auf einem N-terminalen Aminosäurerest des reifen Hedgehog-Polypeptids umfaßt.

4. Verwendung gemäß einem der Ansprüche 1-3, wobei jede Fettsäureeinheit unabhängig aus der Gruppe, bestehend aus Myristoyl, Palmitoyl, Stearoyl und Arachidoyl, ausgewählt ist.

5. Verwendung gemäß einem der Ansprüche 1-3, wobei das Hedgehog-Polypeptid mit einem oder mehreren aromatischen Kohlenwasserstoffen modifiziert ist.

6. Verwendung gemäß Anspruch 5, wobei jeder aromatische Kohlenwasserstoff unabhängig aus der Gruppe, bestehend aus Benzol, Perylen, Phenanthren, Anthracen, Naphthalin, Pyren, Chrysen und Naphthacen, ausgewählt ist.

7. Verwendung gemäß einem der Ansprüche 1-3, wobei das Hedgehog-Polypeptid ein oder mehrere Male mit einem C₇-C₃₀-Alkyl oder -Cycloalkyl modifiziert ist.

8. Verwendung gemäß einem der Ansprüche 1-3, wobei die Herstellung eines Medikaments zur Prophylaxe erfolgt.

9. Therapeutische Zubereitung eines lipophilen modifizierten Hedgehog-Polypeptids, bereitgestellt in einem pharmazeutisch verträglichen Träger und in einer Menge, ausreichend, um das Überleben von dopaminergen Zellen in einem erwachsenen Säuger zu fördern, wobei das Hedgehog-Polypeptid eine Aminosäuresequenz, codierbar durch eine Nucleinsäuresequenz, umfaßt, die unter stringenten Bedingungen zu einer Nucleinsäuresequenz, bezeichnet in mindestens einer der SEQ ID NO: 1-9, hybridisiert und wobei die lipophile Modifizierung mindestens eine Fettsäureeinheit auf einer oder mehreren inneren Stellen oder auf einem N-terminalen Aminosäurerest des reifen Hedgehog-Polypeptids umfaßt.

10. Zubereitung gemäß Anspruch 9, wobei das lipophile modifizierte Hedgehog-Polypeptid in einer Menge bereitgestellt wird, die ausreichend ist, um das Überleben von dopaminergen Zellen in einem erwachsenen Säuger, behandelt mit MPTP mit 1 mg/kg, zu fördern.

11. Zubereitung gemäß Anspruch 10, wobei das lipophile modifizierte Hedgehog-Polypeptid in einer Menge bereitgestellt wird, die ausreichend ist, um das Überleben von dopaminergen Zellen in einem erwachsenen Säuger, behandelt mit MPTP mit 10 mg/kg, zu fördern.

12. Verwendung gemäß Anspruch 2 zum Begrenzen des Schadens von neuronalen Zellen durch Parkinson-Leiden.

13. Therapeutische Zubereitung eines lipophilen modifizierten Hedgehog-Polypeptids, bereitgestellt in einem pharmazeutisch verträglichen Träger und in einer Menge, ausreichend, um Parkinsonsche Krankheit oder Huntingtonsche Krankheit bei einem erwachsenen Säuger zu behandeln oder prophylaktisch zu behandeln, wobei das Hedgehog-Polypeptid eine Aminosäuresequenz, codierbar durch eine Nuclcinsäuresequenz, umfaßt, die unter stringenten Bedingungen zu einer Nucleinsäuresequenz, bezeichnet in mindestens einer der SEQ ID NOs: 1-9, hybridisiert, und wobei die lipophile Modifizierung mindestens eine Fettsäurecinheit auf einer oder mehreren inneren Stellen oder auf einem N-terminalen Aminosäurerest des reifen Hedgehog-Polypeptids umfaßt.

14. Verwendung gemäß einem der Ansprüche 1-3, wobei die lipophile Modifizierung mindestens eine lipophile Einheit auf einer oder mehreren inneren Stellen des reifen Hedgehog-Polypeptids umfaßt.

15. Therapeutische Zubereitung gemäß Anspruch 9 oder Anspruch 13, wobei die lipophile Modifizierung mindestens eine Fettsäureeinheit auf einer oder mehreren inneren Stellen des reifen Hedgehog-Polypeptids umfaßt.

16. Verwendung gemäß einem der Ansprüche 1-3, wobei das Hedgehog-Polypeptid eine Hedgehog/Ig-Fusion umfaßt.

17. Therapeutische Zubereitung gemäß Anspruch 9 oder Anspruch 13, wobei das Hedgehog-Polypeptid eine Hedgehog/Ig-Fusion umfaßt.

18. Verwendung gemäß einem der Ansprüche 1-3, wobei das Hedgehog-Polypeptid nicht mit einer Steroleinheit modifiziert ist.

19. Verwendung gemäß einem der Ansprüche 1-3, wobei das Hedgehog-Polypeptid nicht mit einer Cholesterineinheit modifiziert ist.

20. Zubereitung gemäß Anspruch 9 oder Anspruch 13, wobei das Hedgehog-Polypeptid nicht mit einer Steroleinheit modifiziert ist.

21. Zubereitung gemäß Anspruch 9 oder Anspruch 13, wobei das Hedgehog-Polypeptid nicht mit einer Cholesterineinheit modifiziert ist.

## Revendications

1. Utilisation d'un polypeptide hedgehog modifié lipophile dans la préparation d'un médicament pour le traitement d'un trouble **caractérisé par** la perte de neurones dopaminergiques et/on GABAergiques, où ledit polypeptide hedgehog comprend une séquence d'acides aminés pouvant être codée par une séquence d'acide nucléique qui s'hybride dans des conditions stringentes à une séquence d'acide nucléique désignée dans au moins l'une des SEQ ID NO: 1-9, et où ladite modification lipophile comprend au moins un fragment d'acide gras sur un ou plusieurs sites internes ou sur un résidu d'acide aminé N-terminal du polypeptide hedgehog mature.

2. Utilisation d'un polypeptide hedgehog modifié lipophile dans la préparation d'un médicament pour le traitement ou la prévention de la maladie de Parkinson ou de la maladie de Huntington, où ledit polypeptide hedgehog comprend une séquence d'acides aminés pouvant être codée par une séquence d'acide nucléique qui s'hybride dans des conditions stringentes à une séquence d'acide nucléique désignée dans au moins l'une des SEQ ID NO: 1-9, et où ladite modification lipophile comprend au moins un fragment d'acide gras sur un ou plusieurs sites internes ou sur un résidu d'acide aminé N-terminal du polypeptide hedgehog mature.

3. Utilisation d'un polypeptide hedgehog modifié lipophile dans la préparation d'un médicament pour le traitement ou la prophylaxie d'un trouble sélectionné parmi le groupe consistant en intoxication par l'acide domoïque; traumatisme de la moelle épinière; hypoglycémie; traumatisme mécanique du système nerveux; démence sénile; maladie de Korsakoff; schizophrénie; démence associée au SIDA ; démence à infarctus multiples; troubles de l'humeur; dépression; toxicité chimique; lésion neuronale associée à des attaques incontrôlées, telles que des attaques d'épilepsie; lésion neuronale associée au VIH et au SIDA; neurodégénérescence associée au syndrome de Down; syndrome de douleur neuropathique; atrophie olivopontocérébelleuse; sclérose latérale amyotrophique; anomalies des mitochondries; maladie d'Alzheimer; encéphalopathie hépatique; syndrome de la Tourette; schizophrénic ; et toxicomanie, où ledit polypeptide hedgehog comprend une séquence d'acides aminés pouvant être codée par une séquence d'acide nucléique qui s'hybride dans des conditions stringentes à une séquence d'acide nucléique désignée dans au moins l'une des SEQ ID NO: 1-9, et où ladite modification lipophile comprend au moins un fragment d'acide gras sur un ou plusieurs sites internes ou sur un résidu d'acide aminé N-terminal du polypeptide hedgehog mature.

4. Utilisation selon l'une quelconque des revendications 1-3, où chaque fragment d'acide gras est indépendamment sélectionné parmi le groupe consistant en myristoyl, palmitoyl, stéaroyl et arachidoyl.

5. Utilisation selon l'une quelconque des revendications 1-3, où le polypeptide hedgehog est modifié avec un ou plusieurs hydrocarbures aromatiques.

6. Utilisation selon la revendication 5, où chaque hydrocarbure aromatique est indépendamment sélectionné parmi le groupe consistant en benzène, pérylène, phénanthrène, anthracène, naphtalène, pyrène, chrysène et naphtacène.

7. Utilisation selon l'une quelconque des revendications 1-3, où le polypeptide hedgehog est modifié une ou plusieurs fois avec un C₇-C₃₀ alkyle ou cycloalkyle.

8. Utilisation selon l'une quelconque des revendications 1-3, où ladite préparation d'un médicament est destinée à la prophylaxie.

9. Préparation thérapeutique d'un polypeptide hedgehog modifié lipophile fourni dans un porteur pharmaceutiquement acceptable et en une quantité suffisante pour favoriser la survie de cellules dopaminergiques chez un mammifère adulte, où ledit polypeptide hedgehog comprend une séquence d'acides aminés pouvant être codée par une séquence d'acide nucléique qui s'hybride dans des conditions stringentes à une séquence d'acide nucléique désignée dans au moins l'une des SEQ ID NO: 1-9, et où ladite modification lipophile comprend au moins un fragment d'acide gras sur un ou plusieurs sites internes ou sur un résidu d'acide aminé N-terminal du polypeptide hedgehog mature.

10. La préparation de la revendication 9, dans laquelle le polypeptide hedgehog modifié lipophile est fourni en une quantité suffisante pour favoriser la survie de cellules dopaminergiques chez un mammifère adulte traité avec du MPTP à 1 mg/kg.

11. La préparation de la revendication 10, dans laquelle le polypeptide hedgehog modifié lipophile est fourni en une quantité suffisante pour favoriser la survie de cellules dopaminergiques chez un mammifère adulte traité avec du MPTP à 10 mg/kg.

12. Utilisation selon la revendication 2, pour limiter les lésions aux cellules neuronales causées par des états parkinsoniens.

13. Préparation thérapeutique d'un polypeptide hedgehog modifié lipophile fourni dans un porteur pharmaceutiquement acceptable et en une quantité suffisante pour traiter ou traiter de manière prophylactique la maladie de Parkinson ou la maladie de Huntington chez un mammifère adulte, où ledit polypeptide hedgehog comprend une séquence d'acides aminés pouvant être codée par une séquence d'acide nucléique qui s'hybride dans des conditions stringentes à une séquence d'acide nucléique désignée dans au moins l'une des SEQ ID NO: 1-9, et où ladite modification lipophile comprend au moins un fragment d'acide gras sur un ou plusieurs sites internes ou sur un résidu d'acide aminé N-terminal du polypeptide hedgehog mature.

14. Utilisation selon l'une quelconque des revendications 1-3, où ladite modification lipophile comprend au moins un fragment lipophile sur un ou plusieurs sites internes du polypeptide hedgehog mature.

15. La préparation thérapeutique selon la revendication 9 ou la revendication 13, où ladite modification lipophile comprend au moins un fragment d'acide gras sur un ou plusieurs sites internes du polypeptide hedgehog mature.

16. Utilisation selon l'une quelconque des revendications 1-3, où le polypeptide hedgehog comprend une fusion hedgehog/Ig.

17. La préparation thérapeutique selon la revendication 9 ou la revendication 13, dans laquelle le polypeptide hedgehog comprend une fusion hedgehog/Ig.

18. Utilisation selon l'une quelconque des revendications 1-3, où le polypeptide hedgehog n'est pas modifié avec un fragment de stérol.

19. Utilisation selon l'une quelconque des revendications 1-3, où le polypeptide hedgehog n'est pas modifié avec un fragment de cholestérol.

20. La préparation de la revendication 9 ou de la revendication 13, dans laquelle le polypeptide hedgehog n'est pas modifié avec un fragment de stérol.

21. La préparation de la revendication 9 ou de la revendication 13, dans laquelle le polypeptide hedgehog n'est pas modifié avec un fragment de cholestérol.
